# EUROPEAN PATENT SPECIFICATION

(11) **EP 1 346 030 B1**
(45) Date of publication and mention of the grant of the patent: **02.11.2011**
(21) Application number: 01902597.2
(22) Date of filing: 22.01.2001
(51) Int. Cl.: C12N 9/02, C12N 15/82, A01H 5/10

(54) **LOW-LIPOXYGENASE 1 BARLEY**
GERSTE MIT NIEDRIGEM LIPOXYGENASE-1 GEHALT
ORGE A LIPOXYGENASE 1 FAIBLE

(30) Priority: 29.12.2000 US 751687; 29.12.2000 WO PCT/IB00/02045
(43) Date of publication of application: 24.09.2003
(60) Divisional application: 10011413.1
(73) Proprietor: Carlsberg Research Laboratory, 2500 Copenhagen (DK); HEINEKEN TECHNICAL SERVICES B.V., 2380 BB Zoeterwoude (NL); Brasseries KRONENBOURG Société dite : S.A., 67037 Strasbourg Cedex 2 (FR)
(72) Inventor: DOUMA, Anna Christiana, NL-3707 SB Zeist (NL); DODERER, Albert, NL-2380 BB Zoeterwoude (NL); CAMERON-MILLS, Varena, DK-2500 Valby (DK); SKADHAUGE, Birgitte, Carlsberg Research Laboratory, DK-3460 Birkeroed (DK); BECH, Lene, Molskov, DK-2765 Smorum (DK); SCHMITT, Nathalie, NL-2584 ET Den Haag (NL); HEISTEK, Jolanda Carolina, NL-3132 VA Vlaardingen (NL); VAN MECHELEN, Johannes Reinier, NL-1013 HD Amsterdam (NL)
(74) Representative: Williams, Aylsa
(86) International application number: PCT/IB2001/000207
(87) International publication number: WO 2002/053721

(56) References cited:
- WO-A-97/13851
- CHEMICAL ABSTRACTS, vol. 119, no. 23, 6 December 1993 (1993-12-06) Columbus, Ohio, US; abstract no. 248220m, KOWAKA, M.: "Malting barley improvement for brewing" page 828; column 2; XP002175449 & NIPPON JOZO KYOKAISHI, vol. 88, no. 8, 1993, pages 574-581,
- WAN HENG WANG ET AL: "Molecular basis of a null mutation in soybean lipoxygenase 2: substitution of glutamine for an iron-ligand histidine." PROCEEDINGS OF THE NATIONAL ACADEMY OF SCIENCES, vol. 91, no. 13, 1994, pages 5828-5832, XP002175448 WASHINGTON US
- MCELROY D ET AL: "WHAT'S BREWING IN BARLEY BIOTECHNOLOGY?" BIO/TECHNOLOGY, NEW YORK, US, vol. 13, no. 3, 1995, pages 245-249, XP002024712 ISSN: 0733-222X cited in the application
- DROST B W ET AL: "FLAVOR STABILITY" JOURNAL OF THE AMERICAN SOCIETY OF BREWING CHEMISTS, vol. 48, no. 4, 1990, pages 124-131, XP000926610 ISSN: 0361-0470 cited in the application
- VAN MECHELEN JAN R ET AL: "Molecular characterization of two lipoxygenases from barley." PLANT MOLECULAR BIOLOGY, vol. 39, no. 6, April 1999 (1999-04), pages 1283-1298, XP001015731 ISSN: 0167-4412
- KOBAYASHI, N., ET AL.: "Behaviour of mono-, di-, and trihydroxyoctadecenoic acids during mashing and methods of controlling their production", JOURNAL OF BIOSCIENCE AND BIOENGINEERING, vol. 90, 2000, pages 69-73,

## Description

### FIELD OF THE INVENTION

This invention is in the field of plant biotechnology. More specifically, the invention relates to a mutant barley lipoxygenase 1 gene *(lox-1)* that encodes an enzyme with severely reduced 9-hydroperoxyoctadecanoic acid forming activity. The invention also relates to the use of barley cultivars homozygous for *lox-1* in brewing processes to reduce the formation of off-flavors in brewed products, such as beer, during storage.

### BACKGROUND OF THE INVENTION

Lipoxygenases are a family of enzymes (EC 1.13.11.12) that catalyze the dioxidation of free and esterified poly-unsaturated fatty acids containing a 1(Z), 4(Z)-pentadiene configuration. The products of lipoxygenase-catalyzed reactions have long been suspected as major culprits for the appearance of stale flavors in plant grain/seed and grain/seed derived food products (Robinson et al., 1995, Food Chem., 54: 33-43). Lipoxygenases have been implicated in the production of volatile hexanal aldehydes generated during soybean processing, which have an undesirable aroma, limiting the use of soybean proteins in food products. Three lipoxygenase isozymes expressed in soybean seed are believed to contribute to lipid oxidation and hexanal formation. Soybean mutants lacking one or more of these isozymes have been generated with the aim of reducing hexanal formation and improving their flavor stability. The success of this approach has been evaluated by Hildebrand et al., 1990, J. Agric. Food Chem. 38: 1934-1936. Mutants lacking soybean lipoxygenase 3 produced higher hexanal levels, suggesting that this isozyme diverts 13-hydroxyperoxyoctadecanoids, produced by lipid oxidation, towards non-volatile products. The field performance of triple-null soybean lines, lacking all three seed lipoxygenases, has shown that these enzymes are not essential for normal agronomic and seed characteristics (Narvel et al., 1998, Crop Sci. 38: 926-928).

Lipoxygenases have also been implicated in the generation of off-flavors in rice, which can occur during grain storage. The release of free fatty acids can be detected in stored grain, which is indicative of the metabolism of the triglycerides reserves. The rice variety Daw Dam was found to accumulate lower levels of pentanals and hexanals giving a better flavor stability on storage (Susuki et al., 1999, J. Agric. Food Chem., 47: 1119-1124). This desirable phenotype was attributed to the absence of rice lipoxygenase-3, which oxidises unsaturated lipid acyl chains to form 9-hydroxyperoxyoctadecanoic positional isomers.

It is recognised that the lipoxygenase pathway is complex with many branches and its role in numerous aspects of plant growth and physiology are not fully understood. Modifications of the lipoxygenase pathway which alter 9-hydroperoxidation activity in seed crops are proposed to regulate their susceptibility to mycotoxin contamination by Aspergillus spp. (WO 9726364), which is consistent with the involvement of this pathway in plant pathogen resistance, but is not related to the aims of the invention herein described.

Among the many aroma volatiles which contribute to the flavor of beer, the higher unsaturated aldehydes with a 6-12 carbon chain have particularly low organoleptic flavor thresholds (Meilgaard 1975, MBAA Tech. Quart. 12: 151-168). *Trans*-2-nonenal, which is a member of this group, has both an extremely low flavor threshold of 0.11 ppb and contributes an unpleasant straw-like, "cardboard" flavor to the beer. The characteristic off-flavor caused by trans-2-nonenal is a common characteristic of beers stored for 1-3 months or more and is particularly detrimental to the flavor of lager beer, which is brewed with light malts and has a delicate flavor.

Sulfite has long been known to improve the flavor stability of beer, not only by binding oxygen and acting as an anti-oxidant, but also by forming volatile bisulfite addition compounds with aldehydes and ketones present in the beer. The two major sources of sulfite in beer are sulfite produced by yeast during fermentation via the sulfur assimilation pathway and sulfite added to the beer prior to packaging. Fermentation conditions that enhance yeast sulfite production and secretion will allow the formation of sulfite-carbonyl adducts from carbonyls present in the wort and prevent their further metabolism by the yeast (Dufour 1991, Proc.Eur. Brew. Conv. Congr., Lisbon, pp. 209-216). In this manner carbonyls such as acetaldehyde and diacetyl may be transferred to the beer. The ability of sulfite to prevent the appearance of the carbonyl compound trans-2-nonenal during beer aging has been demonstrated by brewing beer with a yeast strain blocked in the sulfur assimilation pathway (Johannesen et al., 1999, Proc.Eur. Brew. Conv. Congr., Nice, pp. 655-662). Following bottling, the beer was subjected to forced aging by storing it at 37°C for 7 days, after which trans-2-nonenal levels were found to be well above the taste-threshold. If 10 ppm sulfite was added to the low-sulfite beer just prior to bottling, the appearance *of trans-2-*nonenal during forced aging was significantly reduced. The reaction between sulfite and carbonyl compounds is reversible and under thermodynamic and kinetic control. The apparent equilibrium constants for bisulfite compounds ranges from 10⁻⁶ M for carbonyl compounds such as acetaldehyde, hexanal, and decanal, to 10⁻³ for diacetyl and pyruvate (Dufour 1991, *supra*). During beer storage, gas exchange through the packaging will allow oxygen into the beer and sulfite will be lost, such that weaker bisulfite adducts will dissociate, allowing free carbonyls to appear in the beer. While sulfite unquestionably enhances the flavor-stability of beer, particularly in the short-term, its retention in packaged beer is strongly dependent on gas exchange through the packaging and temperature. In a finished beer the natural levels of sulfite produced during fermentation are variable and the addition of sulfite prior to bottling is not a universally accepted practice. For these reasons sulfite alone does not provide a reliable method to enhance the long-term flavor-stability of beer under the different beer storage conditions used around the globe.

It is generally accepted that the *trans*-2-nonenal found in beer results from the oxidation of polyunsaturated fatty acids derived from barley grain lipids, where the 18-carbon chain fatty acid, linoleic acid [classified as an 18:2,n-6 polyunsaturated fatty acid (Broun, Gettner and Sommerville 1999, Annu. Rev. Nutr. 19: 197-216)] is the most abundant. However, there is little agreement in the literature as to the mechanism whereby trans-2-nonenal is formed. The presence of enzymatic pathways leading to *trans*-2-nonenal formation from poly-unsaturated fatty acids has been proposed, but the individual enzymatic steps have never been demonstrated experimentally in barley grain or during the malting process (Gardner 1988, Adv. Cereal Sci. Technol. 9: 161-215). The concept of using anti-sense or co-suppression gene technology to reduce lipoxygenase-1 levels in barley grain, and thereby control 9-hydroperoxidation and reduce aldehyde and alcohol levels in the finished barley grain, has been proposed as a means to control off-flavor formation, but results of such an approach are not reported (McElroy and Jacobsen, 1995, Bio/Technology 13: 245-249).

A forcing test has been developed as a method for assessing the trans-2-nonenal potential of a beer, where trans-2-nonenal formation in wort or beer is induced by subjecting samples to elevated temperatures at reduced pH, (100°C, at pH 4.0 for 2 hours). Attempts to correlate the *trans*-2-nonenal potential in wort and finished beer with the total level of lipoxygenase activity in the kilned malt have indicated that lipoxygenase may contribute to the appearance of trans-2-nonenal in aged beer (Drost et al., 1990, J. Am. Soc. Brew. Chem. 48: 124-131). The conclusions that can be drawn from this study, however, are severely limited by the fact that the lipoxygenase activity in the barley malt was regulated at the end of the malting process by the degree of enzyme inactivation during kiln drying. Thus, only the effect of the residual malt lipoxygenase activity on the *trans*-2-nonenal potential in the derived wort and finished beer was examined. The study failed to evaluate the lipoxygenases that catalyse the first step in the lipoxygenase enzymatic pathway in the barley grain during development and malting, and their role as determinants of *trans*-2-nonenal levels found in beer. Indeed, the absence of barley cultivars deficient in one or more lipoxygenase isoenzyme has made it impossible to provide convincing evidence for the role of the lipoxygenase pathway in barley malt in controlling the formation of *trans*-2-nonenal. Such experiments are needed to evaluate the contribution of enzymatic, as compared to auto-oxidative/chemical pathways, to the formation of trans-2-nonenal in beer. The brewing process involves a high temperature step of wort boiling where these non-enzymatic reactions are proposed to occur (Noël et al., 1999, J. Agric. Food Chem. 47: 4323-4326).

### SUMMARY OF THE INVENTION

This invention provides a barley plant having greatly reduced lipoxygenase-1 activity. In one embodiment, the barley plants of the invention contain a mutant *lox-1* gene expressing greatly reduced levels of the isoenzyme lipoxygenase-1, wherein said mutated LOX-1 protein comprises the amino acid sequence shown in SEQ ID NO: 12, wherein Xaa is aspartic acid, glutamic acid, histidine, lysine, arginine, threonine, serine, tyrosine, trytophan, asparagine or glutamine.

As shown herein, malt and wort produced from the reduced lipoxygenase barley of the invention are useful to produce beer with significantly enhanced flavour stability and reduced *trans*-2-nonenal levels, particularly under conditions known to promote the appearance of T2N. The invention demonstrates a correlation between the activity of barley malt lipoxygenase-1 to produce 9-hydroxyperoxy-octadecadienoic acids (9-HPOD), and the presence of *trans*-2-nonenal in beer. Use of barley homozygous for the mutant *lox-1* gene in the brewing process improves the flavor stability of the beer, both during storage and on exposure to elevated storage temperatures. These properties enhance the quality of the beer, and are useful to extend its shelf-life and reduce the need to cool beer during transport and storage.

The invention provides barley plants and portions thereof having reduced lipoxygenase-1 activity expressing mutant LOX-1 protein as described herein. Methods for producing such barley plants, plant portions, products of the plants, and particularly malt and wort produced from the barley plants of the invention are described herein.

### BRIEF DESCRIPTION OF THE DRAWINGS

Figure 1 is a graph showing the effect of the inhibitor nordihydroguaiaretic acid (NDGA) on immuno-affinity purified lipoxygenase 1 and 2 activity from embryos of 3 day germinated barley grain.
Figure 2 is a graph showing the fresh weight of developing grain of Line G and cv Vintage from 5 days after flowering to full-maturity (FM). Each determination is the mean single grain weight from 6 spikes.
Figure 3 is a graph showing the dry weight of developing grain of Line G and cv Vintage from 5 days after flowering to full-maturity (FM). Each determination is the mean single grain weight from 3 samples of 5 grain.
Figure 4 is a graph showing total lipoxygenase activity in developing grain of Line G and cv Vintage from 5 days after flowering to full-maturity (FM).
Figure 5 is a graph showing 9- and 13-HPOD products of linoleic acid oxidation by lipoxygenase activity in developing grain of Line G.
Figure 6 is a graph showing total lipoxygenase activity in embryos of germinating grain of Line G and cv Vintage expressed as µ mol/min/10 embryos (U/10 embryos).
Figure 7 is a graph showing 9- HPOD and 13-HPOD products of linoleic acid oxidation by lipoxygenase activity in embryos of germinating grain of Line G and cv Vintage, showing levels of 9- HPOD and 13-HPOD.
Figure 8 is a Western blot showing immunodetection of lipoxygenase 1 in embryos of developing grain of Line G and cv Vintage [wt] from 5 days after flowering to full-maturity (FM).
Figure 9 is a Western blot showing immunodetection of lipoxygenase 1 in embryos of grain of Line G and cv Vintage [wild-type] germinated for 0 - 6 days.
Figure 10 is a Northern blot probed with the 3' non-transcribed region of the *lox*-1 cDNA and showing lipoxygenase 1 transcripts detected in developing grain of Line G and cv Vintage [wild-type] from 5 days after flowering to full-maturity (FM).
Figure 11 is a Northern blot probed with the 3' non-transcribed region of the *lox*-1 cDNA and showing lipoxygenase 1 transcripts detected in embryos of grain of Line G and cv Vintage [wt] germinated for 0 - 6 days.
Figures 12A-12G are a nucleotide sequence alignment of the promoter and transcribed region of the *lox*-1 wild-type cv Vintage allele (WT) and the Line G allele (LG). The transcription start site (+1), ATG start codon (+69) and translation stop codon (+4231) in the gene sequences are underlined. Nucleotide mutations identified in the Line G allele are shown in bold italics and indicated by an asterisk.
Figure 13 is a schematic presentation of the *lox*-1 gene of cv Vintage (wild-type) and the mutant *lox*-1 gene of Line G. The transcript from +1 to +4375 is composed of 7 exons (stippled boxes) and 6 introns (white boxes). Two mutations in the *lox*-1 gene are indicated.
Figure 14 is a schematic drawing of gene cassettes for transient expression of the wild-type *lox*-1 cDNA and *lox-*1 gene and the mutant *lox*-1 gene from Line G. The lipoxygenase coding sequences were cloned between the constitutive maize ubiquitin promoter with intron 1 (*Ubi*-1) and the *nos* terminator.
Figure 15 is a bar graph showing Lipoxygenase 1 activity in barley aleurone protoplasts transfected with gene cassettes containing the wild-type *lox*-1 cDNA; the mutant *lox*-1 gene from Line G; WT *lox*-1 gene; and a control GUS reporter gene. Lipoxygenase activity in extracts of transfected protoplasts was assayed in microtiter plates by the oxidation of KI and quantitated spectrophotometrically. Lipoxygenase 1 activity was expressed as units per µg protein in the extract and is shown as the mean of 3 measurements from 2 replicate assays.
Figure 16 is a sequence alignment demonstrating that a RFLP between the wild-type and mutant *lox*-1 gene is due to a point mutation at nucleotide 2347, creating an additional *AatII* restriction site.
Figure 17 is a schematic presentation of the *lox*-1 PCR fragments amplified and cleaved in the polymerase chain reaction - cleavage amplified polymorphic site (PCR-CAPS) assay. The positions of PCR primers are indicated by arrows and the *Aat*II sites are shown above the gene (sequence position). The exon and intron regions within the PCR product are distinguished by stippled and white boxes respectively, and the sizes of the *Aat*II digestion fragments are given.
Figure 18 is an electrophoretic agarose gel showing *lox*-1 PCR fragments (652 bp) amplified in the first step of the PCR-CAPS assay from Line G and cv Vintage genomic DNA.
Figure 19 is an electrophoretic agarose gel showing RFLP detected by PCR-CAPS in the wild-type and mutant *lox*-1 gene. The *AatII* digestion fragments of the mutant gene include a unique 313 bp restriction fragment, indicated by an asterisk.

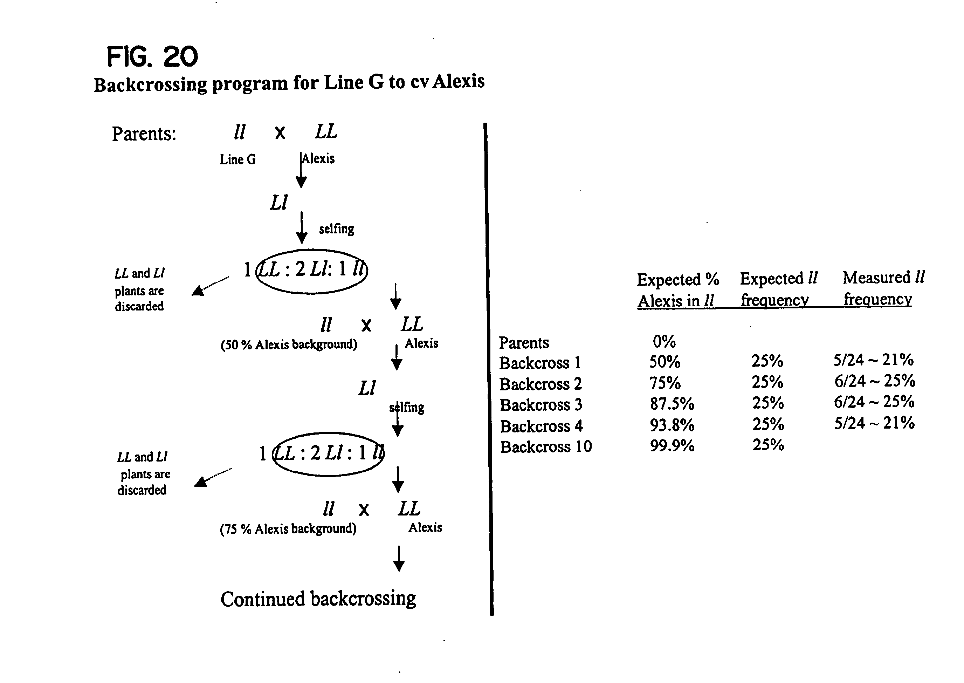

Figure 20 is a table showing a back-crossing program for the single recessive gene pair *ll* (low lipoxygenase trait) of Line G to cv Alexis. The *LL* genotype are plants expressing wild-type lipoxygenase activity (dominant allele), the *ll* genotype are plants expressing the low-lipoxygenase (recessive allele). *Ll* are heterozygous plants containing both the wild-type and the low-lipoxygenase allele. Since the low-lipoxygenase trait is a recessive trait, *Ll* plants show wild-type lipoxygenase activity. After each round of back-crossing (including self-pollination), the *ll* progeny is expected to represent 25% of the progeny. The observed frequencies of low-lipoxygenase activity are indicated. The calculated percentage of the cv Alexis genetic background having the homozygous low-lipoxygenase allele is indicated as % Alexis.
Figure 21 is an electrophoretic agarose gel showing PCR-CAPS detection of the mutant *lox*-1 gene in *ll* progeny of the Line G - Alexis back-cross program. PCR-CAPS assay on genomic DNA of Line G (Lane 2), cv Vintage (Lane 3), *ll* progeny of 3^{rd} (Lane 4) and 4^{th} back-cross (Lanes 5 - 9). DNA ladder (Lane 1). Control, backcrossed high lox line (lane 10).
Figures 22A-22B are a comparative alignment of amino acid sequences of soybean lipoxygenases LOX-1 (Gm1), LOX-2 (Gm2), LOX-3 (Gm3), and barley lipoxygenases LOX-1 (Hvl) and LOX-2 (Hv2).

### DETAILED DESCRIPTION OF THE INVENTION

Plant materials, plant products, and methods are provided for producing a beverage, such as beer, the beverage having a reduced content of the off-flavor compound trans-2-nonenal, such that the flavor stability of the beverage, e.g., beer, during storage and on exposure to elevated temperatures is improved, relative to a control beverage. More particularly, the invention provides barley plants whose developing and germinating grain produce greatly reduced activity levels of the enzyme lipoxygenase-1, denoted LOX-1, which, for example, when used in a beer brewing process, results in a beer having reduced trans-2-nonenal levels, as compared with a control barley plant.

The methods used to generate, characterize, and validate a barley plant having greatly reduced LOX-1 activity, and use of this type of barley for the production of flavor-stable beer are described below.

### 1. Definitions

As used herein, the following terms have the indicated definitions:
"Plant portion" means a plant or specific part of a plant, such as the stem, leaves, roots, flowers, seeds, grains, fruits, or buds.
"LOX-1" means lipoxygenase-1 protein; "*lox*-1" means the gene encoding LOX-1.
"Mutant barley *lox*-1" means a mutagenized barley gene encoding a mutant lipoxygenase 1 polypeptide.
"Non-mutated control" means a plant, nucleic acid, gene, polypeptide, plant portion, or plant product containing wild type gene or protein.
"Heterologous" means a non-native sequence, e.g., a sequence derived from another species, or a recombinantly engineered or synthetic sequence that differs from the native sequence.
"Plant product" means a product resulting from the processing of a plant or plant portion, and includes, for example, malt and wort.
"Acidic amino acid" means aspartic or glutamic acid.
"Basic amino acid" means histidine, lysine, or arginine.
"Polar amino acid" means threonine, serine, tyrosine, tryptophan, asparagine, or glutamine.
"Organoleptic properties" means properties appealing to the olfactory and taste senses that are analysed, for example, by a trained taste panel.
"Brewed product" means a product prepared by mashing, boiling, and fermenting, e.g., beer.
"Reduced trans-2-nonenal" means less than about 50%, as compared with wild-type (control) conditions.

### 2. Lipoxygenase Activity

Lipoxygenase enzymes catalyze the oxidation of polyunsaturated fatty acids. In barley, the isoenzymes LOX-1 and LOX-2 are known. LOX-1 primarily catalyzes 9-hydroperoxidation, whereas LOX-2 primarily catalyzes 13-hydroperoxidation of polyunsaturated octadecanoic fatty acids. The data shown in the Examples below demonstrates a correlation between barley LOX-1 9-hydroperoxidation activity and the presence of trans-2-nonenal in beer. Accordingly, barley having reduced LOX-1 activity is useful to produce beer having a reduced *trans*-2-nonenal level and/or potential as compared with a control.

### 3. Production of low lipoxygenase barley

A variety of known genetic approaches can be used to produce the plants of the invention, that is, to reduce the level of lipoxygenase 1 enzyme activity expressed in a barley plant in a stable, inheritable manner. These approaches include, but are not restricted to antisense technology and mutagenesis, such as chemical and radiation induced mutagenesis, as well as site-directed mutagenesis.

**Barley transformation.** Barley can be transformed with various nucleic acid molecules designed to manipulate *lox*-1 gene expression or alter the architecture of the *lox*-1 gene. Various methods, for example, *Agrobacterium* tumofaciens-mediated transfer (Tingay et al, 1997, Plant J., 11: 1369-1376), particle bombardment (Wan and Lemaux, 1994, Plant Physiol., 104: 37-48, or polyethylene glycol (PEG)-mediated DNA uptake (Funatsuki and Kihara, 1995, Theor. Appl. Genet., 91:707-712), can be used to successfully introduce nucleic acids into a barley cell, for example into a protoplast, callus, or an embryo.

Various promoters can be used to drive expression of the gene of interest. For expression of *lox*-1*-*containing vectors, including antisense sequences, the native *lox*-1 promoter region can be used. The promoter sequence of *lox*-1 is contained in nucleotides 2602 - 3511, which includes the 5' UTR of EMBL accession no. U83904. Alternatively, promoters that drive expression of the gene of interest constitutively, for example the *Ubi*.1 maize ubiquitin promoter, can be used (Wan and Lemaux, *Supra;* Kjærulff et al., in P. Mathis, Ed., 1995, Photosynthesis: from Light to Biosphere, Vol. II, 151-154). Expression vectors can also contain a transcription termination region, for example, the 3' terminator of the nopaline synthase gene (3'-*nos*) (Bevan, et al, 1983, Nucl. Acids Res., 11: 369-385) has been fused to genes expressed in transgenic barley (Wan and Lemaux, *Supra;* Funatsuki and Kihara, *Supra*).

Expression vectors can also contain a gene that allows for selection of transformed cells when the vector has been successfully integrated in the cell. These genes can encode antibiotic or herbicide resistance genes, for example the neomycin phosphotransferase (*npt*) or the phosphinothricin acetyl transferase (*bar*) gene. When expressed, such resistance genes allow for growth of the transformed cell in neomycin - or bialaphos-containing media, respectively (See, for example, Wan and Lemaux, *Supra;* Funatsuki and Kihara, *Supra*; Kjærulff et al., in P. Mathis, *Supra*).

Following transformation, cells can be grown in selective media for a period of time and then cultured to allow for the formation of shoots, followed by root systems, and then plantlets. A successful barley transformation procedure was developed by Funatsuki and Kihara, (Supra), where transformation of barley protoplasts by PEG with neomycin phosphotransferase-containing expression vectors and subsequent selection in neomycin yielded fertile plants containing the transgene. The transgene was shown to integrate into the genome and most of the transgenic plants expressed the protein encoded by the transgene. These transgenic plants also were able to transmit and express the transgene following crosses.

It is understood that a variety of transformation methods, expression vectors, promoters, selectable markers, and the like are known and useful for transformation of barley.

**Barley mutagenesis**. The *lox*-1 gene can be targeted for site-specific mutagenesis using chimeric RNA/DNA oligonucleotides. These chimeric RNA/DNA oligonucleotides have been shown to successfully introduce mutations in plant cells (Zhu et al., 1999, Proc. Natl. Acad. Sci. 96: 8768-8773; and Beetham et al., 1999, Proc. Natl. Acad. Sci. 96: 8774-8778) and mammalian cells (Yoon et al., 1999, Proc. Natl. Acad. Sci. 93:2071-2076) at desired locations. The chimeric RNA/DNA oligonucleotides can be transformed into the barley protoplasts or cells of interest in a variety of ways, for example using the PEG-mediated or particle bombardment-mediated transformation methods described above. The individual protoplasts or cells can then regenerated by tissue culture to whole fertile plants, and the mutational event can be confirmed and followed, for example using a PCR-based approach as detailed in the Examples below.

This site-directed mutagenesis method can be applied to mutate specific residues in the *lox*-1 gene. The *lox-1* gene can be mutated at one or more nucleotide position in the promoter region to downregulate or abolish *lox*-1 transcription. Specific mutagenesis can also be applied to introduce changes in the *lox*-1 coding region that, for example, reduce the enzyme's activity. Such mutations include, but are not limited to, insertions, deletions, and substitutions resulting in a frameshift, truncation of the LOX-1 protein, and/or alteration of the neutral and hydrophobic nature of the enzyme's substrate cavity.

**Antisense expression.** Reduction in *lox*-1 expression can also be accomplished by expression of a *lox*-1 antisense construct in the barley cells. Methods for the expression of antisense constructs in barley to reduce the expression of a targeted protein have been reported, for example, in Gilpin, M.J. et al., 1998, In: Photosynthesis: Mechanisms and Effects, G. Garab, ed., Vol. IV, 2983-2986; Kjærulff et al., 1995, In: Photosynthesis: from Light to Biosphere, P. Mathis, Ed., Vol. II, 151-154.

Barley cells can be transformed with an expression construct containing an antisense nucleic acid sequence. The expression construct produces an antisense RNA molecule capable of specifically binding to at least a portion of the mRNA produced from the wild type *lox-1* gene, through complimentary base pairing, and capable of disrupting the splicing of the pre-mRNA or translation of this mRNA. A constitutive or tissue/temporal specific promoter, for example, the barley *lox*-1 promoter described above, can drive expression of the antisense nucleic acid sequence.

**Chemical mutagenesis**. The chemical mutagen sodium azide (NaN₃) has commonly been used for barley mutagenesis and is known to induce stable mutations in the DNA (deoxyribonucleic acid) sequence of the barley genome (Olsen et al., 1993, Proc. Natl. Acad. Sci. USA, 90: 8043-8047). Other chemical mutagens, for example, ethyl methanesulfonate (EMS), azidoglycerol (AG, 3-azido-1,2-propanediol), methyl nitrosourea (MNU), and maleic hydrazide (MH) can also be used to induce DNA mutations (Rank, J. et al., 1997, Mutat. Res. 390:121-7), as can UV irradiation.

As shown in the Examples below, the grain of the barley cultivars (cv) Vintage and Caruso were treated with sodium azide and propagated by self-fertilization through to the 3^{rd} generation (M3).

### 4. Identification and Selection of Low Lipoxygenase Barley

Identification and selection of barley plants having reduced lipoxygenase isoenzyme activity in the grain can be achieved, for example, by analysis of lipoxygenase activity. Enzymatic assays can be used to determine the activity of the two major lipoxygenases known to be present in either mature or germinating grain, LOX-1 and LOX-2. Such assays should distinguish LOX-1 activity from that of LOX-2.

One selective assay of LOX-1 and LOX-2 is based on the oxidation of a poly-unsaturated fatty acid by lipoxygenase and the spectrophotometric detection of the hydroperoxide product of such oxidation. The specificity of this assay for LOX-1 takes advantage of the comparative insensitivity of LOX-1 to an inhibitor, for example, NDGA, relative to LOX-2.

Selective assay can also be achieved using immunoprecipitation to selectively remove LOX-1 or LOX-2 from the assay. Specific anti-LOX-1 and anti-LOX-2 antibodies, for example, monoclonal antibodies, can be prepared from purified LOX-1 or LOX-2 as described in Holtman et. al, 1996, *Supra.*

These assay methods can be adapted for microtiter plate assay procedures, or other known repetitive, high throughput assay formats, allowing the rapid screening of many samples. These assays can be validated for screening leaf tips of germinating grain in a non-destructive manner, such that seedlings selected in the screen can be further propagated.

The loss of LOX-1 activity in putative mutants can be confirmed by assay of enzymatic activity. For example, grain extracts can be incubated with linoleic acid and the oxidation products of linoleic acid analyzed, for example, by reverse phase HPLC. The relative amounts of 9-HPOD and 13-HPOD formed from linoleic acid provides a measure of LOX-1 activity, whose major product is 9-HPOD.

As shown in the Examples below, approximately 20,000 grain of the M3 generation ofmutagenized cv Vintage and cv Caruso were screened for LOX-1 and LOX-2 activity by oxidation assay in the presence of inhibitor and also by immunoprecipitation assays. Using these screening methods, a mutant in cv Vintage was found having a major reduction in LOX-1 activity, and was denoted Line G. The mutant phenotype was inherited in the M4 and M5 generations.

Seed produced from the Line G barley was deposited on January 4, 2001, with the National Collections of Industrial, Food and Marine Bacteria (NCIMB), 23 St. Machar Drive, Aberdeen, AB243RY, Scotland, UK, under the terms of the Budapest Treaty, as Accession Number: NCIMB 41078.

### 5. Genetic Sequences

A precise description of the genotypic alteration that accounts for the low-lipoxygenase phenotype in barley plants of the invention is useful for identifying plants having this genetic alteration and for crossing this genetic character into other barley cultivars in a breeding program. A variety of known molecular and biochemical methods can be used to determine the genetic basis for the low lipoxygenase phenotype.

It is generally recognized that both cis-acting and trans-acting genetic sequences can determine the expression of a given gene in the genome and the activity of the gene product. Control points in gene expression include the regulation of the timing, tissue-specificity and rate of gene transcription, the stability of the transcript and the rate of transcript translation. Both the level of gene expression and the stability and specific activity of the encoded enzyme will determine the level of enzyme activity detected in a tissue.

Alterations in a plant gene sequence can be determined by DNA sequencing of known relevant parts of the genome, while Northern analysis provides a tool to monitor stable transcript levels in a given plant tissue. Enzyme expressed in plant tissue can be evaluated by extracting the enzyme from the tissue and measuring the enzymatic activity.

As shown in the Examples below, the identity of the genetic changes that determine the low-lipoxygenase phenotype of the Line G mutant induced in cv Vintage were determined in the following manner. The structural gene encoding the LOX-1 protein, both in the parent cv Vintage and in the Line G, was amplified by the polymerase chain reaction (PCR), and the upstream promoter sequences, which regulate expression of the gene, as well as the entire coding sequence, comprising intron and exon sequences, were sequenced.

Comparison of the nucleotide sequences of the *lox*-1 gene from Line G and from wild-type cv Vintage revealed 2 nucleotide substitutions in 2 exons, of which one (at position + 2347) led to a non-conservative amino acid substitution (Glycine³⁶⁸→ Aspartate) in the expressed protein.

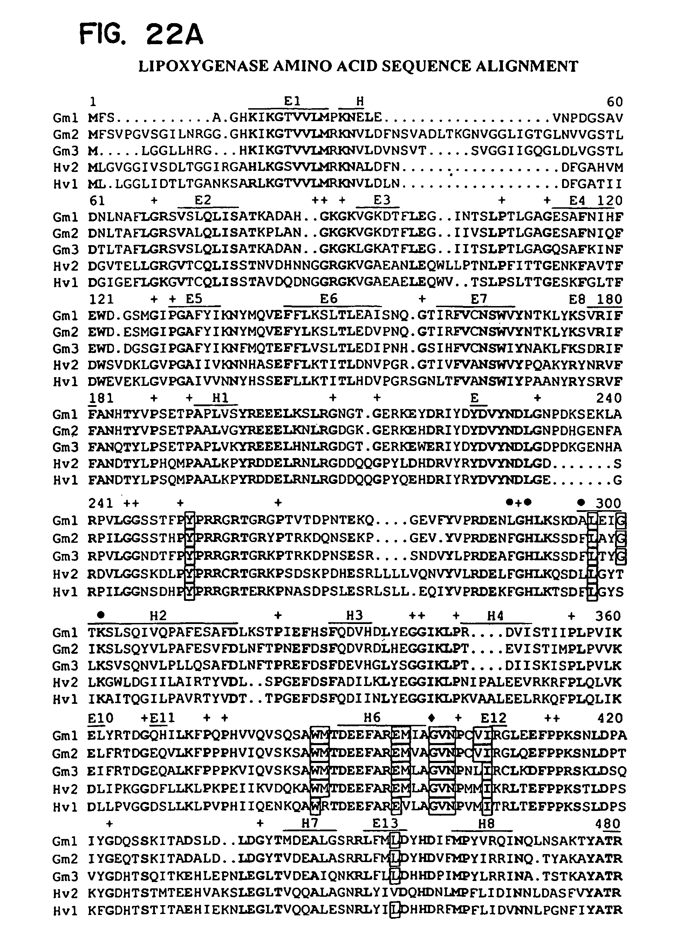

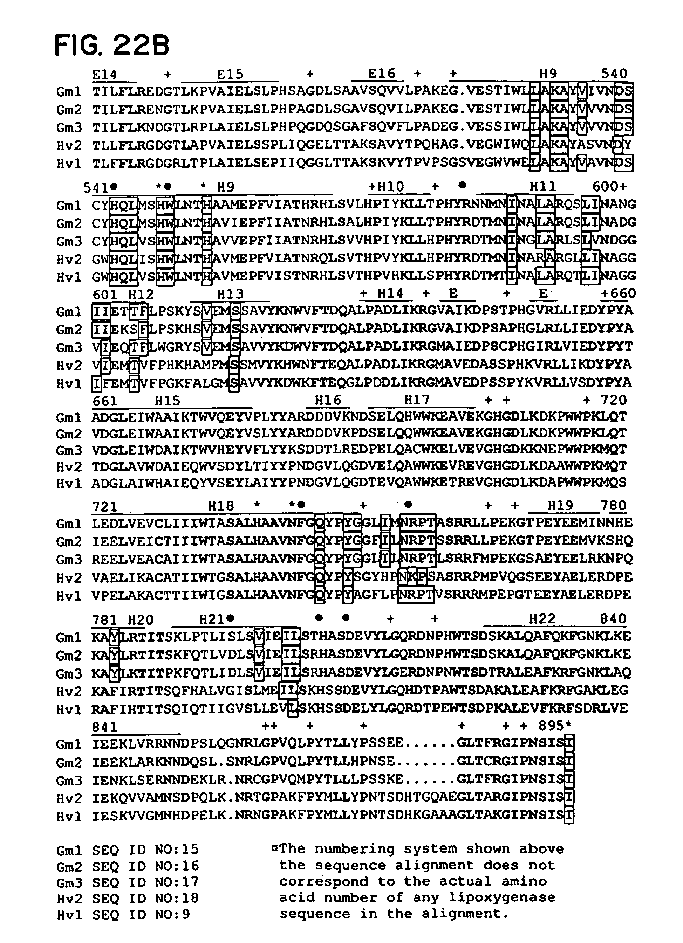

Figure 22 shows an alignment of soybean (Gm: *Glycine max L*) lipoxygenases LOX1 (Acc. No. P08170), LOX2 (Acc. No. P08170), LOX3 (Acc. No. AAB41272) and barley (hv: *Hordeum vulgare*) lipoxygenases LOX1 (Acc.No. P29114) and LOX2 (Acc. No. AAB70865.1). Conserved amino acid residues and conservative substitutions of charged residues are shown in bold. Secondary structure assignments for LOX3 of soybean *Glycine max,* where H=alpha helices and E=Beta strands, are shown above the alignment, and residues relevant to enzyme function (identified by an asterix or filled circle) are shown, as described in Skrzypczak-Jankun et.al., 1997, Proteins 29:15-31.

Amino acid residues that participate in non-heme iron binding or essential for catalysis (*) in soybean LOX3 include: H₅₁₈, H₅₂₃, H₇₀₉ [3 N atoms]; N₇₁₃, I₈₄₇. The equivalent residues in barley LOX 1 are H₅₁₇, H₅₂₂, H₇₀₈, N₇₁₂, and I₈₆₂, Residues in soybean LOX3 with a predicted role in catalysis(•) are: H₂₆₆, H_{513,} H₇₇₆, F₂₆₄, F₂₇₂, F₇₁₄, W₅₁₉, R_{552,} R₇₂₆, D₇₆₆, D₇₇₉, K₂₇₈. The equivalent residues in barley LOX1 are: H₂₆₁, H₅₁₂, H₇₇₅, F₂₅₉, F₂₆₇, F₇₁₃, W₅₁₈, R₅₅₁, R₇₂₅, D₇₇₈, and K₂₇₃.

Proline (P₈₆, ₁₀₉, ₁₆₇, ₁₇₁, ₂₂₃, ₂₃₄, ₂₉₁, ₃₁₁, ₃₂₄, ₃₄₃, ₃₄₅, ₃₇₁, ₃₈₁, ₃₈₂, ₄₈₆, ₅₄₁, ₅₄₈, ₆₀₀, ₆₁₆, ₆₂₇, ₆₈₅, ₇₂₆, ₇₃₄, ₇₈₈, ₈₂₉, ₈₃₃, ₈₃₉, ₈₅₇) and glycine (G₄₉, ₆₇, ₆₈, ₇₀, ₉₁, ₁₀₇, ₁₃₇, ₁₈₇, ₁₉₂, _{210, 217}, ₂₁₈, ₂₆₀, ₃₀₆, ₃₀₇, ₃₃₆, ₃₉₂, ₄₀₉, ₄₅₈, ₄₇₄, ₄₉₀, ₅₆₉, ₆₀₇, ₆₇₄, ₆₇₆, ₇₂₀, ₇₃₆, ₇₈₃, ₈₂₈, ₈₅₀, ₈₅₅) residues (+) located in loops and helix-capping positions in protein secondary structures, may facilitate sharp turns and folding of the peptide backbone.

Alignment of related plant lipoxygenases indicated that the Glycine-368 in barley LOX-1 is strongly conserved. Furthermore, this residue, which corresponds to Glycine-353 in soybean Low-1, is one of 35 highly-conserved residues out a total of 58 residues that line the substrate cavity II of the enzyme, as seen from its crystal structure. These conserved residues are highlighted (boxes) in the alignment of plant lipoxygenase sequences shown in Figure 22 (Minor et.al., 1996, Biochemistry 35:10687-10701), and include the following barley LOX-1 residues: Y₂₂₄, L₂₆₈, W₃₅₅, E₃₆₄, G₃₆₈, V₃₆₉, N₃₇₀, I₃₇₄, L₄₂₄, L₄₉₉, K₅₀₁, A₅₀₂,V₅₀₄, D₅₀₈, S₅₀₉, H₅₁₂, Q₅₁₃, L₅₁₄, H₅₁₇, W₅₁₈, H₅₂₂, I₅₅₆, L₅₅₉, A₅₆₀, L₅₆₄, I₅₆₅, I₅₇₀, T₅₇₄, S₅₈₅, Q₇₁₅, Y₇₁₈, N₇₂₄, R₇₂₅, P₇₂₆, T₇₂₇, I₇₇₂, and I₈₆₂. All but 7 of the 35 conserved residues are neutral or hydrophobic residues. The substitution of a charged residue at position Glycine - 368 in barley or at another conserved neutral or hydrophobic residue lining the substrate cavity II, is likely is likely to disturb the structural and functional properties of the enzyme. The G→D₃₆₈ mutation in barley Line G LOX1( ◆) is located between alpha-helix H6 and beta-strand E12.

As shown in Figure 22, the lipoxygenase family of enzymes shares a high degree of sequence conservation, which is reflected in their conserved secondary structure, determined for several members of the plant lipoxygenase family including soybean LOX1 and LOX3 (Skrzypczak-Jankun *et al.,* 1997, *supra*). Barley LOX1 shares 56.9% sequence identity and 67.8% sequence similarity with soybean LOX3. Several amino acid residues in the soybean LOX3 isoenzyme have been identified as ligands for the non-heme iron, or are suggested to be essential for its activity (denoted by * •). In view of the high sequence conservation between the barley LOX1 and the soybean LOX3, it is reasonable to predict that residues in the barley LOX1 sequence that are homologous to those identified as important for the function of LOX3 may also be essential for enzymatic activity. Thus, non-conservative amino acid substitutions at any of these positions, including substitutions of those residues in barley LOX1 corresponding to the 35 highly conserved residues of soybean LOX3 that line the substrate cavity and in other positions essential for enzyme activity, are likely to reduce lipoxygenase activity.

The amino acid residues proline and glycine are known to facilitate turns in a peptide backbone when they are located between secondary structural elements, which allow a protein to assume a folded tertiary structure. Proline and glycine residues are also common in helix capping motifs (Parker and Hefford, 1997, Protein Eng., 10: 487-496, http://www.expasy.ch). The single non-conservative substitution in Line G LOX1, where a glycine located between two predicted structural elements was replaced by aspartate, led to a significant loss of enzyme activity. It is thus predicted that mutation in the *LOX*-1 gene causing a non-conservative amino acid substitutions at one or more of the proline or glycine residues in the barley LOX1, located in regions outside the structural elements, may similarly prevent folding of the native protein and consequently reduce the activity of the encoded enzyme.

Thus, in one embodiment, a useful mutant barley plant of the invention having reduced lipoxygenase 1 activity contains a mutated nucleic acid sequence that alters the neutral or hydrophobic nature of the substrate cavity of the enzyme by insertion of one or more acidic, basic, or polar amino acids. The useful nucleic acid sequence [SEQ ID NO: 11] encodes a barley LOX-1 protein [SEQ ID NO: 12] having a substitution at amino acid 368 from Glycine to Xaa, where Xaa is aspartic acid, glutamic acid, histidine, lysine, arginine, threonine, serine, tyrosine, trytophan, asparagine or glutamine. One specific amino acid sequence of the barley mutant LOX-1 of the invention is that where Xaa is aspartic acid, e.g., Line G.

As shown in the Examples below, the genotypic changes in Line G had no detectable influence on *lox*-1 gene expression, but the LOX-1 activity detected in mature and germinating grain of Line G were approximately 9% of that detected in grain of the parent line, cv Vintage. In order to provide direct evidence that the amino acid mutation in LOX-1 of Line G was responsible for the low-LOX-1 phenotype, the coding sequence of Line G *lox*-1 and cv Vintage *lox*-1 were expressed transiently in protoplasts from barley aleurone, and the activity of the mutant LOX-1 enzyme was shown to be strongly reduced in comparison to the wild-type LOX-1 enzyme.

### 6. Transfer between breeding lines

The detection of alterations in genetic character of the barley plants of the invention genotype is useful to identify the presence of a specific genetic character in a barley line, and to facilitate the transfer of this character between breeding lines in a breeding program. A variety of molecular tools are available for the detection of alterations in genomic sequence. Such methods include, but are not restricted to, detection of restriction fragment length polymorphisms (Gebhardt and Salamini 1992, Int. Rev. Cytology., 135: 201-237) and quantitative PCR based detection methods such as amplification using fluorescent primers, e.g. the TaqMan primer probe systems (Ibraham et al., 1998, Anal. Chem 70, 2013-2017). The choice of detection method will depend on the specific genetic character but should preferably be rapid and provide clearly interpretable data.

As shown in the Examples below, a PCR-Cleavage Amplified Polymorphic Site assay (PCR-CAPS) was provided for the detection of the mutant lipoxygenase-1 gene of Line G. The nucleotide substitution in the *lox-1* gene in Line G at position + 2347 introduced an additional site of recognition by the *Aat*II restriction endonuclease that can be detected by the PCR-CAPS assay. Suitable detection methods for *lox*-1 are not restricted to this assay, but can equally well be based on TaqMan technology, and other known detection methods.

Also shown in the Examples below, the PCR-CAPS assay was applied to 4 generations of breeding material from a back-cross program, where the low-lipoxygenase phenotype in Line G was systematically back-crossed into cv Alexis. Inheritance of the low-lipoxygenase phentoype was shown to follow the inheritance of the *lox*-1 gene, and the phenotype was identified as recessive and only seen in lines homozygous for the *lox*-1 gene.

Accordingly, plant progeny of the invention includes breeding lines, for example, derived in a back-crossing program, that contain mutant *lox*-1 and express a low lipoxygenase phenotype.

### 7. Brewing

The barley plants of the invention, including plant parts, plant progeny, grain, and malt and wort, having low lipoxygenase 1 activity, are demonstrated herein to be useful for the manufacture of a beverage having reduced levels of free *trans*-2-nonenal over a measured period of time, or under conditions of elevated storage temperature, as compared to a beverage produced from a wild-type control barley variety. For the purpose of these comparisons the sulfite content of the beer is controlled to 5ppm or below, since it is recognised that higher sulfite levels at the time of bottling will temporarily delay the appearance of free *trans-*2-nonenal. For example, beer brewed from malt derived from the mutated barley Line G described herein, possessed stabilized organoleptic properties over a measured period of time as compared with beer brewed from malt derived from a control, non-mutated barley.

Brewing trials and evaluation of bottled beer provide the best method for evaluating the influence of different ingredients on the quality and stability of the finished beer. In order to test the influence of different barley malts, sufficient barley grain is needed to perform the malting and brewing trials on a pilot scale and semi-industrial scale. During the period of barley propagation, the field performance of the barley line can be evaluated. The malting properties of a barley line can be evaluated during pilot or industrial scale malting, and should preferably lie within national malting quality recommendations eg. the European Brewing Convention recommendations for malting quality (Analytica-EBC/European Brewing Convention, 1998, Publ. Hans Carl Getränke-Fachverlag, Nürnberg, Germany). Following pilot or semi-industrial scale brewing, the beer is packaged in brown bottles and cooled to 5°C for optimal storage. At this stage the fresh beer can be analysed by trained taste panels able to detect specific beer flavors, including the off-flavor compound trans-2-nonenal. Additionally, the beer is chemically analysed for major flavor components including *trans*-2-nonenal. These methods of beer quality analysis are then repeated on the beer following various storage conditions known to reveal the long-term storage stability of the beer, for example, forced aging treatments.

As shown in the Examples below, Line G barley was propagated in the field over several seasons in order to malt 10 tons of this line in an industrial malthouse. The control barley varieties cv Vintage and cv Nevada, both having the wild-type LOX-1 phenotype, were malted under similar conditions. The kilned malt from Line G and the control barley cultivars lay within the specifications required for the semi-industrial brewing trials.

Brewing trials were performed on a 30-hl scale and evaluation of the freshly bottled beers revealed that beers brewed from malt of both Line G and the control cultivars had a *trans*-2-nonenal content below the taste-threshold and were deemed satisfactory by a taste-panel. Two forced-aging treatments, either storage at 37°C for 7 days or storage for 6 to 12 weeks at 30°C, were used to evaluate the flavor-stability of the beer. The flavor-stability of beer brewed from Line G malt were found to be superior to that of control malt, both with respect to taste panel evaluation as well as the level of free *trans*-2-nonenal, and the improvement was found to be statistically significant.

### EXAMPLES

### Example 1

### Screening and Selection of Lipoxygenase Isoenzyme Mutants from Mutagenised Barley

### 1. Barley mutagenesis

Grains of barley, *Hordeum vulgare* cv Vintage and cv Caruso, were mutagenised with sodium azide according to a published procedure (Kleinhofs et al., 1978 Mutation Research 51: 29-35). The mutagenesis introduces point mutations in the genomic DNA that, for example, may result in amino acid changes in encoded proteins. The mutated M1 grains were propagated in the greenhouse through two generations, and the M3 grain collected for screening. The observed frequency of single gene trait mutants in the M2 generation, according to Kleinhofs *et al*., 1978, *supra*, are 1.0-2.7 mutants per 10,000 grain from the M2 generation. Since most gene mutations are recessive and only detectable in the homozygous state, the mutagenized population was screened at the M3 generation where the expected proportion of homozygous mutant grain would be higher. A mutation frequency of 0.9 - 2.3 per 10,000 grain was expected in the mutagenized material at M3.

### 2. A non-destructive assay of lipoxygenase 1 (LOX-1) and lipoxygenase-2 (LOX-2) activity in M3 mutagenized grain

A rapid screening procedure for detection of mutant barley grain with reduced LOX-1 activity was developed with the following criteria: The screening procedure should not prevent propagation of the grain/seedling; the selected grain/seedling tissue should express quantifiable levels of lipoxygenase activity; the assay should distinguish LOX-1 activity from that of LOX-2; and the assay procedure should encompass multiple samples.

The levels of total lipoxygenase activity in different tissues of the germinating grain, namely the shoot, root, and scutellum tissue of embryo and the endosperm were assayed as follows: Extracts of barley seedling tissue were prepared by homogenising the tissue in ice-cold 20 mM Tris-HCl, pH 7.5, containing 2 mM NaN₃ and 0.5 mM phenylmethylsulfonyl fluoride (PMSF), followed by removal of insoluble material by centrifugation at 1000 *g* for 10 minutes. Lipoxygenase activity in 100 µl extract was assayed at 25°C, by addition of 2.9 ml of 20 mM linoleic acid substrate, prepared by dispersing 35 µl linoleic acid (free acid, L-1376, Sigma, USA) in 5 ml H₂O containing 1% Tween 20. The reaction was followed spectrophotometrically, where the rate of increase in absorbance at 234 nm (A₂₃₄nm), due to the formation of conjugated diene in the hydroperoxide product, is proportional to the enzyme activity present. One unit of lipoxygenase activity is defined as Δ A₂₃₄ = 0.001 per minute in a 3-ml reaction, equivalent to the oxidation of 0.12 µmole linoleic acid.

The leaf tissue of grain germinated for 4 days in the dark had the highest detected levels of lipoxygenase activity *(*Holtman et al., 1996, Plant Physiology 111: 569-576). Leaf tips from 4-day seedlings were thus selected for the non-destructive lipoxygenase screening assay. The pH optimum of total barley lipoxygenase activity was tested between pH 4.5 and pH 9.0 and found to be pH 6.5. Hence a 25 mM HEPES buffer (pH 6.5) containing 0.2 M boric acid was selected for the screening assay.

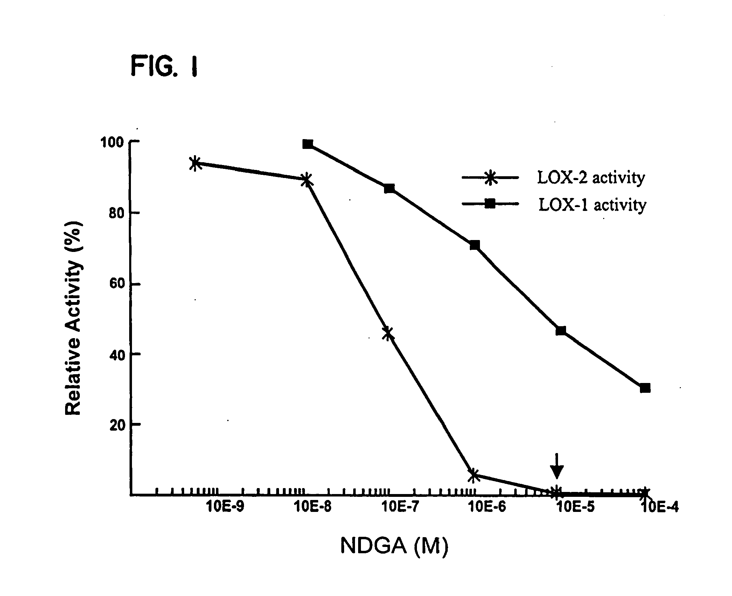

Since both LOX-1 and LOX-2 enzymes were immuno-detected in shoots of 4-day seedlings (Holtman *et al.,* 1996, *supra),* a LOX-1 and LOX-2 specific assay was used. The lipoxygenase inhibitor nordihydroguaiaretic acid (NDGA), identified by Eskin et al., 1977, Crit. Rev. Food, Science and Nutrition 9: 1-40, was found to be a selective inhibitor of barley lipoxygenases. NDGA at 1x10⁻⁵ M strongly inhibited purified barley LOX-2, while LOX-1 retained 47 % activity **(****Figure 1****)**. The selectivity of this inhibitor was tested in the leaf tip assay, by determining the ratio of 9-hydroperoxyoctadecanoid (9-HPOD) to 13-hydroperoxyoctadecanoid (13-HPOD), which result from linoleic acid oxidation by LOX-1 and LOX-2, respectively. In the lipoxygenase assay of cv Vintage leaf tips, the proportion of 13-HPOD formed fell from 24.5% to 9.5% on addition of 1·10⁻⁵ M NDGA.

A selective assay for LOX-2 activity in leaf tip extracts was based on the use of LOX-1-specific monoclonal antibody (5D2) (Holtman *et al.,* 1996, *supra*) to immunoprecipitate LOX-1 present in the extracts. The residual lipoxygenase activity detected in the extracts after LOX-1 precipitation provided a measure of LOX-2 activity. The efficiency of this immunoprecipitation (described below) was evaluated by quantifying the residual LOX-1 and LOX-2 in the extract supernatant by ELISA assay, using specific monoclonal antibodies against LOX-1 (denoted 5D2) and LOX-2 (denoted 5.8) (Holtman *et al*., 1996, *supra*). LOX-1 immunoprecipitation from extracts of cv Vintage leaf tips removed 85% of (LOX-1) protein and 15% of LOX-2 protein.

Immunoprecipitation was performed in a V-bottom 96-well plate by adding 5 µl 5D2-coated Dynabeads (Dynal) and 75 µl buffer [20 mM Tris-HCl pH 7.5, 1% v/v Bovine Calf Serum (HyClone)] to 20 µl of each leaf tip extract. The plate was incubated on a titerplate shaker (MTS4, IKA, Labor Technik) for 1 hour at 4°C. The immunoprecipitate was pelleted by centrifugation at 4°C in a Sigma 302-K centrifuge for 10 minutes at 2000 rpm. The supernatant (70 µl) from each sample was assayed for lipoxygenase activity in a flat bottom 96 well plate, as described below, but with addition of 100 µl assay buffer (25 mM HEPES, 0.2 M boric acid, pH 6.5).

The LOX-1 and LOX-2 assays were adapted for a high-throughput screening method. Leaf tips (1 cm) from eight 4 day-germinated grains were individually homogenised in 150 µl ice-cold buffer (20 mM Tris-HCl, pH 7.5) for 2 x 30 seconds in a multi-well homogeniser (Berg et al., 1992, Electrophoresis 13: 76-81). After centrifugation for 15 minutes at 3000 rpm, 40 µl of the supernatant of each extract was transferred to a flat bottom 96 well plate. To each well, 170 µl buffer (25 mM HEPES, 0.2 M boric acid pH 6.5, 1·10⁻⁵ M NDGA) and 10 µl substrate (20 mM linoleic acid) were added and then incubated for 20 minutes at 25°C. The reaction was terminated by the addition of 20 µl saturated potassium iodide solution (KI) and incubated for a further 8 minutes at 25°C. The redox reaction between hydroperoxydienes and KI yields I₂, which was monitored by its extinction maximum at 350 nm in a microplate reader (Multiskan MCC/340).

### 3. Identification of potential lipoxygenase 1 mutants in the M3 and M4 grain of mutagenised barley

Grain of the M3 generation of cv Vintage and cv Caruso was stored at 45°C for 6.5 days to break dormancy, ensuring a 95% germination frequency. M3 grain of cv Vintage (9318) and cv Caruso (9633) was germinated and screened for lines whose LOX-1 activity was 15% or less of wild-type grain. The putative mutant lines (50 cv Vintage and 42 cv Caruso lines) were propagated to the M4 generation, harvested, and the germinated grain re-screened. The mutant LOX-1 phenotype was confirmed in one cv Vintage line and six cv Caruso lines, after measuring the lipoxygenase activity in extracts of 5 leaf-tips from each line. When the LOX-1 and LOX-2 activities in germinating embryos of these 7 putative mutants were examined, only the cv Vintage mutant (denoted Line G) showed a major reduction in LOX-1. In mature quiescent grain, lipoxygenase activity present in the embryo is almost exclusively LOX-1 activity, due to the differential expression pattern of the two isoenzymes (Schmitt and van Mechelen, 1997, Plant Sci. 128: 141-150). The total lipoxygenase activity in extracts of embryos from Line G mature dry grain (M5 generation) was 0.06±0.04 U/mg protein in comparison to 0.74 ±0.44 U/mg protein in cv Vintage embryo extracts, as determined by the spectrophotometric lipoxygenase assay described in section 2 of Example 1. The residual lipoxygenase activity in mature embryos of Line G in both the M4 and M5 generations was found to be approximately 9 % of the parental line.

### Example 2

### Line G is a cv Vintage Mutant with a Low-Lipoxygenase Phenotype

The agronomic properties and mutant phenotype of Line G were analysed in material of the M5 generation. Initial analyses were conducted to confirm that the analysed M5 material was homozygous for the mutant phenotype. The low LOX-1 phenotype in Line G, detected in the M3 generation, could result from a dominant or a recessive mutation. If the Line G selected at the M3 generation was heterozygous for a dominant mutation, then subsequent generations would show segregation for the phenotype. The lipoxygenase activity in 26 individual Line G embryos from quiescent grain of the M5 generation was measured and compared to cv Vintage wild type embryos. The lipoxygenase activity in all Line G embryos was very low, with an average of 0.06 ±0.04 U lipoxygenase per mg protein, compared to 0.74±0.44 U lipoxygenase per mg protein in wild type cv Vintage embryos. These data confirmed that Line G in the M5 generation was homozygous for the low lipoxygenase trait.

### 1. Line G has a wild type plant growth physiology and grain development.

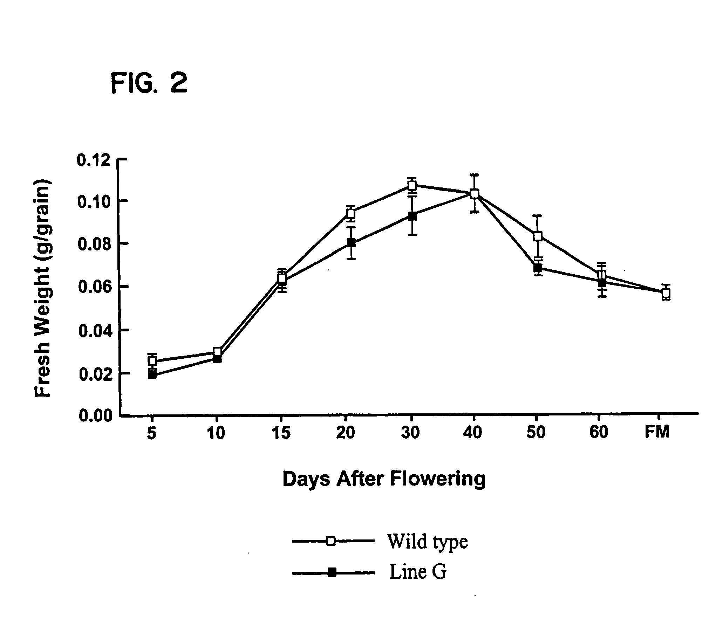

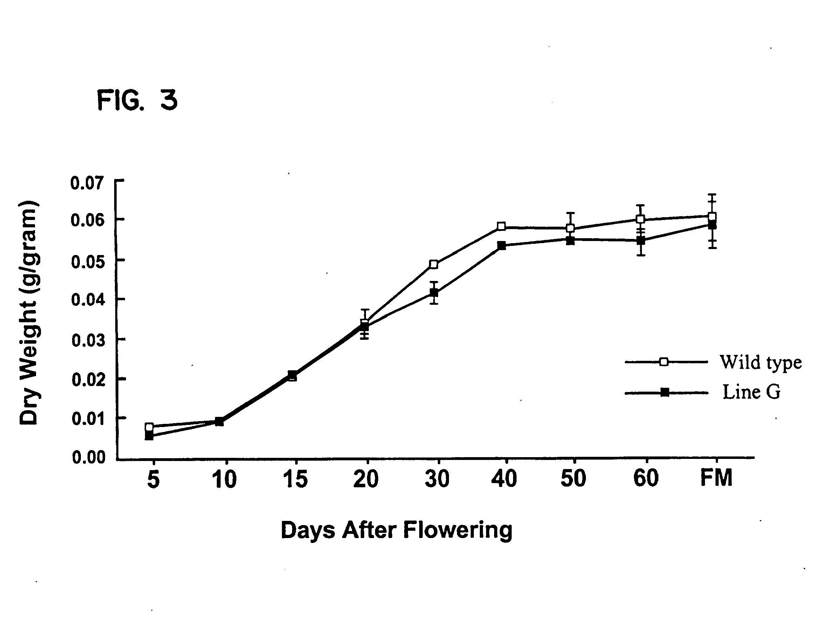

Line G and cv Vintage grain were germinated and grown in a climate chamber under 16 hours light at 15°C and 8 hours dark at 12°C at a relative humidity of 80%. The growth characteristics of Line G and cv Vintage plants were similar with regard to plant height, number of tillers per plant, the onset of flowering and number of grains per spike. The fresh weight (**Figure 2**) and dry weight (**Figure 3**) of grain of Line G and wild type cv Vintage during development from 5 days after flowering (DAF) until full maturity, approximately 90 DAF, were very similar.

### 2. Line G grain have a low-lipoxygenase 1 phenotype throughout development

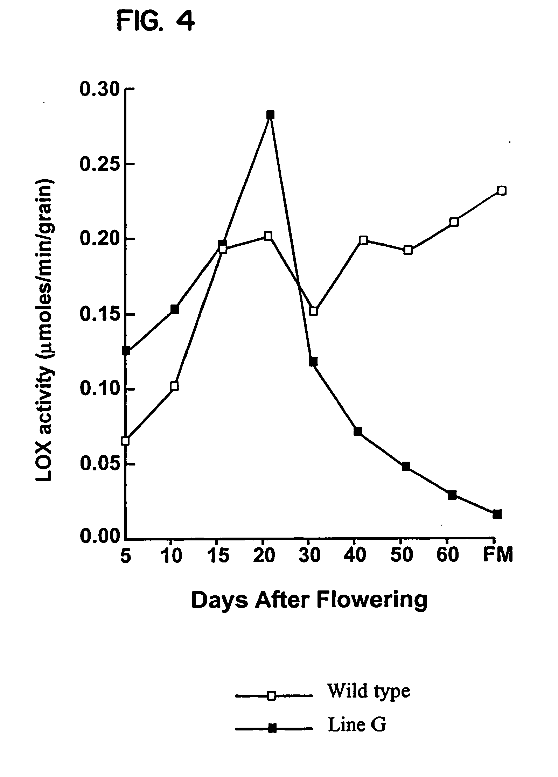

Lipoxygenase activity was measured in extracts of developing barley grain of Line G (M5 generation) and wild type cv Vintage. Grain was homogenised in ice-cold 20 mM Tris-HCl buffer pH 7.5 containing 0.1% (v/v) Nonidet P-40, a non-ionic detergent that enhances lipoxygenase extraction, and centrifuged at 15,000 ***g*** for 20 minutes to remove insoluble material. Lipoxygenase activity in the extracts was measured polarographically in 200 µl oxygen-saturated buffer (0.2 M boric acid, 25 mM HEPES, pH 6.5) containing 1.2 mM linoleic acid at 25°C, using a Clark-type electrode to measure oxygen consumption. Lipoxygenase activity increased during the first 20 days of grain development in both Line G and wild-type grain, but only in Line G did the activity level fall during grain maturation (**Figure 4**).

The relative amounts of 9-HPOD and 13-HPOD formed during linoleic acid oxidation provides a measure of the levels of LOX-1 and LOX-2 activity in the grain extracts. In this case Nonidet P-40 was omitted from the grain extraction buffer to avoid the co-extraction of hydroperoxide-consuming enzymes. The extracts (100 µl), mixed with 10 ml 50 mM phosphate buffer pH 6.5 containing 200 µM linoleic acid, were incubated for 20 minutes. The reaction was terminated by adjusting the pH to 3.5, and an internal standard was added. The hydroperoxides formed in the assay were bound on an octadecyl solid phase column (Bakerbond, Baker) and eluted with methanol. The 9-HPOD and 13-HPOD were then separated by reverse phase HPLC on a C-18 column with an isocratic elution solvent (tetrahydrofuran:Methanol:H₂O:acetic acid; 25:30:44.9:0.1 (v/v) adjusted to pH 5.5 with concentrated ammonia) at a flow rate of 0.5 ml/minute as described by Aarle et al., 1991, FEBS Letters 280: 159-162. Hydroperoxides were detected at 234 nm and the HPOD peaks were corrected against the internal standard, prostaglandin B2.

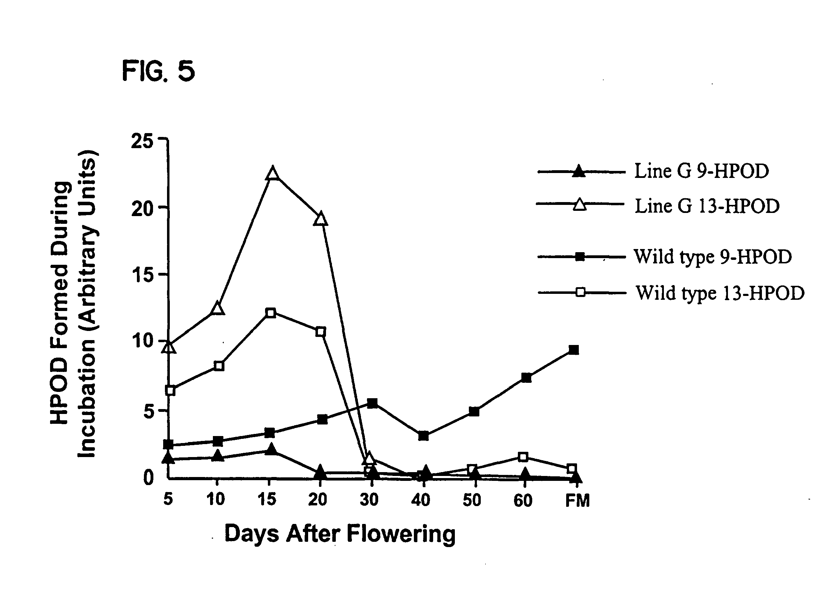

**Figure 5** shows that 13-HPOD was the major product of lipoxygenase activity present in grain during the first 20 DAF, while 9-HPOD was formed by lipoxygenases active during grain maturation. While both Line G and wild-type grain extracts shared a similar profile of 13-HPOD synthesising activity, Line G did not show the wild-type rise in 9-HPOD synthesising activity. These data are consistent with a loss of LOX-1 activity in maturing Line G barley grain.

### 3. Line G grain have a low-lipoxygenase 1 phenotype on germination

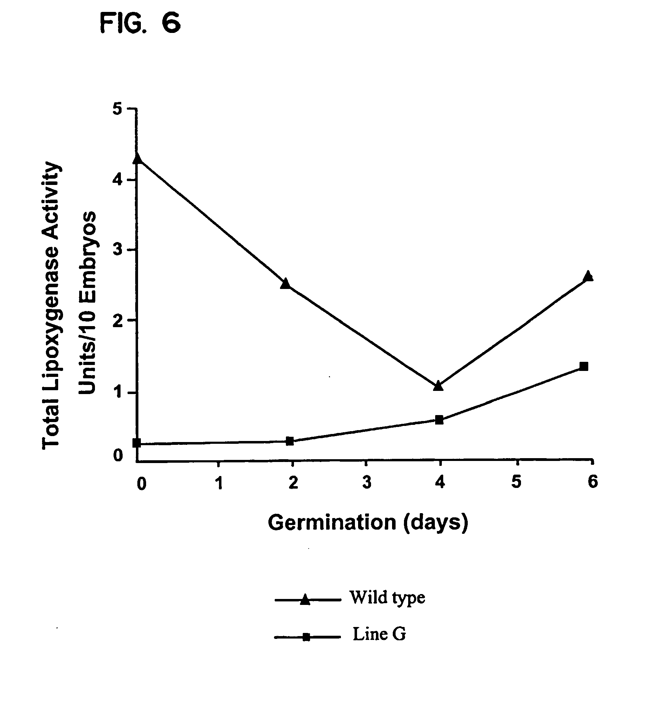

Total lipoxygenase activity in extracts of embryos of grain germinated at 15°C was assayed as described in Example 1. The lipoxygenase activity present in quiescent wild-type grain declined during the first 4 days of germination and then increased (**Figure 6**). In Line G, lipoxygenase activity in quiescent grain was very low but increased after 4 days.

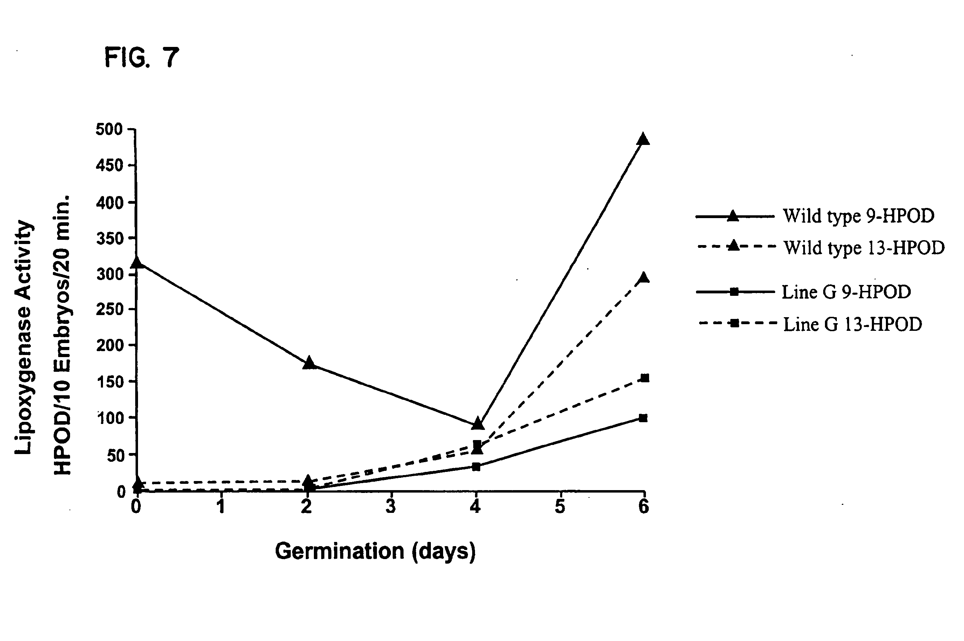

Analysis of the HPODs formed by the lipoxygenase activity in germinating embryos showed that 9-HPOD was the major product of lipoxygenases present in quiescent wild-type grain (**Figure 7**). The level of 9-HPOD formation fell with the decline in lipoxygenase activity in the extracts. The rise in lipoxygenase activity after 4 days was accompanied by the formation of both 9-HPOD and 13-HPOD. The low lipoxygenase activity in Line G quiescent grain was associated with an absence of HPOD formation, while the rise in activity after 4 days mainly produced 13-HPOD. These data provide evidence that LOX-1 activity leading to the formation of 9-HPOD is greatly reduced in the embryos of both developing, quiescent and germinating barley grain of Line G, while LOX-2 activity leading to formation of 13-HPOD is unchanged in Line G.

### Example 3

### Line G has a Mutant Lipoxygenase 1 Gene (lox-1) Causing a Low Lipoxygenase Phenotype

The molecular basis for the low-LOX-1 phenotype of Line G was investigated in order to provide a complete description of the mutant. The following analyses were performed to provide a complete characterization of the phenotype:

### 1. Lipoxygenase-1 is synthesised in the developing and germinating grain of Line G

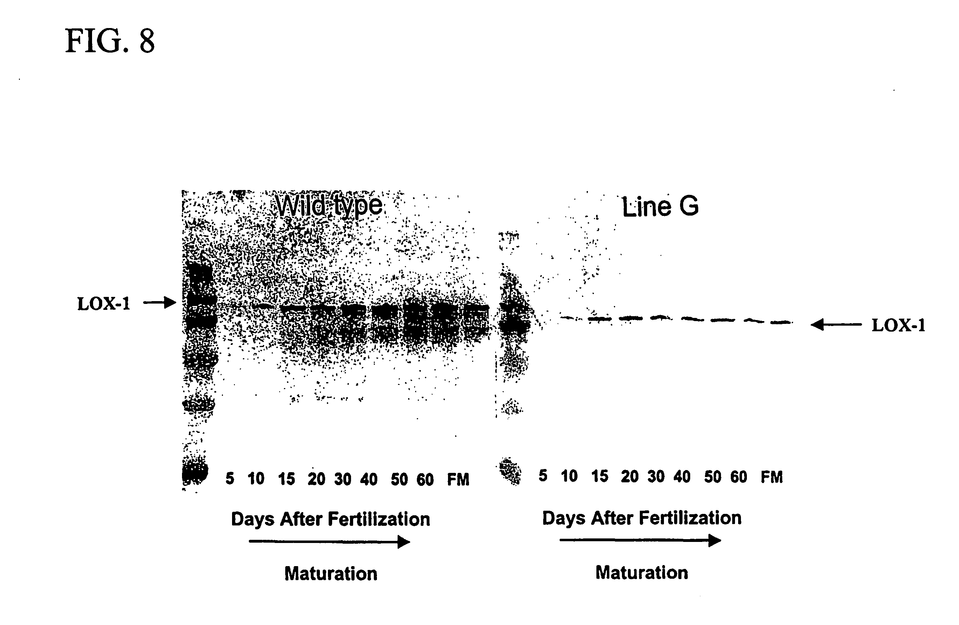

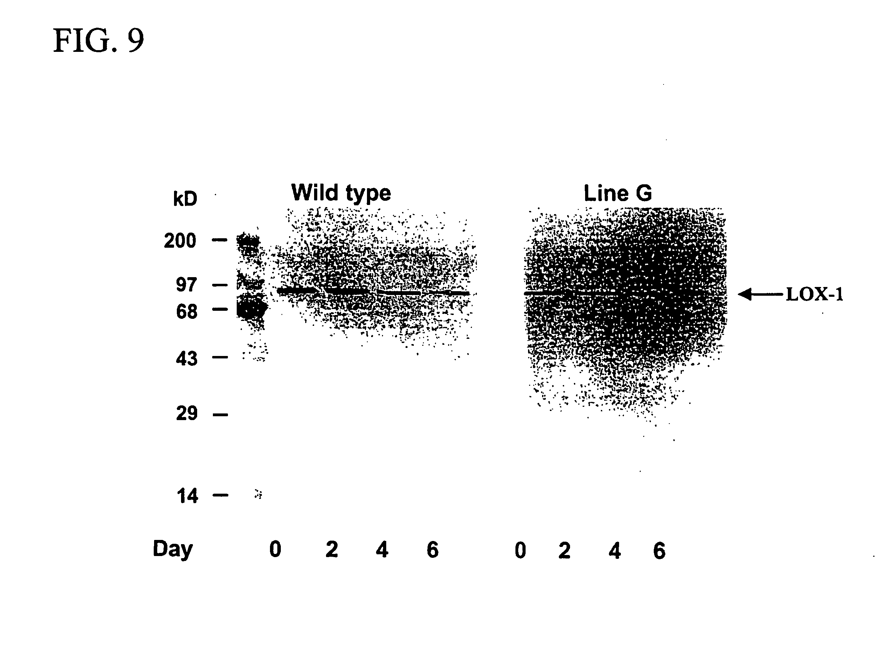

Western blot analysis of extracts of embryos from developing and germinating barley grain were performed in parallel with the measurement of lipoxygenase activity, as described in Example 2. The crude extracts were separated by sodium dodecyl sulphate-polyacrylamide gel electrophoresis (SDS-PAGE) according to Laemmli, 1970, Nature 227: 680-685. The separated proteins were transferred to nitrocellulose by semi-dry blotting, according to Towbin et al., (1979) Proc. Natl. Acad. Sci. USA 76: 4350-4354. The blot was probed with the LOX-1 specific monoclonal antibody, 5D2, as described Holtman et al., 1996, Plant Physiology 111: 569-576, at 500x dilution, followed by incubation with goat anti-mouse antibody coupled to alkaline phosphatase, and detected with the alkaline phosphatase substrates nitro blue tetrazolium and 5-bromo-4-chloro-3-indolyl-phosphate as described by Holtman et al., 1996, Plant Physiol. 111: 569-576. The Western analyses revealed that LOX-1 protein was detected in developing grain from 10 DAF in cv Vintage embryos and the level increased during grain maturation (**Figure 8**). The protein was also present in the embryo of cv Vintage quiescent grain but declined slowly during germination (**Figure 9**). Although LOX-1 is recognised in extracts of Line G embryos and migrates in SDS-PAGE as a protein of similar size to cv Vintage LOX-1, the immunodetectable levels of the protein in Line G were slightly lower than in cv Vintage.

### 2. The lox-1 gene is expressed in the developing and germinating grain of Line G

Total RNA was isolated from embryos of developing and germinating barley grain, according to the procedure of Hensgens and van Os-Ruygrok, 1989, Rice Genet. Newslett. 6: 163-168, in parallel with the measurement of lipoxygenase activity, described in Example 2. The RNA samples (7.5 µg) were separated on denaturing agarose gels and Northern blotted as described by Sambrook et al., 1989 in Molecular Cloning, a Laboratory Manual, Cold Spring Harbour Laboratory Press, NY. The blots were hybridised with a ³²P-labelled probe generated from the barley 3' untranslated region, nucleotides 2659-2801 [**SEQ ID NO:1**], of the *lox* 1 cDNA (EMBL Accession no. L35931) as described by Holtman et al., 1996 Plant Physiol. 111: 569-576, using the Amersham Random Prime Kit.

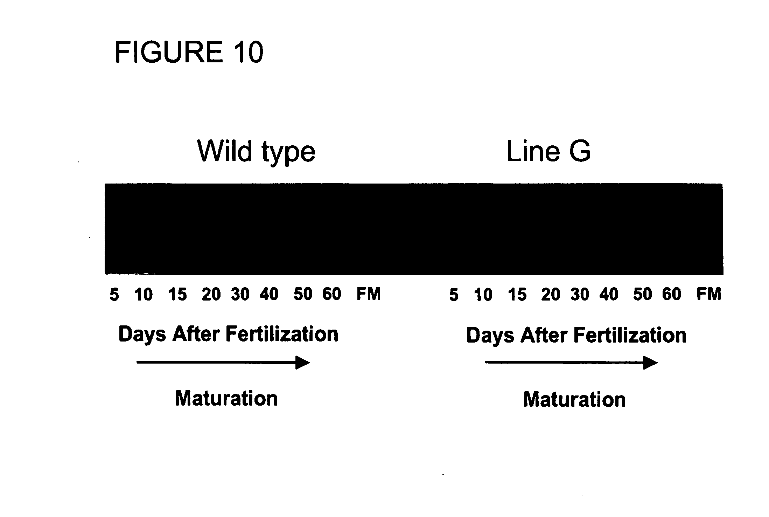

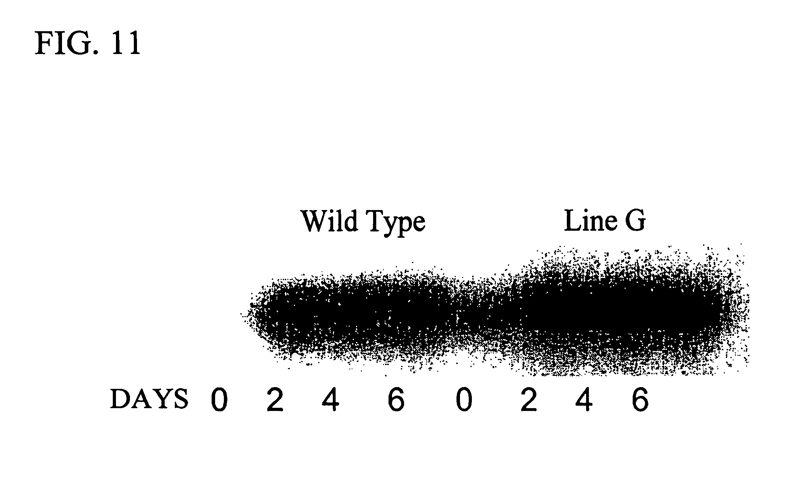

*Lox*-1 transcripts encoding LOX-1 were detected in embryos of developing and mature cv Vintage and Line G grain from 30 DAF (**Figure 10**). The level of *lox*-1 trancripts increased during germination in both cv Vintage and Line G embryos, indicating *de novo* expression of the *lox*-1 gene (**Figure 11**). Since the detectable levels of *lox*-1 transcripts were similar in Line G and cv Vintage embryos, neither reduced *lox*-1 transcription or transcript stability can account for the low-lipoxygenase phenotype of Line G.

### 3. The lox-1 gene of Line G encodes a mutant form of lipoxygenase-1

The nucleotide sequence of the *lox-1* gene of Line G and cv Vintage were analysed and compared in order to determine the molecular basis for the low-LOX-1 phenotype of Line G, which is characterised by normal transcription of the *lox-1* gene, but reduced accumulation and activity in the expressed lipoxygenase enzyme in grain.

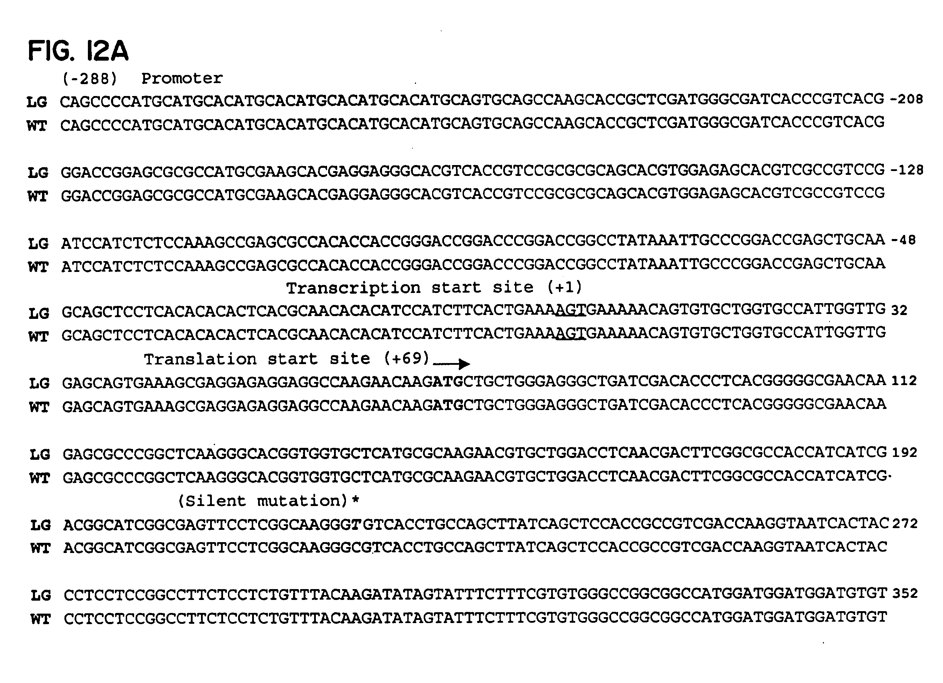

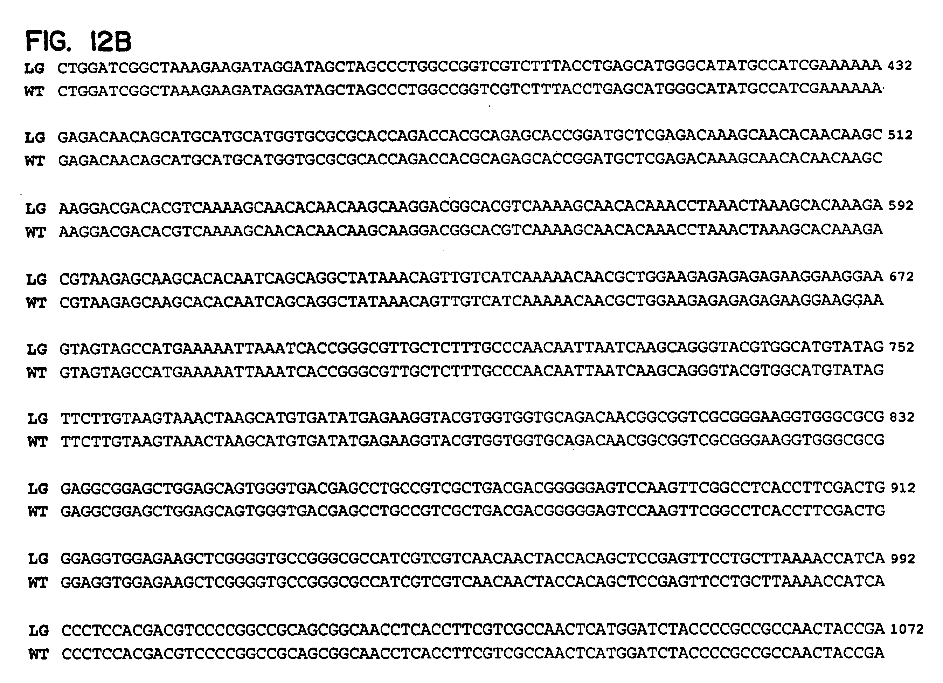

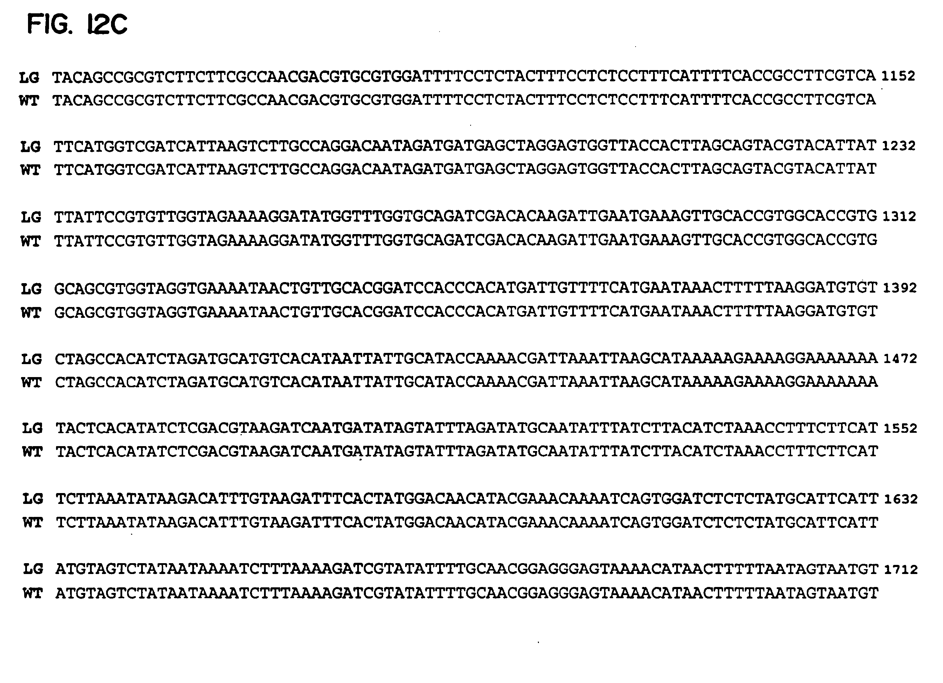

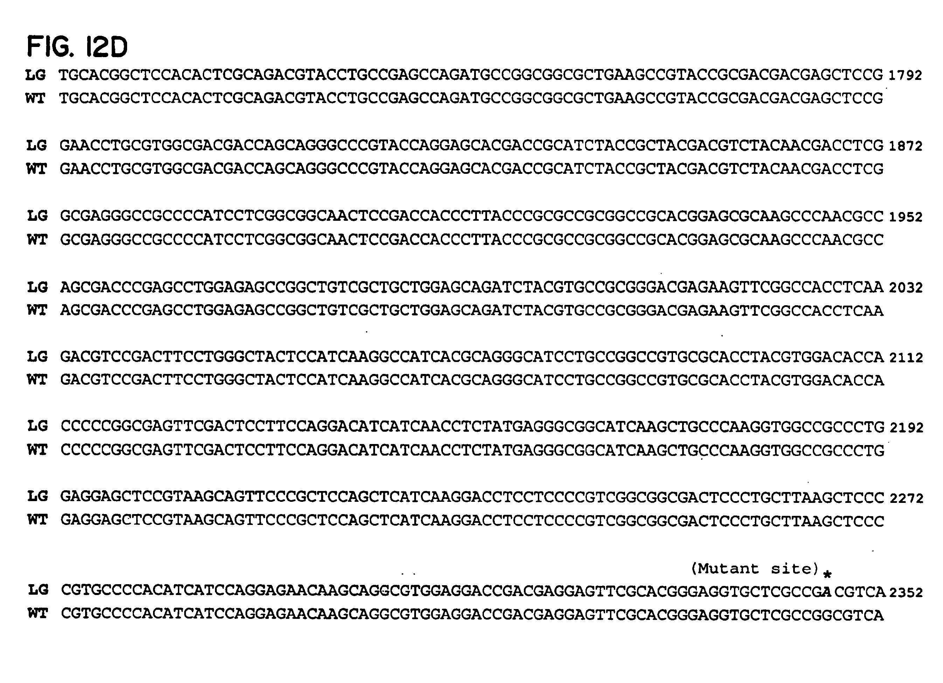

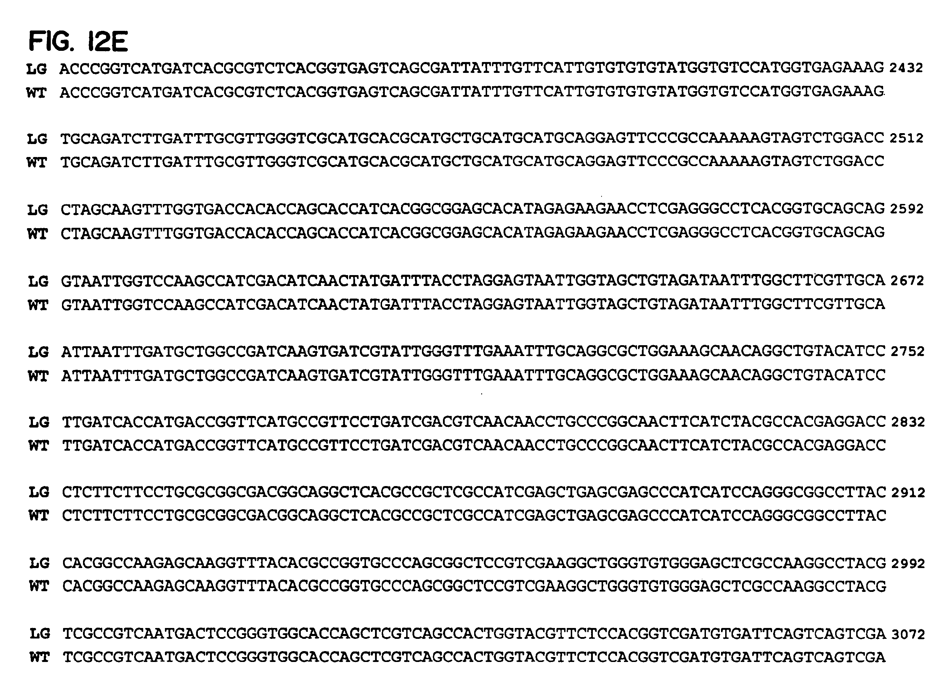

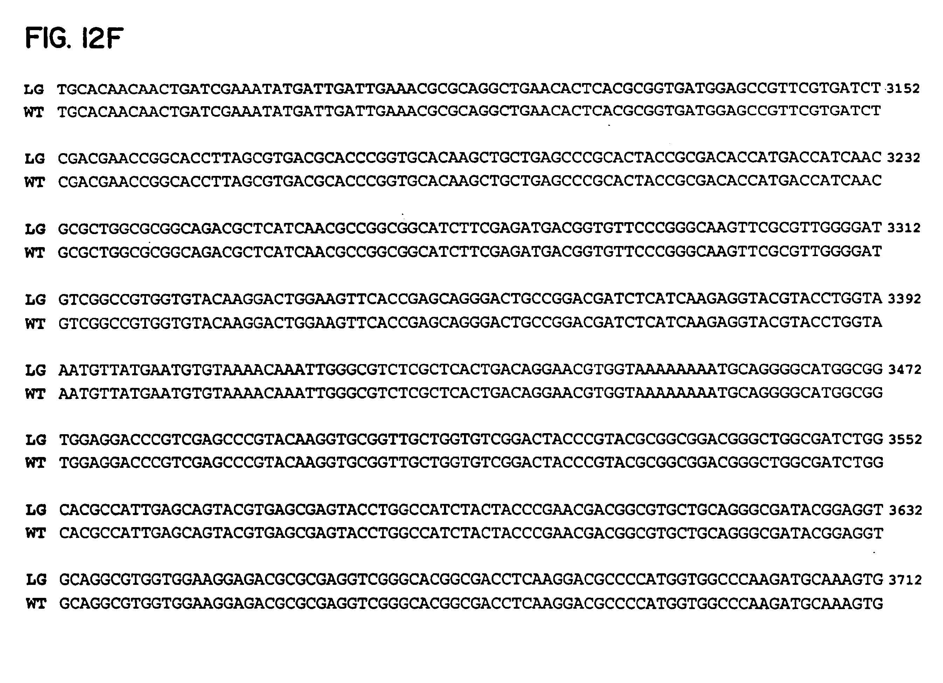

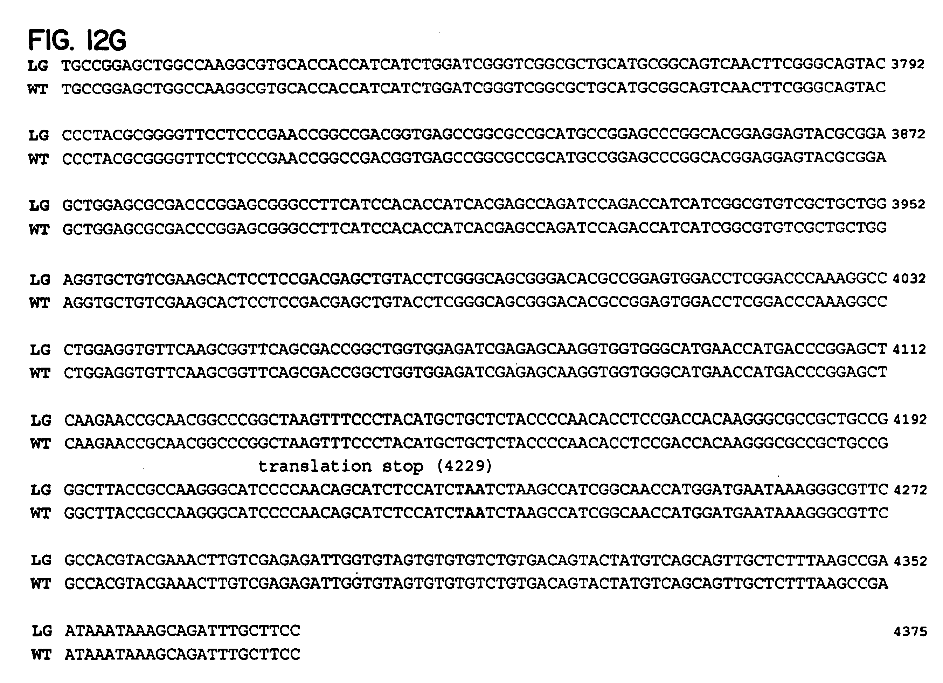

Genomic DNA from Line G and wild-type cv Vintage was isolated from seedling leaf tissue according to a method described by Pich and Schubert 1993, Nucleic Acids Res. 21: 3328. The *lox-1* gene in the genomic DNA preparations was amplified by polymerase chain reaction (PCR) using primers based on the sequence of the barley *lox-1* gene (van Mechelen et al. 1995, BBA 1254: 221-225; Rouster et al., 1997, Plant J. 11: 513-523). The position and sequence of the oligonucleotide primers used to amplify the *lox-1* promoter and coding regions, indicated in **Figure 12** were as follows:

Forward primer 5'-GAA AAG CTT GGA GGT AGA CGC TGC-3' [**SEQ ID NO:2**] and reverse primer 5'-TAT AGG ATC CTT GTT CTT GGC CTC CTC TCC TCG-3' [**SEQ ID NO:3**] were used to PCR amplify the *lox*-1 promoter domain (-361 to +68) of Line G and cv Vintage *lox*-1.

Forward primer 5'-ACT GAA AAA CAG TGT GCT GGT G-3' [**SEQ ID NO:4**] and reverse primer 5'-GGC TTA AAG AGC AAC TGC TGA-3' [**SEQ ID NO:5**] were used to PCR amplify the Line G *lox*-1 coding region.

Forward primer 5'-CAA GAT GCA TAT GCT GCT GGG AG-3' [**SEQ ID NO:6**] and reverse primer 5'-CGA TGG TTT AAA TTA GAT GGA GAT GCT GT-3' [**SEQ ID NO:7**] PCR amplified the cv Vintage *lox*-1 coding region.

The PCR reactions consisted of 250 ng genomic DNA in a 50 µl volume containing 50 pmol primer and 2 U *Pfu* DNA polymerase (Promega) according to the enzyme suppliers instructions. The PCR amplifications were carried out in a Stratagene Robocycler: 1 minute at 94°C, 1 cycle; 1 minute at 94°C, 2 minutes at 62°C, and 5 minutes at 72°C, 30 cycles; 10 minutes at 72°C, 1 cycle. The PCR products were separated on 1.2 % agarose gels. DNA fragments, corresponding in length to the amplified region, were purified using Qiax II Gel extraction kit (Qiagen) and cloned into the plasmid pcDNA2.1 (Invitrogen). The nucleotide sequence of both strands of the cloned *lox*-1 promoter and coding regions was determined using the dideoxynucleotide chain termination reaction with specific oligonucleotide primers and analysed on an ABI PRISM^{®} 310 Genetic Analyzer (PE Biosystems). Sequence comparisons were performed using the DNA STAR sequence analysis software package (DNA STAR Inc., USA).

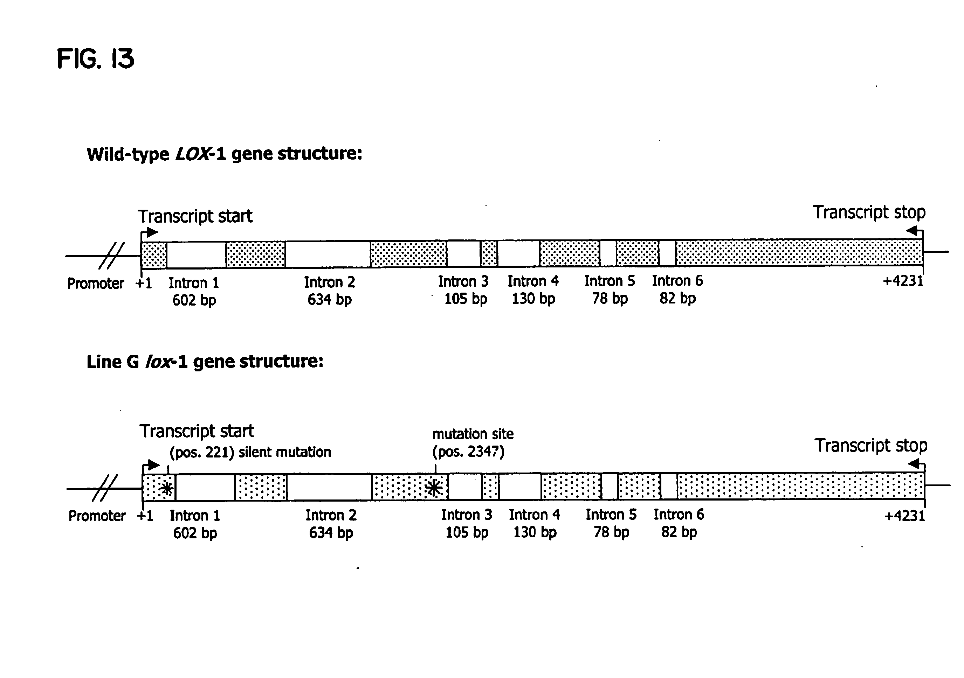

The promoter region and intron-exon structure of the barley *lox*-1 coding region are shown in **Figure 13****,** and were deduced from a comparison of the nucleotide sequence of the wild-type *lox*-1 genomic and cDNA sequences (**Figure 12**). The sequenced region of the *lox*-1 promoter region from -363 to +68, (numbered relative to the determined transcription start site; van Mechelen et al., 1995, BBA 1254: 221-225), is sufficient to direct embryo-specific and temporally-regulated gene expression characteristic of the native gene (Rouster et al, 1998, Plant J 15: 435-440). The promoter and transcribed region of the wild-type *lox*-1 gene [**SEQ ID NO:8**] **is** 4663 nt in length and contains 6 introns of between 82 nt and 634 nt in length, which are absent from the respective cDNA [**SEQ ID NO:10**] and must therefore be removed during RNA transcript splicing.

Comparison of the nucleotide sequence of *lox*-1 of Line G with that of wild-type (**Figure 12**) showed that the Line G *lox*-1 allele has two point mutations. One is a silent C→T substitution at position 221 in exon 1, and the second is a G→A substitution at position 2347 in exon 3 (**Figure 13**). The wild-type barley *lox*-1 gene encodes a protein of 862 amino acid residues [**SEQ ID NO:9**], while the mutation at position 2347 in Line G *lox*-1 allele causes an amino acid substitution of glycine to aspartic acid at residue 368 in the encoded protein.

Alignment of related plant lipoxygenases indicated that the glycine-368 in barley LOX-1, is strongly conserved. Furthermore this residue, which corresponds to glycine-353 in soybean LOX-1, is one of 51 neutral or hydrophobic residues which line the substrate cavity of the enzyme, as seen from its crystal structure (Minor et al., 1996, Biochemistry 35: 10687-10701) and is shown in (Figure 22). The insertion of a charged amino acid residue at this position is thus likely to disturb the structural and functional properties of the enzyme.

### 4. The mutated LOX-1 protein encoded by the Line G lox-1 allele has low enzymic activity and is responsible for the low lipoxygenase phenotype of Line G.

The sodium azide mutagenesis of cv Vintage grain, which induced the mutated *lox*-1 allele in Line G, may have induced additional mutations in the Line G genome. Two experimental approaches have been taken to demonstrate that the mutant *lox*-1 allele in Line G is responsible for its low lipoxygenase phenotype, rather than other mutations in the genome. The enzymic activity of the LOX-1 encoded by the mutant and wild-type *lox*-1 allele have been determined in order to prove that the glycine→aspartic acid substitution in the mutant enzyme causes reduced stability and activity. The two *lox*-1 genes were transiently expressed in aleurone protoplasts isolated from imbibed mature grain, since the level of endogenous lipoxygenase expression in these cells was expected to be below detection limits. None of the identified barley lipoxygenase genes, which are expressed in germinating barley, are detected in the aleurone tissue (van Mechelen *et al.,* 1999 *supra*). In order to direct transient expression of the *lox*-1 gene in aleurone protoplasts, their coding regions were translationally fused to a constitutive promoter known to be active in these protoplasts.

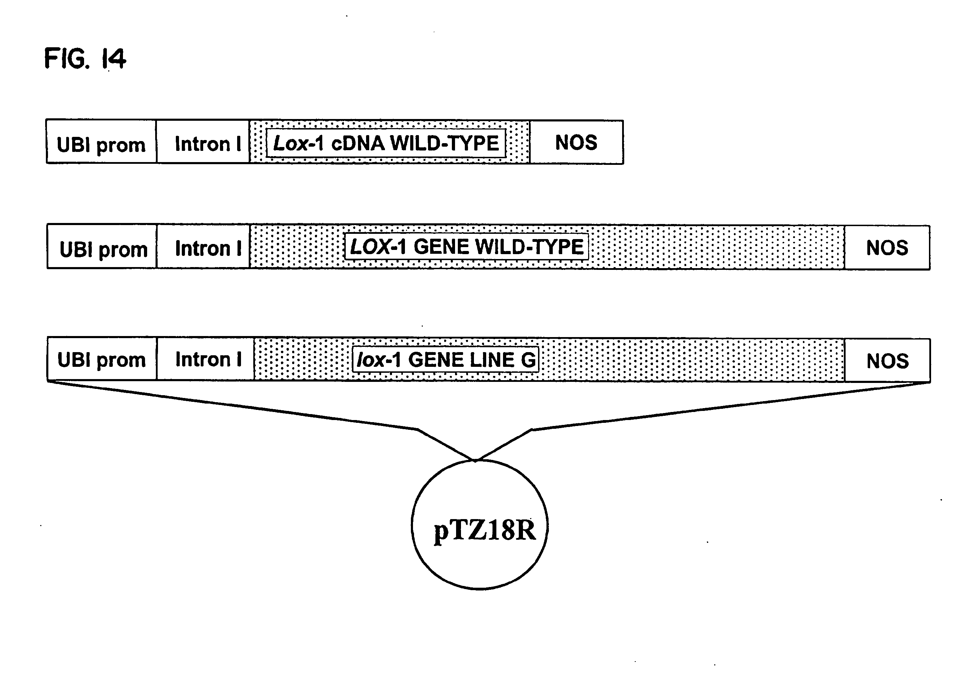

The coding regions of the mutant (sequence positions +1 to +4350) and the wild-type *lox*-1 gene (sequence positions +69 to +4230) and the wild-type *lox*-1 cDNA (sequence positions +69 to +2654) each cloned in plasmid pcDNA2.1 (see section 3), were excised by digestion of the *Kpn*I and *Eco*RV sites in vector polylinker. The coding regions were cloned in the pUBARN plasmid (Jensen et al., 1998, Hereditas 129: 215-225) between the constitutively active maize ubiquitin *Ubi* promoter (as described in U.S. patent No. 005510474A) and the *Nos* terminator, in place of the *bar* gene which encodes phosphinotricin acetyl transferase (**Figure 14**).

Protoplasts were isolated from aleurone tissue of imbibed *Hordeum vulgare* cv Himalaya according to the protocol of Skriver et al. 1991, Proc. Natl. Acad. Sci. USA 88: 7266-7270. Aliquots of 2·10⁵ protoplasts were transfected at 0°C witch ∼100 µg plasmid DNA (equimolar amounts of each plasmid) by polyethylene glycol (PEG) mediated DNA uptake (Lee et al., 1997, Plant Mol. Biol. 13: 21-29), and then incubated in aleurone protoplast culture media at 25°C as described previously (Skriver *et al.,* 1991 *supra*). After 48 hours incubation, the culture medium was carefully removed and the protoplasts were resuspended and homogenised in 300 µl lipoxygenase assay buffer (0.2 mM boric acid, 25 mM HEPES, pH 6.5). The homogenates were centrifuged at 15,000 ***g*** for 5 minutes to pellet insoluble material, and the supernatants (10 µl) were subsequently assayed for total lipoxygenase activity using the rapid screening assay described in Example 1, section 1, but with omission of the NDGA inhibitor. The protein content of the protoplast extracts was measured by a Bradford dye-binding assay (Bradford 1976, Anal. Biochem., 72: 248) supplied by Bio-Rad Laboratories, Hercules, California, USA, and lipoxygenase activity was expressed per mg protein in the extract.

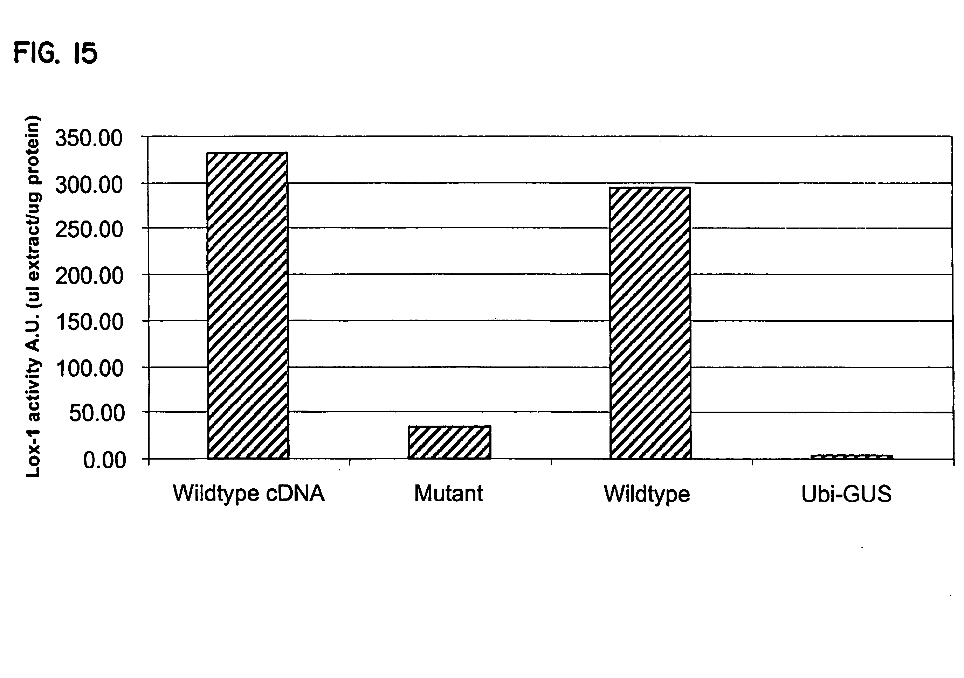

Protoplasts transfected with the control plasmid, pUBI-GUS, where the maize ubiquitin-1 promoter directs expression of the β-glucuronidase reporter gene, gave no detectable lipoxygenase activity. Transient expression of the wildtype *lox*-1 gene and cDNA in protoplasts both gave high levels of lipoxygenase activity in the protoplast extracts (**Figure 15**). The higher expression of the wild-type *lox*-1 cDNA in comparison to the genomic sequence may be due to a higher transfection frequency for the smaller *lox*-1 cDNA expression plasmid (4929 bp versus the 6505 bp *lox*-1 gene construct). Transient expression of the mutant *lox*-1 gene gave low levels of lipoxygenase activity, ∼10 % of wild-type lipoxygenase activity. These data clearly demonstrate that the mutant *lox*-1 gene in Line G encodes a lipoxygenase with greatly reduced activity, which accounts for the low lipoxygenase phenotype.

### Example 4

### PCR-Cleavage Amplified Polymorphic Site (PCR-CAPS) Assay: A Method Used for Identification of the Mutant lox-1 Gene

An analytical method allowing the identification of the Line G mutant *lox*-1 gene in any genetic background was developed based on the PCR-CAPS assay. The assay involves PCR amplification of genomic DNA fragments, followed by digestion of the amplified sequences with a specific restriction endonuclease to display a restriction fragment length polymorphism (RFLP).

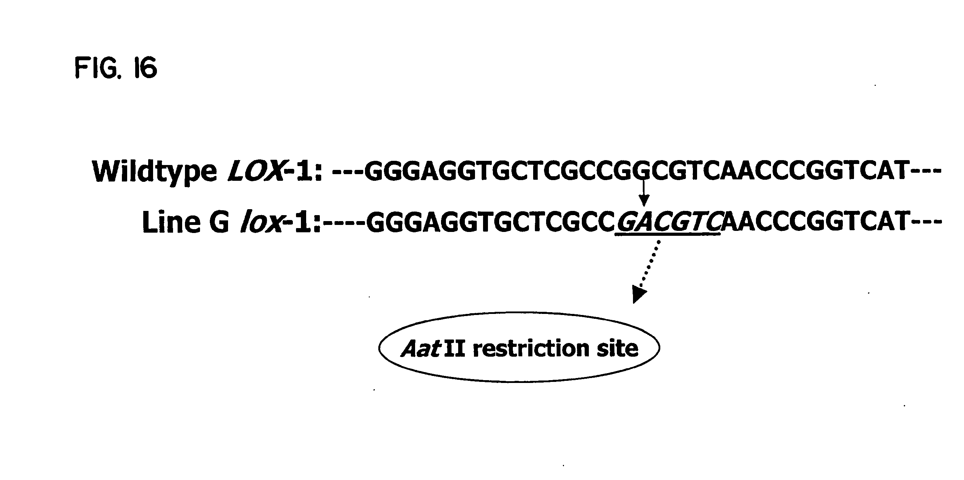

The coding sequence of the mutant *lox*-1 gene harbours two point mutations (see Example 3), where the mutation at position 2347 (**Figure 12**) introduces an additional *Aat* II restriction endonuclease cleavage site, not found in the wild-type *lox*-1 gene (**Figure 16**). The following PCR-CAPS assay, based on the polymorphism created by the presence of this restriction site in the *lox*-1 gene, is shown to descriminate between a wild-type *lox*-1 gene and a mutated *lox*-1 gene.

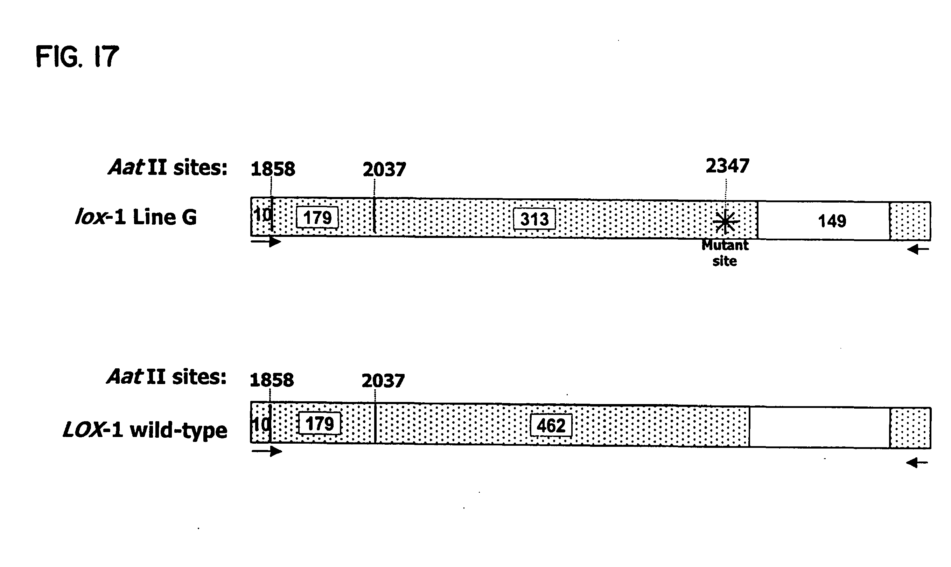

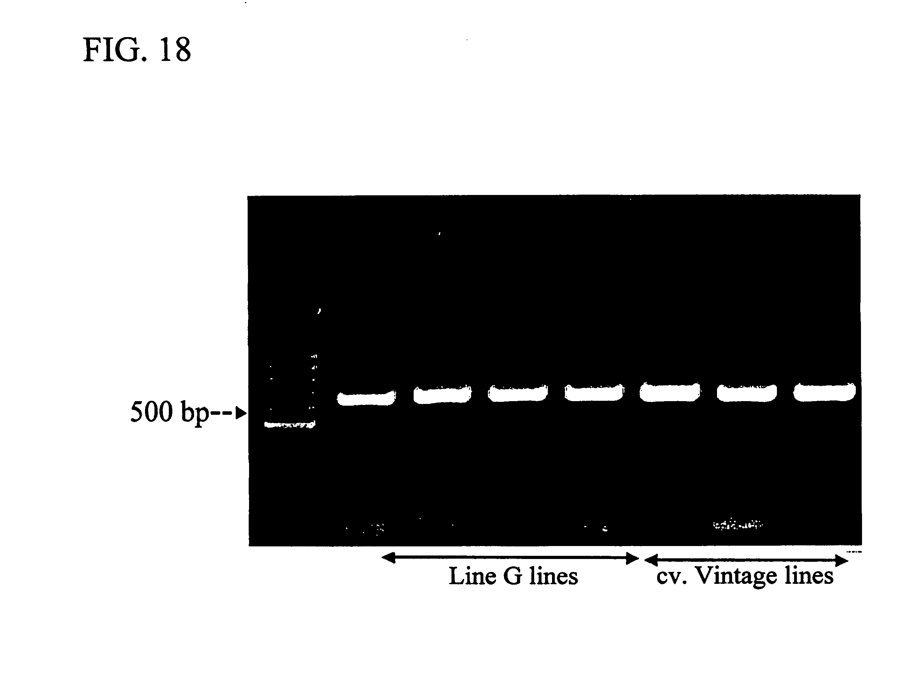

Genomic DNA was isolated from young leaves of M6 seedlings of *Hordeum vulgare,* L. cv Vintage and Line G according to the procedure of Pich and Schubert (1993, *supra*). The DNA sequence encompassing position 2347 (Line G *lox*-1 gene mutation site) was amplified by PCR, using primers specific for the *lox*-1 gene [**SEQ ID NO:8**]. The DNA fragments amplified by the selected forward primer 5'-CGCTACGACGTCTACAACGA-3' [**SEQ ID NO:13**] and reverse primer 5'-CAGACTACTTTTTGGCGGGA-3' [**SEQ ID NO:14**] are shown in **Figure 17****.** PCR reactions were carried out with 250 ng genomic DNA in a 50-µl volume containing 50 pmol of each primer and 1 unit Taq DNA polymerase (Promega) according to the suppliers instructions. PCR amplifications were carried out on a Stratagene Robocycler as follows: 1 minute at 94°C, 1 cycle; 1 minute at 94°C, 1.5 minutes at 60°C, and 2 minutes at 72°C, 30 cycles; 10 minutes at 72°C, 1 cycle. The amplified fragments of the mutant and wild-type *lox*-1 gene were ~650 bp (**Figure 18**), corresponding to the expected size (**Figure 17**). The PCR products, purified on a spin column (Qiagen), were digested with 25 unit *Aat* II restriction endonuclease for 24 hours at 37°C and analyzed on a 1.2% agarose gel.

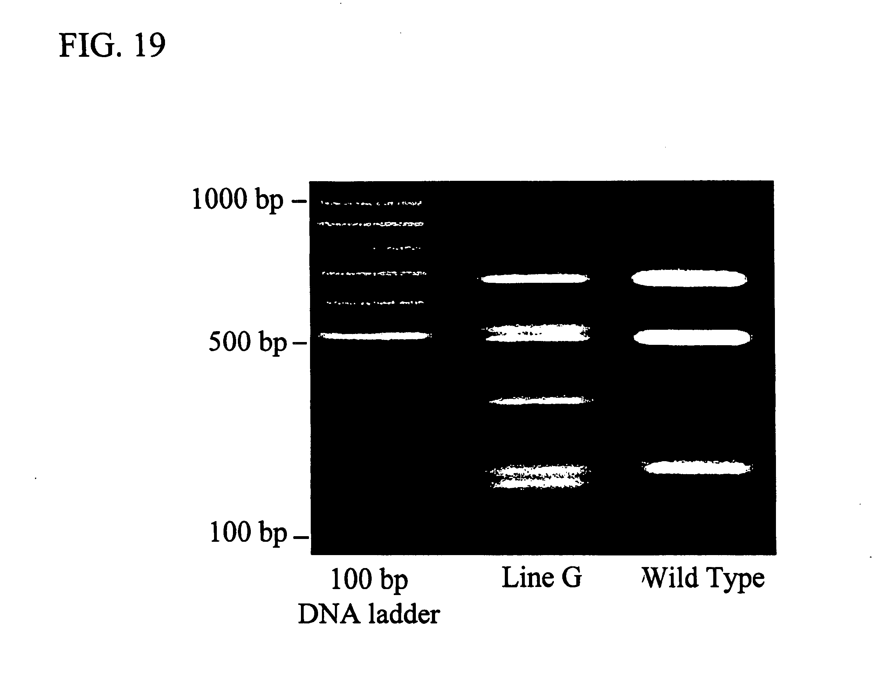

Digestion of the wild-type *lox*-1 PCR product yielded DNA fragments of 10, 179, and 462 bp, and the fragments from the mutant *lox-*1 PCR product were 10, 149, 179, and 313 bp, where additional DNA fragments were due to partial digestion of the *lox*-1 PCR product (**Figure 19**). The fragment pattern corresponds to the expected RFLP resulting from this mutation, where the 313 bp fragment is unique to the mutant *lox*-1. This PCR-CAPS assay provides a reproducible and specific tool for identification of the *lox*-1 allele in barley and can thus be exploited in barley breeding programs aimed at introducing this gene in new barley varieties.

### Example 5

### Back-Crossing the Low Lipoxygenase Phenotype of Line G to cv Alexis Demonstrates a Genetic Linkage to the Mutant lox-1 Gene

Repeated back-crossing was used to transfer the low-lipoxygenase phenotype from line G into a recurrent parent (in this case the cv. Alexis). The back-crossing program shown in **Figure 20****,** combined with selection for the low-lipoxygenase phenotype, progressively substitutes the Line G genome by the recurrent parent genome. Furthermore, other mutations introduced into the Line G genome during the sodium azide mutagenesis treatment will be eliminated. In the first back-cross of the homozygous low-lipoxygenase Line G (denoted genotype *ll*) to cv Alexis (denoted genotype *LL*) the progeny lines will be heterozygous (denoted genotype *Ll*). A low-lipoxygenase phenotype due to a recessive mutation will not be detectable in lines heterozygous for the mutation. The progeny are self-pollinated and will give a normal Mendelian segregating population, namely 1*LL*: 2 *Ll* : 1 *ll.* The low lox homozygous *ll* genotype resulting from the first back-cross will have 50 % cv Alexis genetic background. After ten rounds of back-crossing, the recurrent parent background will be approximately 99.9 %.

*Hordeum vulgare,* L. cv Alexis and Line G were propagated in a greenhouse throughout the back-crossing program. Back-crossed progeny grains were germinated in petri dishes on filterpaper, soaked with 4 ml H₂O for 3 days at 22°C in the dark. The low-lipoxygenase lines were screened by measuring total lipoxygenase activity in extracts of the coleoptile (top 7 mm) from the germinating seedlings, as described in Example 1. Progeny of the 3^{rd} and 4^{th} back-cross were also analysed for inheritance of the mutant *lox*-1 gene using the PCR-CAPS assay described in Example 4.

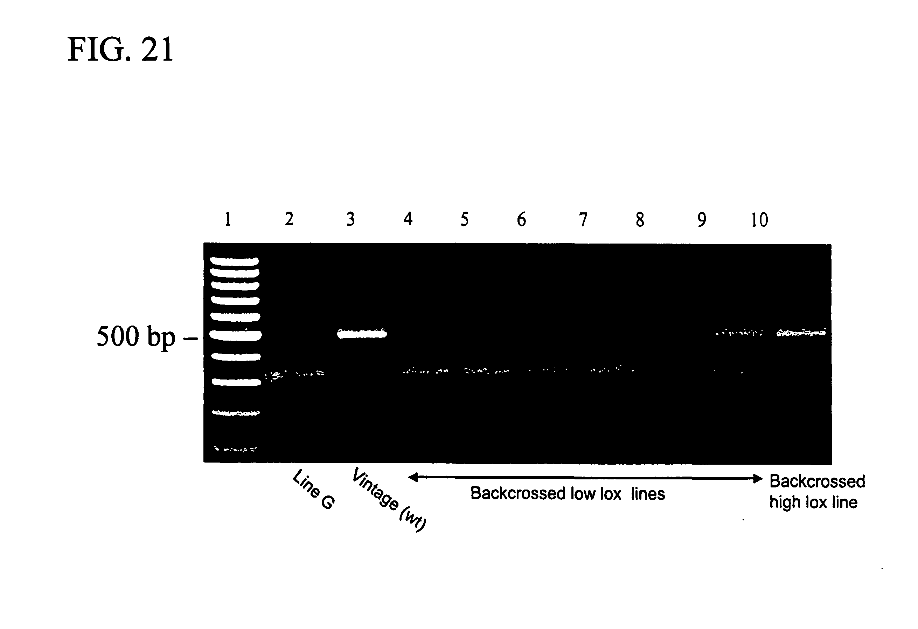

The expected frequency of the low-lipoxygenase phenotype in the segregating progeny of the four back-cross generations was 25% for a recessive mutation. The observed frequency of low-lipoxygenase activity in the progeny (24 grains) of the four back-cross generations is in agreement with the expected frequency (**Figure 20**). When the 3^{rd} and 4^{th} back-cross progeny having the low lox homozygous *ll* genotype were analysed with the PCR-CAPS assay, they were all found to have the diagnostic 313 bp fragment, while progeny having wild-type lipoxygenase activity lacked this fragment (**Figure 21**).

The back-crossing program demonstrates that the mutant *lox*-1 allele can be transferred to a new genetic background and is inherited in a recessive monofactorial manner following Mendelian segregation. Since the recurrent parent background is 93.8% in the 4^{th} back-cross progeny, the co-inheritance of the mutant *lox*-1 gene and the low-lipoxygenase phenotype provides confirmation of their genetic linkage.

### Example 6

### Beer Brewed From Line G Barley Malt Accumulates Less trans-2-nonenal During Storage, Giving an Improved Flavour Stability

*Hordeum vulgare* L cv Vintage and Line G were propagated in the field over several seasons in order to provide sufficient grain for industrial malting. The following industrial scale malting and brewing trials as well as analyses of the finished beer were performed to demonstrate the value of the Line G low-lipoxygenase barley for improved flavour stability.

### 1. Industrial malting and kilning of Line G and cv Vintage

Malting was performed on a 10-ton scale in an industrial malthouse in two trials as follows:

### Trial 1: Line G barley grain (1996 harvest)

Steeping conditions: 8 hours wet; 14 hours dry; 8 hours wet; 10 hours dry; 4 hours wet in 16°C steeping water. Malting conditions: 12 hours at 18°C; 24 hours at 16°C; 24 hours at 14°C; 60 hours at 12°C. Kilning conditions: 12 hours at 60°C; 3 hours at 68°C; 4 hours at 74°C; 3 hours at 80°C.

### Trial 2: cv Vintage and Line G (1996/1997 harvest)

Steeping conditions: 8 hours wet; 10 hours dry; 6 hours wet; 15 hours dry; 4 hours wet in 15°C steeping water. Malting conditions: 5 days with inlet air at 15°C and spraying to maintain moisture level. Kilning conditions: 10 hours at 50°C; 2 hours at 60°C; 2.5 hours at 80°C.

Malting analyses of 2 samples of the Line G malt from Trial 1 compared to the control malt, cv Nevada (Table 1) and from Trial 2 compared to cv Vintage (Table 2) confirmed that Line G malt was suitable for brewing trials.

**TABLE 1**

| **MALTING TRIAL 1** | | | | |
|---|---|---|---|---|
| Barley variety | | cv Nevada | LineG | LineG |
| Crop year | | 1996 | 1996 | 1996 |
| | | | | |
| Malt analyses | | | | |
| Moisture content | % | 4.7 | 4.3 | 4.4 |
| Extract fine as is. | %ai. | 76.9 | 76.1 | 75.3 |
| Extract fine d.m. | %dm. | 81.4 | 79.5 | 78.7 |
| Saccharification time | Min | <10 | <10 | <10 |
| Diastatic power | %WK | 252 | 373 | 365 |
| Color | EBC | 2.8 | 4.4 | 3.8 |
| pH | | 6.16 | 5.97 | 5.99 |
| Turbidity | EBC | 9.0 | 2.5 | 2.4 |
| Total protein d.m. | % | 10.35 | 10.74 | 12.03 |
| Soluble nitrogen | mg/l | 647 | 787 | 765 |
| Sol. Protein % malt d.m. | %dm. | 3.7 | 4.4 | 4.3 |
| Kolbach | | 35.3 | 40.8 | 35.4 |
| Free Amino Nitrogen | mg/l | 97 | 125 | 118 |
| Friability | % | 89.5 | 85.6 | 89.5 |
| Whole unmodified grains | % | 1.1 | 0.6 | 0.5 |
| Partly unmodified grains | % | 2.3 | 1.0 | 0.6 |
| β-glucan in wort | mg/l | 114 | 66 | 36 |
| β-glucan in malt | % w/w | 0.24 | 0.11 | 0.05 |
| S-methylmethionine/DMS eq. | µg/g | 2.4 | 6.4 | 8.4 |
| Free DMS | µg/g | 1.0 | 6.6 | 4.7 |
| NDMA | µg/kg | n.d. | 0.3/0.6 | 0.3/0.3 |

**TABLE 2**

| **MALTING TRIAL 2** | | | | |
|---|---|---|---|---|
| Barley variety | | Vintage | Line G | Line G |
| Crop year | | 1996 | 1996 | 1997 |
| | | | | |
| Moisture content | % | 4.1 | 4.1 | 4.3 |
| Extract fine as is. | %ai. | 77.0 | 75.6 | 77.5 |
| Extract fine d.m. | %dm. | 80.3 | 78.8 | 80.9 |
| Fine/coarse difference | %dm. | 0.7 | 1.6 | 1.7 |
| Saccharification time | min | - | - | < 10 |
| Diastatic power | %WK | 343 | 342 | 268 |
| Color | EBC | 2.5 | 2.8 | 3.4 |
| PH | | 6.05 | 6.01 | 6.12 |
| Turbidity | EBC | 1.5 | 1.3 | 2.5 |
| Total protein d.m. | % | 10.98 | 12.22 | 9.82 |
| Soluble nitrogen | mg/l | 696 | 741 | 610 |
| Sol. Protein % malt d.m. | %dm. | 3.9 | 4.1 | 3.4 |
| Kolbach | | 35.2 | 33.7 | 34.6 |
| Free Amino Nitrogen | mg/l | 110 | 117 | 100 |
| Friability | % | 91.3 | 81.8 | 89.5 |
| Whole unmodified grains | % | 0.7 | 1.1 | 1.3 |
| Partly unmodified grains | % | 1.0 | 2.7 | 2.7 |
| β-glucan in wort | mg/l | 97 | 172 | 117 |
| Beta-glucan in malt | % w/w | - | - | 0.3 |
| S-methylmethionine/DMS eq. | µg/g | - | - | - |
| Free DMS | µg/g | - | | - |

### 2. Industrial brewing with Line G, cv Vintage malt, and control malt cv Nevada

Two brewing trials were performed, using wort prepared from Line G and control malt cv Nevada malt in Trial 1 and from Line G and control malt cv Vintage in Trial 2.

Beer was brewed on a 30-hl industrial scale with 475 kg malt according to the following scheme: Mashing in at 50°C; 30 minutes at 50°C; 30 minutes heating from 50 - 70°C; 15 minutes at 70°C. A portion of the wort was heated for 20 minutes from 70 - 100°C and 5 minutes at 100°C, while the main mash was kept at 70°C for another 25 minutes and then the two mashes was combined and kept for 10 minutes at 76°C. The brewing steps of wort boiling, whirlpool separation of spent grain, cooling, fermentation, lagering and packaging in brown glass bottles were according to standard brewing practise.

### 3. Flavor stability and T2N content of beer brewed from Line G, cv Vintage malt and control malt cv Nevada

The freshly bottled beer was stored at 5°C and analysed within 2 months of production. The flavor-stability of the fresh and stored beer was evaluated in two independent laboratories following two different types of beer storage conditions. In laboratory A the beer was subjected to a forced aging process, where the beer was stored at 37°C for a period of 7 days, while in laboratory B the beer was stored at 30°C for 6 and 12 weeks. *Trans*-2-nonenal levels in beer were determined by gas chromatography and mass spectrometric detection following derivatisation of carbonyls with O-(2,3,4,5,6-pentafluorobenzyl)-hydroxylamine, essentially as described by Grönqvist et al. 1993 Proceedings of the 24th EBC Congress, Oslo, 421-428. A trained beer taste panel evaluated the overall flavor score of the beer, which includes detection of a cardboard flavor, indicative of free *trans*-2-nonenal in the beer.

### Laboratory A: Forced-Aging Tolerance

Comparison of beer, brewed from Line G and the control malt, cv Nevada, in the first brewing trial (**Table 3**) demonstrated that beer from Line G had a greater flavor stability and a lower trans-2-nonenal content following forced-aging as compared to the controls. The second trial, comparing beer brewed from Line G malt with beer brewed from cv Vintage malt, the parental cultivar, confirmed the initial data (**Table 4**).

**TABLE 3**

| **BREWING TRIAL 1** | | |
|---|---|---|
| Barley Malt | cv Nevada | Line G |
| SO₂ Content (mg/ml) | 1 | 1 |
| T2N** (ppb) - Fresh Beer | 0.009 | 0.005 |
| T2N**(ppb) - Aged Beer (37°C / 7 DAY) | 0.117 | 0.025 |
| Flavor* - Fresh Beer | 5.9 | 5.3 |
| Flavor* - Aged Beer (37°C / 7 DAY) | 1.3 | 5.1 |

| | | |
|---|---|---|
| * Flavor evaluation scale 1-10 of increasing quality; ***trans*-2-nonenal. | | |

**TABLE 4**

| **BREWING TRIAL 2** | | |
|---|---|---|
| Barley Malt | cv Vintage | Line G |
| SO₂ Content (mg/ml) | 2 | 2.5 |
| T2N** (ppb) - Fresh Beer | 0.023 | 0.019 |
| T2N** (ppb) - Aged Beer (37°C / 7 Day) | 0.078 | 0.035 |
| FLAVOR* - Fresh Beer | 5.5 | 6.1 |
| FLAVOR* - Aged Beer (37°C / 7 Day) | 2.9 | 5.9 |

| | | |
|---|---|---|
| * Flavor evaluation scale 1-10 of increasing quality; ***trans*-2-nonenal. | | |

### Laboratory B: 30°C Storage Tolerance

Beer brewed from Line G malt had lower *trans*-2-nonenal levels following 6 and 12 weeks at the elevated storage temperature of 30°C, when compared to beer brewed from either of the reference malts (Table 5 and 6) and had a better flavor-stability as judged by a taste panel. The taste-threshold for *trans*-2-nonenal in these analysed beers lies close to 0.08 ppb.

**TABLE 5**

| **BREWING TRIAL 1** | | |
|---|---|---|
| Barley Malt | cv Nevada | Line G |
| *trans*-2-nonenal (ppb) - Fresh Beer | 0.050 | 0.044 |
| *trans*-2-nonenal (ppb) - 30°C / 6 weeks | 0.072 | 0.037 |
| *trans-2-nonenal* (ppb) - 30°C /12 weeks | 0.095 | 0.046 |
| *trans*-2-nonenal flavor *- Fresh Beer | 0.6 | 0.3 |
| *trans*-2-nonenal flavor *- 30°C / 6 weeks | 3.7 | 1.4 |
| *trans*-2-nonenal flavor* - 30°C /12 weeks | 2.5 | 0.6 |

| | | |
|---|---|---|
| * *trans*-2-nonenal flavor detection score on a scale of 1 -10 | | |

**TABLE 6**

| **BREWING TRIAL 2** | | |
|---|---|---|
| Barley Malt | cv Vintage | Line G |
| *trans*-2-nonenal (ppb) - Fresh Beer | 0.070 | 0.062 |
| *trans*-2-nonenal (ppb) - 30°C / 6 weeks | 0.093 | 0.070 |
| *trans*-2-nonenal (ppb) - 30°C / 12 weeks | 0.133 | 0.080 |
| *trans*-2-nonenal flavor* - Fresh Beer | 0.3 | 0.9 |
| *trans*-2-nonenal flavor* - 30°C / 6 weeks | 2.5 | 1.7 |
| *trans*-2-nonenal flavor* - 30°C / 12 weeks | 2.2 | 1.3 |

| | | |
|---|---|---|
| * *trans*-2-nonenal flavor detection score on a scale of 1 -10 | | |

The improved flavor-stability of beer brewed from Line G malt, as measured by the levels of *trans*-2-nonenal detected in the beer following storage at 30°C from the combined brewing trial data, is shown to be statistically significant (Table 7).

**TABLE 7**

| ***TRANS*-2-NONENAL IN STORED BEER** | | | | | |
|---|---|---|---|---|---|
| **fresh** | **Mean** | **Stdev** | **Difference** | **2-tailed p** | **Significant (p** < **0.05)** |
| reference | 0.060 | 0.012 | 0.007 | 0.34 | no |
| line-G | 0.053 | 0.011 | | | |
| | | | | | |

| **6 weeks, 30 °C** | **Mean** | **Stdev** | **Difference** | **2-tailed p** | **Significant (p** < **0.05)** |
|---|---|---|---|---|---|
| reference | 0.083 | 0.013 | 0.029 | 0.031 | yes |
| line-G | 0.054 | 0.021 | | | |
| | | | | | |

| **12 weeks, 30 °C** | **Mean** | **Stdev** | **Difference** | **2-tailed p** | **Significant (p** < **0.05)** |
|---|---|---|---|---|---|
| reference | 0.114 | 0.023 | 0.051 | 0.003 | yes |
| line-G | 0.063 | 0.020 | | | |

Since the natural sulfite levels were low in both brewing trials, the free *trans*-2-nonenal levels in the aged beer would closely reflect the *trans*-2-nonenal potential of the different beers, namely the level of *trans*-2-nonenal adducts present in the fresh beer. Addition of sulfite can temporarily delay the staling process, by complexing free-*trans*-2-nonenal, until sulfite levels are reduced by oxidation due to gaseous exchange through the packaging.

The described brewing trials with low-LOX-1 barley malt provide the first unequivocal evidence that LOX-1 activity in barley during the malting and brewing process is a key determinant of the appearance of the off-flavor compound *trans*-2-nonenal in aged beer.

## Claims

1. A barley plant or portion thereof comprising a mutated LOX-1 protein, wherein LOX-1 activity of said plant or plant portion is reduced or absent as compared to a non-mutated control, wherein said mutated LOX-1 protein comprises the amino acid sequence shown as SEQ ID NO: 12, wherein Xaa is aspartic acid, glutamic acid, histidine, lysine, arginine, threonine, serine, tyrosine, tryptophan, asparagine or glutamine.

2. The barley plant or portion thereof according to claim 1, wherein the LOX-1 activity comprises catalysis of oxidation of free and esterified polyunsaturated fatty acids and polyunsaturated octadecanoic fatty acids to form 9-hydroperoxy fatty acid derivatives.

3. The barley plant or portion thereof according to claim 1, wherein Xaa is glutamic acid or aspartic acid.

4. The barley plant or portion of claim 3, wherein Xaa is aspartic acid.

5. Grain or plant progeny comprising a mutated LOX-1 protein, wherein the mutated LOX-1 protein comprises the amino acid sequence shown as SEQ ID No. 12, wherein Xaa is D, E, H, K, R, T, S, Y, W, N or Q produced from the barley plant or plant portion thereof according to any one of claims 1-4.

6. A malt or wort comprising a mutated LOX-1 protein, wherein the mutated LOX-1 protein comprises the amino acid sequence shown as SEQ ID No. 12, wherein Xaa is D, E, H, K R, T, S, Y W, N or Q produced from the barley plant or plant portion thereof according to any one of claims 1-4 or from the grain or plant progeny of claim 5.

7. Use of a malt or wort according to claim 6 for the preparation of a beverage presenting organoleptic qualities that remain stable over a measured period of time or at elevated storage temperatures as compared to a beverage prepared from a plant product of a non-mutated control.

8. Use of a barley plant or portion thereof, grain or plant progeny, or malt or wort according to any one of claims 1-6 for the manufacture of a beverage.

9. Use of a barley plant or portion thereof, grain or plant progeny, or malt or wort according to any one of claims 1-6 for the manufacture of a malt or beer.

10. Use of a barley plant or portion thereof, grain or plant progeny, or malt or wort according to any one of claims 1-6 for the stabilisation of organoleptic properties of a brewed product over a measured period of time as compared to a control brewed product produced using a non-mutated barley plant or portion thereof, grain or plant progeny, or plant product.

11. Use of a barley plant or portion thereof, grain or plant progeny, or malt or wort according to any one of claims 1-6 for the manufacture of a brewed product having reduced levels of free trans-2-nonenal over a measured period of time or under conditions of elevated storage temperature, as compared to a control brewed product produced using a non-mutated barley plant or portion thereof, grain or plant progeny, or plant product.

12. A process for producing beer from barley wherein the barley or a portion thereof comprises a mutated LOX-1 protein, wherein the LOX-1 activity of said plant or plant portion is reduced or absent as compared to a non-mutated control, and wherein said mutated LOX-1 protein comprises the amino acid sequence shown as SEQ ID NO: 12, wherein Xaa is aspartic acid, glutamic acid, histidine, lysine, arginine, threonine, serine, tyrosine, tryptophan, asparagine or glutamine.

13. A process according to claim 12, wherein Xaa is glutamic acid or aspartic acid.

14. A process according to claim 13, wherein Xaa is aspartic acid.

## Patentansprüche

1. Gerstenpflanze oder Teil davon, umfassend ein mutiertes LOX-1 Protein, wobei die LOX-1 Aktivität der Pflanze oder des Pflanzenteils im Vergleich zu einer nicht mutierten Kontrolle verringert oder abwesend ist, wobei das mutierte LOX-1 Protein die als SEQ ID NO: 12 gezeigte Aminosäuresequenz umfasst, wobei Xaa Asparaginsäure, Glutaminsäure, Histidin, Lysin, Arginin, Threonin, Serin, Tyrosin, Tryptophan, Asparagin oder Glutamin ist.

2. Gerstenpflanze oder Teil davon nach Anspruch 1, wobei die LOX-1 Aktivität die Katalyse der Oxidation von freien und veresterten mehrfach ungesättigten Fettsäuren und mehrfach ungesättigten Octadecan-Fettsäuren zum Bilden von 9-Hydroperoxy-Fettsäurederivaten umfasst.

3. Gerstenpflanze oder Teil davon nach Anspruch 1, wobei Xaa Glutaminsäure oder Asparaginsäure ist.

4. Gerstenpflanze oder Teil nach Anspruch 3, wobei Xaa Asparaginsäure ist.

5. Korn oder Pflanzennachkommenschaft umfassend ein mutiertes LOX-1 Protein, wobei das mutierte LOX-1 Protein die als SEQ ID No. 12 gezeigte Aminosäuresequenz umfasst, wobei Xaa D, E, H, K, R, T, S, Y, W, N oder Q ist, hergestellt aus der Gerstenpflanze oder einem Pflanzenteil davon nach einem der Ansprüche 1-4.

6. Malz oder Würze umfassend ein mutiertes LOX-1 Protein, wobei das mutierte LOX-1 Protein die als SEQ ID No. 12 gezeigte Aminosäuresequenz umfasst, wobei Xaa D, E, H, K, R, T, S, Y, W, N oder Q ist, hergestellt aus der Gerstenpflanze oder einem Pflanzenteil davon nach einem der Ansprüche 1-4 oder aus dem Korn oder der Pflanzennachkommenschaft nach Anspruch 5.

7. Verwendung eines Malzes oder einer Würze nach Anspruch 6 für die Herstellung eines Getränks, das organoleptische Eigenschaften aufweist, welche über einen gemessenen Zeitraum oder bei erhöhten Lagertemperaturen stabil bleiben, im Vergleich zu einem Getränk, das aus einem Pflanzenprodukt von einer nicht mutierten Kontrolle hergestellt ist.

8. Verwendung einer Gerstenpflanze oder eines Teils davon, eines Korns oder einer Pflanzennachkommenschaft, oder von Malz oder Würze nach einem der Ansprüche 1-6 für die Herstellung eines Getränks.

9. Verwendung einer Gerstenpflanze oder eines Teils davon, eines Korns oder einer Pflanzennachkommenschaft, oder von Malz oder Würze nach einem der Ansprüche 1-6 für die Herstellung eines Malzes oder Bieres.

10. Verwendung einer Gerstenpflanze oder eines Teils davon, eines Korns oder einer Pflanzennachkommenschaft, oder von Malz oder Würze nach einem der Ansprüche 1-6 für die Stabilisierung der organoleptischen Eigenschaften eines gebrauten Produktes über einen gemessenen Zeitraum im Vergleich zu einem gebrauten Kontrollprodukt, das unter Verwendung einer nicht mutierten Gerstenpflanze oder eines Teils davon, eines nicht mutierten Korns oder einer nicht mutierten Pflanzennachkommenschaft oder eines nicht mutierten Pflanzenprodukts hergestellt ist.

11. Verwendung einer Gerstenpflanze oder eines Teils davon, eines Korns oder einer Pflanzennachkommenschaft, oder von Malz oder Würze nach einem der Ansprüche 1-6 für die Herstellung eines gebrauten Produkts mit verringerten Gehalten an freiem *trans-*2-Nonenal über einen gemessenen Zeitraum oder unter Bedingungen einer erhöhten Lagertemperatur, im Vergleich zu einem gebrauten Kontrollprodukt, das unter Verwendung einer nicht mutierten Gerstenpflanze oder eines Teils davon, eines nicht mutierten Korns oder einer nicht mutierten Pflanzennachkommenschaft oder eines nicht mutierten Pflanzenprodukts hergestellt ist.

12. Verfahren zum Herstellen von Bier aus Gerste, wobei die Gerste oder ein Teil davon ein mutiertes LOX-1 Protein umfasst, wobei die LOX-1 Aktivität der Pflanze oder des Pflanzenteils im Vergleich zu einer nicht mutierten Kontrolle verringert oder abwesend ist, und wobei das mutierte LOX-1 Protein die als SEQ ID NO: 12 gezeigte Aminosäuresequenz umfasst, wobei Xaa Asparaginsäure, Glutaminsäure, Histidin, Lysin, Arginin, Threonin, Serin, Tyrosin, Tryptophan, Asparagin oder Glutamin ist.

13. Verfahren nach Anspruch 12, wobei Xaa Glutaminsäure oder Asparaginsäure ist.

14. Verfahren nach Anspruch 13, wobei Xaa Asparaginsäure ist.

## Revendications

1. Plant d'orge ou une partie de celui-ci comprenant une protéine LOX-1 mutante, dans lequel l'activité de LOX-1 dudit plant ou de ladite partie de plant est réduite ou absente comparativement à un témoin non mutant, ladite protéine LOX-1 mutante comprenant la séquence d'acides aminés illustrée comme étant SEQ ID NO. 12, dans laquelle Xaa est l'acide aspartique, l'acide glutamique, l'histidine, la lysine, l'arginine, la thréonine, la sérine, la tyrosine, le tryptophane, l'asparagine ou la glutamine.

2. Plant d'orge ou une partie de celui-ci selon la revendication 1, dans lequel l'activité de LOX-1 comprend la catalyse de l'oxydation des acides gras polyinsaturés libres et estérifiés et des acides gras octadécanoïques polyinsaturés pour former des dérivés d'acides gras 9-hydroxyperoxy.

3. Plant d'orge ou une partie de celui-ci selon la revendication 1, dans lequel Xaa est l'acide glutamique ou l'acide aspartique.

4. Plant d'orge ou une partie de celui-ci selon la revendication 3, dans lequel Xaa est l'acide aspartique.

5. Graine ou descendance de plante comprenant une protéine LOX-1 mutante, dans laquelle la protéine LOX-1 mutante comprend la séquence d'acides aminés illustrée comme étant SEQ ID NO. 12, dans laquelle Xaa est D, E, H, K, R, T, S, Y, W, N ou Q, produite à partir du plant d'orge ou d'une partie de celui-ci selon l'une quelconque des revendications 1 à 4.

6. Malt ou moût comprenant une protéine LOX-1 mutante, dans lequel la protéine LOX-1 mutante comprend la séquence d'acides aminés illustrée comme étant SEQ ID NO. 12, dans laquelle Xaa est D, E, H, K, R, T, S, Y, W, N ou Q, produit à partir du plant d'orge ou d'une partie de celui-ci selon l'une quelconque des revendications 1 à 4 ou à partir d'une graine ou d'une descendance de plante selon la revendication 5.

7. Utilisation de malt ou moût selon la revendication 6 pour la préparation d'une boisson présentant des qualités organoleptiques qui restent stables sur une certaine période de temps mesurée ou à des températures de stockage élevées comparativement à une boisson préparée à partir d'une plante d'un témoin non mutant.

8. Utilisation d'un plant d'orge ou d'une partie de celui-ci, d'une graine ou d'une descendance de plante, ou de malt ou de moût selon l'une quelconque des revendications 1 à 6 pour la préparation d'une boisson.

9. Utilisation d'un plant d'orge ou d'une partie de celui-ci, d'une graine ou d'une descendance de plante, ou de malt ou de moût selon l'une quelconque des revendications 1 à 6 pour la préparation d'un whisky de malt ou d'une bière.

10. Utilisation d'un plant d'orge ou d'une partie de celui-ci, d'une graine ou d'une descendance de plante, ou de malt ou de moût selon l'une quelconque des revendications 1 à 6 pour la stabilisation des propriétés organoleptiques d'un produit brassé sur une période de temps mesurée comparativement à un produit brassé témoin produit en utilisant un plant d'orge ou une partie de celui-ci, une graine ou une descendance de plante, ou un produit végétal non mutant.

11. Utilisation d'un plant d'orge ou d'une partie de celui-ci, d'une graine ou d'une descendance de plante, ou de malt ou de moût selon l'une quelconque des revendications 1 à 6 pour la préparation d'un produit brassé ayant des taux réduits en trans-2-nonénal libre sur une période de temps mesurée ou dans des conditions de température de stockage élevée, comparativement à un produit brassé témoin produit en utilisant un plant d'orge ou une partie de celui-ci, une graine ou une descendance de plante, ou un produit végétal non mutant.

12. Procédé de préparation d'une bière à partir d'orge, dans lequel l'orge ou une partie de celui-ci comprend une protéine LOX-1 mutante, l'activité de LOX-1 dudit plant ou de ladite partie de plant étant réduite ou absente comparativement à un témoin non mutant, et ladite protéine LOX-1 mutante comprenant la séquence d'acides aminés illustrée comme étant SEQ ID NO: 12, dans laquelle Xaa est l'acide aspartique, l'acide glutamique, l'histidine, la lysine, l'arginine, la thréonine, la sérine, la tyrosine, le tryptophane, l'asparagine ou la glutamine.

13. Procédé selon la revendication 12, dans lequel Xaa est l'acide glutamique ou l'acide aspartique.

14. Procédé selon la revendication 13, dans lequel Xaa est l'acide aspartique.


### SEQUENCE LISTING'

<110> Douma, Anneke
   Doderer, Albert
   Cameron-Mills, Verena
   Skadhauge, Birgitte
   Bech, Lene
<120> LOW LIPOXYGENASE 1 BARLEY
<130> 11225.12wo01
<160> 18
<170> PatentIn version 3.0
<210> 1
   <211> 143
   <212> DNA
   <213> Hordeum vulgare
<400> 1
   ctaagccatc ggcaaccatg gatgaataaa gggcgttcgc cacgtacgaa acttgtcgag 60
   agattggtgt agtgtgtgtc tgtgacagta ctatgtcagc agttgctctt taagccgaat 120
   aaataaagca gatttgcttc caa 143
<210> 2
   <211> 24
   <212> DNA
   <213> Hordeum vulgare
<400> 2
   gaaaagcttg gaggtagacg ctgc 24
<210> 3
   <211> 33
   <212> DNA
   <213> Hordeum vulgare
<400> 3
   tataggatcc ttgttcttgg cctcctctcc tcg 33
<210> 4
   <211> 22
   <212> DNA
   <213> Hordeum vulgare
<400> 4
   agtgaaaaac agtgtgctgg tg 22
<210> 5
   <211> 21
   <212> DNA
   <213> Hordeum vulgare
<400> 5
   ggcttaaaga gcaactgctg a 21
<210> 6
   <211> 23
   <212> DNA
   <213> Hordeum vulgare
<400> 6
   caagatgcat atgctgctgg gag 23
<210> 7
   <211> 29
   <212> DNA
   <213> Hordeum vulgare
<400> 7
   cgatggttta aattagatgg agatgctgt 29
<210> 8
   <211> 4663
   <212> DNA
   <213> Hordeum vulgare
<400> 8
   cagccccatg catgcacatg cacatgcaca tgcacatgca gtgcagccaa gcaccgctcg 60
   atgggcgatc acccgtcacg ggaccggagc gcgccatgcg aagcacgagg agggcacgtc 120
   accgtccgcg cgcagcacgt ggagagcacg tcgccgtccg atccatctct ccaaagccga 180
   gcgccacacc accgggaccg gacccggacc ggcctataaa ttgcccggac cgagctgcaa 240
   gcagctcctc acacacactc acgcaacaca catccatctt cactgaaaag tgaaaaacag 300
   tgtgctggtg ccattggttg gagcagtgaa agcgaggaga ggaggccaag aacaagatgc 360
   tgctgggagg gctgatcgac accctcacgg gggcgaacaa gagcgcccgg ctcaagggca 420
   cggtggtgct catgcgcaag aacgtgctgg acctcaacga cttcggcgcc accatcatcg 480
   acggcatcgg cgagttcctc ggcaagggcg tcacctgcca gcttatcagc tccaccgccg 540
   tcgaccaagg taatcactac cctcctccgg ccttctcctc tgtttacaag atatagtatt 600
   tctttcgtgt gggccggcgg ccatggatgg atggatgtgt ctggatcggc taaagaagat 660
   aggatagcta gccctggccg gtcgtcttta cctgagcatg ggcatatgcc atcgaaaaaa 720
   gagacaacag catgcatgca tggtgcgcgc accagaccac gcagagcacc ggatgctcga 780
   gacaaagcaa cacaacaagc aaggacgaca cgtcaaaagc aacacaacaa gcaaggacgg 840
   cacgtcaaaa gcaacacaaa cctaaactaa agcacaaaga cgtaagagca agcacacaat 900
   cagcaggcta taaacagttg tcatcaaaaa caacgctgga agagagagag aaggaaggaa 960
   gtagtagcca tgaaaaatta aatcaccggg cgttgctctt tgcccaacaa ttaatcaagc 1020
   agggtacgtg gcatgtatag ttcttgtaag taaactaagc atgtgatatg agaaggtacg 1080
   tggtggtgca gacaacggcg gtcgcgggaa ggtgggcgcg gaggcggagc tggagcagtg 1140
   ggtgacgagc ctgccgtcgc tgacgacggg ggagtccaag ttcggcctca ccttcgactg 1200
   ggaggtggag aagctcgggg tgccgggcgc catcgtcgtc aacaactacc acagctccga 1260
   gttcctgctt aaaaccatca ccctccacga cgtccccggc cgcagcggca acctcacctt 1320
   cgtcgccaac tcatggatct accccgccgc caactaccga tacagccgcg tcttcttcgc 1380
   caacgacgtg cgtggatttt cctctacttt cctctccttt cattttcacc gccttcgtca 1440
   ttcatggtcg atcattaagt cttgccagga caatagatga tgagctagga gtggttacca 1500
   cttagcagta cgtacattat ttattccgtg ttggtagaaa aggatatggt ttggtgcaga 1560
   tcgacacaag attgaatgaa agttgcaccg tggcaccgtg gcagcgtggt aggtgaaaat 1620
   aactgttgca cggatccacc cacatgattg ttttcatgaa taaacttttt aaggatgtgt 1680
   ctagccacat ctagatgcat gtcacataat tattgcatac caaaacgatt aaattaagca 1740
   taaaaagaaa aggaaaaaaa tactcacata tctcgacgta agatcaatga tatagtattt 1800
   agatatgcaa tatttatctt acatctaaac ctttcttcat tcttaaatat aagacatttg 1860
   taagatttca ctatggacaa catacgaaac aaaatcagtg gatctctcta tgcattcatt 1920
   atgtagtcta taataaaatc tttaaaagat cgtatatttt gcaacggagg gagtaaaaca 1980
   taacttttta atagtaatgt tgcacggctc cacactcgca gacgtacctg ccgagccaga 2040
   tgccggcggc gctgaagccg taccgcgacg acgagctccg gaacctgcgt ggcgacgacc 2100
   agcagggccc gtaccaggag cacgaccgca tctaccgcta cgacgtctac aacgacctcg 2160
   gcgagggccg ccccatcctc ggcggcaact ccgaccaccc ttacccgcgc cgcggccgca 2220
   cggagcgcaa gcccaacgcc agcgacccga gcctggagag ccggctgtcg ctgctggagc 2280
   agatctacgt gccgcgggac gagaagttcg gccacctcaa gacgtccgac ttcctgggct 2340
   actccatcaa ggccatcacg cagggcatcc tgccggccgt gcgcacctac gtggacacca 2400
   cccccggcga gttcgactcc ttccaggaca tcatcaacct ctatgagggc ggcatcaagc 2460
   tgcccaaggt ggccgccctg gaggagctcc gtaagcagtt cccgctccag ctcatcaagg 2520
   acctcctccc cgtcggcggc gactccctgc ttaagctccc cgtgccccac atcatccagg 2580
   agaacaagca ggcgtggagg accgacgagg agttcgcacg ggaggtgctc gccggcgtca 2640
   acccggtcat gatcacgcgt ctcacggtga gtcagcgatt atttgttcat tgtgtgtgta 2700
   tggtgtccat ggtgagaaag tgcagatctt gatttgcgtt gggtcgcatg cacgcatgct 2760
   gcatgcatgc aggagttccc gccaaaaagt agtctggacc ctagcaagtt tggtgaccac 2820
   accagcacca tcacggcgga gcacatagag aagaacctcg agggcctcac ggtgcagcag 2880
   gtaattggtc caagccatcg acatcaacta tgatttacct aggagtaatt ggtagctgta 2940
   gataatttgg cttcgttgca attaatttga tgctggccga tcaagtgatc gtattgggtt 3000
   tgaaatttgc aggcgctgga aagcaacagg ctgtacatcc ttgatcacca tgaccggttc 3060
   atgccgttcc tgatcgacgt caacaacctg cccggcaact tcatctacgc cacgaggacc 3120
   ctcttcttcc tgcgcggcga cggcaggctc acgccgctcg ccatcgagct gagcgagccc 3180
   atcatccagg gcggccttac cacggccaag agcaaggttt acacgccggt gcccagcggc 3240
   tccgtcgaag gctgggtgtg ggagctcgcc aaggcctacg tcgccgtcaa tgactccggg 3300
   tggcaccagc tcgtcagcca ctggtacgtt ctccacggtc gatgtgattc agtcagtcga 3360
   tgcacaacaa ctgatcgaaa tatgattgat tgaaacgcgc aggctgaaca ctcacgcggt    3420
   gatggagccg ttcgtgatct cgacgaaccg gcaccttagc gtgacgcacc cggtgcacaa    3480
   gctgctgagc ccgcactacc gcgacaccat gaccatcaac gcgctggcgc ggcagacgct    3540
   catcaacgcc ggcggcatct tcgagatgac ggtgttcccg ggcaagttcg cgttggggat    3600
   gtcggccgtg gtgtacaagg actggaagtt caccgagcag ggactgccgg acgatctcat    3660
   caagaggtac gtacctggta aatgttatga atgtgtaaaa caaattgggc gtctcgctca    3720
   ctgacaggaa cgtggtaaaa aaaatgcagg ggcatggcgg tggaggaccc gtcgagcccg    3780
   tacaaggtgc ggttgctggt gtcggactac ccgtacgcgg cggacgggct ggcgatctgg    3840
   cacgccattg agcagtacgt gagcgagtac ctggccatct actacccgaa cgacggcgtg    3900
   ctgcagggcg atacggaggt gcaggcgtgg tggaaggaga cgcgcgaggt cgggcacggc    3960
   gacctcaagg acgccccatg gtggcccaag atgcaaagtg tgccggagct ggccaaggcg    4020
   tgcaccacca tcatctggat cgggtcggcg ctgcatgcgg cagtcaactt cgggcagtac    4080
   ccctacgcgg ggttcctccc gaaccggccg acggtgagcc ggcgccgcat gccggagccc    4140
   ggcacggagg agtacgcgga gctggagcgc gacccggagc gggccttcat ccacaccatc    4200
   acgagccaga tccagaccat catcggcgtg tcgctgctgg aggtgctgtc gaagcactcc    4260
   tccgacgagc tgtacctcgg gcagcgggac acgccggagt ggacctcgga cccaaaggcc    4320
   ctggaggtgt tcaagcggtt cagcgaccgg ctggtggaga tcgagagcaa ggtggtgggc    4380
   atgaaccatg acccggagct caagaaccgc aacggcccgg ctaagtttcc ctacatgctg    4440
   ctctacccca acacctccga ccacaagggc gccgctgccg ggcttaccgc caagggcatc    4500
   cccaacagca tctccatcta atctaagcca tcggcaacca tggatgaata aagggcgttc    4560
   gccacgtacg aaacttgtcg agagattggt gtagtgtgtg tctgtgacag tactatgtca    4620
   gcagttgctc tttaagccga ataaataaag cagatttgct tcc                      4663
<210> 9
   <211> 862
   <212> PRT
   <213> Hordeum vulgare
<400> 9
   Met Leu Leu Gly Gly Leu Ile Asp Thr Leu Thr Gly Ala Asn Lys Ser
   1               5                   10                  15
   Ala Arg Leu Lys Gly Thr Val Val Leu Met Arg Lys Asn Val Leu Asp
               20                  25                  30
   Leu Asn Asp Phe Gly Ala Thr Ile Ile Asp Gly Ile Gly Glu Phe Leu
           35                  40                  45
   Gly Lys Gly Val Thr Cys Gln Leu Ile Ser Ser Thr Ala Val Asp Gln
       50                  55                  60
   Asp Asn Gly Gly Arg Gly Lys Val Gly Ala Glu Ala Glu Leu Glu Gln
   65                  70                  75                  80
   Trp Val Thr Ser Leu Pro Ser Leu Thr Thr Gly Glu Ser Lys Phe Gly
                   85                  90                  95
   Leu Thr Phe Asp Trp Glu Val Glu Lys Leu Gly Val Pro Gly Ala Ile
               100                 105                 110
   Val Val Asn Asn Tyr His Ser Ser Glu Phe Leu Leu Lys Thr Ile Thr
           115                 120                 125
   Leu His Asp Val Pro Gly Arg Ser Gly Asn Leu Thr Phe Val Ala Asn
       130                 135                 140
   Ser Trp Ile Tyr Pro Ala Ala Asn Tyr Arg Tyr Ser Arg Val Phe Phe
   145                 150                 155                 160
   Ala Asn Asp Thr Tyr Leu Pro Ser Gln Met Pro Ala Ala Leu Lys Pro
                   165                 170                 175
   Tyr Arg Asp Asp Glu Leu Arg Asn Leu Arg Gly Asp Asp Gln Gln Gly
               180                 185                 190
   Pro Tyr Gln Glu His Asp Arg Ile Tyr Arg Tyr Asp Val Tyr Asn Asp
           195                 200                 205
   Leu Gly Glu Gly Arg Pro Ile Leu Gly Gly Asn Ser Asp His Pro Tyr
       210                 215                 220
   Pro Arg Arg Gly Arg Thr Glu Arg Lys Pro Asn Ala Ser Asp Pro Ser
   225                 230                 235                 240
   Leu Glu Ser Arg Leu Ser Leu Leu Glu Gln Ile Tyr Val Pro Arg Asp
                   245                 250                 255
   Glu Lys Phe Gly His Leu Lys Thr Ser Asp Phe Leu Gly Tyr Ser Ile
               260                 265                 270
   Lys Ala Ile Thr Gln Gly Ile Leu Pro Ala Val Arg Thr Tyr Val Asp
           275                 280                 285
   Thr Thr Pro Gly Glu Phe Asp Ser Phe Gln Asp Ile Ile Asn Leu Tyr
       290                 295                 300
   Glu Gly Gly Ile Lys Leu Pro Lys Val Ala Ala Leu Glu Glu Leu Arg
   305                 310                 315                 320
   Lys Gln Phe Pro Leu Gln Leu Ile Lys Asp Leu Leu Pro Val Gly Gly
                   325                 330                 335
   Asp Ser Leu Leu Lys Leu Pro Val Pro His Ile Ile Gln Glu Asn Lys
               340                 345                 350
   Gln Ala Trp Arg Thr Asp Glu Glu Phe Ala Arg Glu Val Leu Ala Gly
           355                 360                 365
   Val Asn Pro Val Met Ile Thr Arg Leu Thr Glu Phe Pro Pro Lys Ser
           370                 375                 380
   Ser Leu Asp Pro Ser Lys Phe Gly Asp His Thr Ser Thr Ile Thr Ala
   385                 390                 395                 400
   Glu His Ile Glu Lys Asn Leu Glu Gly Leu Thr Val Gln Gln Ala Leu
                   405                 410                 415
   Glu Ser Asn Arg Leu Tyr Ile Leu Asp His His Asp Arg Phe Met Pro
               420                 425                 430
   Phe Leu Ile Asp Val Asn Asn Leu Pro Gly Asn Phe Ile Tyr Ala Thr
           435                 440                 445
   Arg Thr Leu Phe Phe Leu Arg Gly Asp Gly Arg Leu Thr Pro Leu Ala
       450                 455                 460
   Ile Glu Leu Ser Glu Pro Ile Ile Gln Gly Gly Leu Thr Thr Ala Lys
   465                 470                 475                 480
   Ser Lys Val Tyr Thr Pro Val Pro Ser Gly Ser Val Glu Gly Trp Val
                   485                 490                 495
   Trp Glu Leu Ala Lys Ala Tyr Val Ala Val Asn Asp Ser Gly Trp His
               500                 505                 510
   Gln Leu Val Ser His Trp Leu Asn Thr His Ala Val Met Glu Pro Phe
           515                 520                 525
   Val Ile Ser Thr Asn Arg His Leu Ser Val Thr His Pro Val His Lys
       530                 535                 540
   Leu Leu Ser Pro His Tyr Arg Asp Thr Met Thr Ile Asn Ala Leu Ala
   545                 550                 555                 560
   Arg Gln Thr Leu Ile Asn Ala Gly Gly Ile Phe Glu Met Thr Val Phe
                   565                 570                 575
   Pro Gly Lys Phe Ala Leu Gly Met Ser Ala Val Val Tyr Lys Asp Trp
               580                 585                 590
   Lys Phe Thr Glu Gln Gly Leu Pro Asp Asp Leu Ile Lys Arg Gly Met
           595                 600                 605
   Ala Val Glu Asp Pro Ser Ser Pro Tyr Lys Val Arg Leu Leu Val Ser
       610                 615                 620
   Asp Tyr Pro Tyr Ala Ala Asp Gly Leu Ala Ile Trp His Ala Ile Glu
   625                 630                 635                 640
   Gln Tyr Val Ser Glu Tyr Leu Ala Ile Tyr Tyr Pro Asn Asp Gly Val
                   645                 650                 655
   Leu Gln Gly Asp Thr Glu Val Gln Ala Trp Trp Lys Glu Thr Arg Glu
               660                 665                 670
   Val Gly His Gly Asp Leu Lys Asp Ala Pro Trp Trp Pro Lys Met Gln
           675                 680                 685
   Ser Val Pro Glu Leu Ala Lys Ala Cys Thr Thr Ile Ile Trp Ile Gly
       690                 695                 700
   Ser Ala Leu His Ala Ala Val Asn Phe Gly Gln Tyr Pro Tyr Ala Gly
   705                 710                 715                 720
   Phe Leu Pro Asn Arg Pro Thr Val Ser Arg Arg Arg Met Pro Glu Pro
   725 730 735
   Gly Thr Glu Glu Tyr Ala Glu Leu Glu Arg Asp Pro Glu Arg Ala Phe
   740 745 750
   Ile His Thr Ile Thr Ser Gln Ile Gln Thr Ile Ile Gly Val Ser Leu
   755 760 765
   Leu Glu Val Leu Ser Lys His Ser Ser Asp Glu Leu Tyr Leu Gly Gln
   770 775 780
   Arg Asp Thr Pro Glu Trp Thr Ser Asp Pro Lys Ala Leu Glu Val Phe
   785 790 795 800
   Lys Arg Phe Ser Asp Arg Leu Val Glu Ile Glu Ser Lys Val Val Gly
   805 810 815
   Met Asn His Asp Pro Glu Leu Lys Asn Arg Asn Gly Pro Ala Lys Phe
   820 825 830
   Pro Tyr Met Leu Leu Tyr Pro Asn Thr Ser Asp His Lys Gly Ala Ala
   835 840 845
   Ala Gly Leu Thr Ala Lys Gly Ile Pro Asn Ser Ile Ser Ile
   850 855 860
<210> 10
   <211> 2818
   <212> DNA
   <213> Hordeum vulgare
<400> 10
   agtgaaaaac agtgtgctgg tgccattggt tggagcagtg aaagcgagga gaggaggcca 60
   agaacaagat gctgctggga gggctgatcg acaccctcac gggggcgaac aagagcgccc 120
   ggctcaaggg cacggtggtg ctcatgcgca agaacgtgct ggacctcaac gacttcggcg 180
   ccaccatcat cgacggcatc ggcgagttcc tcggcaaggg cgtcacctgc cagcttatca 240
   gctccaccgc cgtcgaccaa gacaacggcg gtcgcgggaa ggtgggcgcg gaggcggagc 300
   tggagcagtg ggtgacgagc ctgccgtcgc tgacgacggg ggagtccaag ttcggcctca 360
   ccttcgactg ggaggtggag aagctcgggg tgccgggcgc catcgtcgtc aacaactacc 420
   acagctccga gttcctgctt aaaaccatca ccctccacga cgtccccggc cgcagcggca 480
   acctcacctt cgtcgccaac tcatggatct accccgccgc caactaccga tacagccgcg 540
   tcttcttcgc caacgacacg tacctgccga gccagatgcc ggcggcgctg aagccgtacc 600
   gcgacgacga gctccggaac ctgcgtggcg acgaccagca gggcccgtac caggagcacg 660
   accgcatcta ccgctacgac gtctacaacg acctcggcga gggccgcccc atcctcggcg 720
   gcaactccga ccacccttac ccgcgccgcg gccgcacgga gcgcaagccc aacgccagcg 780
   acccgagcct ggagagccgg ctgtcgctgc tggagcagat ctacgtgccg cgggacgaga 840
   agttcggcca cctcaagacg tccgacttcc tgggctactc catcaaggcc atcacgcagg 900
   gcatcctgcc ggccgtgcgc acctacgtgg acaccacccc cggcgagttc gactccttcc 960
   aggacatcat caacctctat gagggcggca tcaagctgcc caaggtggcc gccctggagg 1020
   agctccgtaa gcagttcccg ctccagctca tcaaggacct cctccccgtc ggcggcgact 1080
   ccctgcttaa gctccccgtg ccccacatca tccaggagaa caagcaggcg tggaggaccg 1140
   acgaggagtt cgcacgggag gtgctcgccg gcgtcaaccc ggtcatgatc acgcgtctca 1200
   cggagttccc gccaaaaagt agtctggacc ctagcaagtt tggtgaccac accagcacca 1260
   tcacggcgga gcacatagag aagaacctcg agggcctcac ggtgcagcag gcgctggaaa 1320
   gcaacaggct gtacatcctt gatcaccatg accggttcat gccgttcctg atcgacgtca 1380
   acaacctgcc cggcaacttc atctacgcca cgaggaccct cttcttcctg cgcggcgacg 1440
   gcaggctcac gccgctcgcc atcgagctga gcgagcccat catccagggc ggccttacca 1500
   cggccaagag caaggtttac acgccggtgc ccagcggctc cgtcgaaggc tgggtgtggg 1560
   agctcgccaa ggcctacgtc gccgtcaatg actccgggtg gcaccagctc gtcagccact 1620
   ggctgaacac tcacgcggtg atggagccgt tcgtgatctc gacgaaccgg caccttagcg 1680
   tgacgcaccc ggtgcacaag ctgctgagcc cgcactaccg cgacaccatg accatcaacg 1740
   cgctggcgcg gcagacgctc atcaacgccg gcggcatctt cgagatgacg gtgttcccgg 1800
   gcaagttcgc gttggggatg tcggccgtgg tgtacaagga ctggaagttc accgagcagg 1860
   gactgccgga cgatctcatc aagaggggca tggcggtgga ggacccgtcg agcccgtaca 1920
   aggtgcggtt gctggtgtcg gactacccgt acgcggcgga cgggctggcg atctggcacg 1980
   ccattgagca gtacgtgagc gagtacctgg ccatctacta cccgaacgac ggcgtgctgc 2040
   agggcgatac ggaggtgcag gcgtggtgga aggagacgcg cgaggtcggg cacggcgacc 2100
   tcaaggacgc cccatggtgg cccaagatgc aaagtgtgcc ggagctggcc aaggcgtgca 2160
   ccaccatcat ctggatcggg tcggcgctgc atgcggcagt caacttcggg cagtacccct 2220
   acgcggggtt cctcccgaac cggccgacgg tgagccggcg ccgcatgccg gagcccggca 2280
   cggaggagta cgcggagctg gagcgcgacc cggagcgggc cttcatccac accatcacga 2340
   gccagatcca gaccatcatc ggcgtgtcgc tgctggaggt gctgtcgaag cactcctccg 2400
   acgagctgta cctcgggcag cgggacacgc cggagtggac ctcggaccca aaggccctgg 2460
   aggtgttcaa gcggttcagc gaccggctgg tggagatcga gagcaaggtg gtgggcatga 2520
   accatgaccc ggagctcaag aaccgcaacg gcccggctaa gtttccctac atgctgctct 2580
   accccaacac ctccgaccac aagggcgccg ctgccgggct taccgccaag ggcatcccca 2640
   acagcatctc catctaatct aagccatcgg caaccatgga tgaataaagg gcgttcgcca 2700
   cgtacgaaac ttgtcgagag attggtgtag tgtgtgtctg tgacagtact atgtcagcag 2760
   ttgctcttta agccgaataa ataaagcaga tttgcttcca aaaaaaaaaa aaaaaaaa 2818
<210> 11
   <211> 4663
   <212> DNA
   <213> Hordeum vulgare
<220>
   <221> variation
   <222> (2346)..(2348)
   <223> "n" is a, c, t, or g encoding an acidic, basic, or polar amino ac
   i
<400> 11
   cagccccatg catgcacatg cacatgcaca tgcacatgca gtgcagccaa gcaccgctcg 60
   atgggcgatc acccgtcacg ggaccggagc gcgccatgcg aagcacgagg agggcacgtc 120
   accgtccgcg cgcagcacgt ggagagcacg tcgccgtccg atccatctct ccaaagccga 180
   gcgccacacc accgggaccg gacccggacc ggcctataaa ttgcccggac cgagctgcaa 240
   gcagctcctc acacacactc acgcaacaca catccatctt cactgaaaag tgaaaaacag 300
   tgtgctggtg ccattggttg gagcagtgaa agcgaggaga ggaggccaag aacaagatgc 360
   tgctgggagg gctgatcgac accctcacgg gggcgaacaa gagcgcccgg ctcaagggca 420
   cggtggtgct catgcgcaag aacgtgctgg acctcaacga cttcggcgcc accatcatcg 480
   acggcatcgg cgagttcctc ggcaagggtg tcacctgcca gcttatcagc tccaccgccg 540
   tcgaccaagg taatcactac cctcctccgg ccttctcctc tgtttacaag atatagtatt 600
   tctttcgtgt gggccggcgg ccatggatgg atggatgtgt ctggatcggc taaagaagat 660
   aggatagcta gccctggccg gtcgtcttta cctgagcatg ggcatatgcc atcgaaaaaa 720
   gagacaacag catgcatgca tggtgcgcgc accagaccac gcagagcacc ggatgctcga 780
   gacaaagcaa cacaacaagc aaggacgaca cgtcaaaagc aacacaacaa gcaaggacgg 840
   cacgtcaaaa gcaacacaaa cctaaactaa agcacaaaga cgtaagagca agcacacaat 900
   cagcaggcta taaacagttg tcatcaaaaa caacgctgga agagagagag aaggaaggaa 960
   gtagtagcca tgaaaaatta aatcaccggg cgttgctctt tgcccaacaa ttaatcaagc 1020
   agggtacgtg gcatgtatag ttcttgtaag taaactaagc atgtgatatg agaaggtacg 1080
   tggtggtgca gacaacggcg gtcgcgggaa ggtgggcgcg gaggcggagc tggagcagtg 1140
   ggtgacgagc ctgccgtcgc tgacgacggg ggagtccaag ttcggcctca ccttcgactg 1200
   ggaggtggag aagctcgggg tgccgggcgc catcgtcgtc aacaactacc acagctccga 1260
   gttcctgctt aaaaccatca ccctccacga cgtccccggc cgcagcggca acctcacctt 1320
   cgtcgccaac tcatggatct accccgccgc caactaccga tacagccgcg tcttcttcgc 1380
   caacgacgtg cgtggatttt cctctacttt cctctccttt cattttcacc gccttcgtca 1440
   ttcatggtcg atcattaagt cttgccagga caatagatga tgagctagga gtggttacca 1500
   cttagcagta cgtacattat ttattccgtg ttggtagaaa aggatatggt ttggtgcaga 1560
   tcgacacaag attgaatgaa agttgcaccg tggcaccgtg gcagcgtggt aggtgaaaat 1620
   aactgttgca cggatccacc cacatgattg ttttcatgaa taaacttttt aaggatgtgt 1680
   ctagccacat ctagatgcat gtcacataat tattgcatac caaaacgatt aaattaagca 1740
   taaaaagaaa aggaaaaaaa tactcacata tctcgacgta agatcaatga tatagtattt 1800
   agatatgcaa tatttatctt acatctaaac ctttcttcat tcttaaatat aagacatttg 1860
   taagatttca ctatggacaa catacgaaac aaaatcagtg gatctctcta tgcattcatt 1920
   atgtagtcta taataaaatc tttaaaagat cgtatatttt gcaacggagg gagtaaaaca 1980
   taacttttta atagtaatgt tgcacggctc cacactcgca gacgtacctg ccgagccaga 2040
   tgccggcggc gctgaagccg taccgcgacg acgagctccg gaacctgcgt ggcgacgacc 2100
   agcagggccc gtaccaggag cacgaccgca tctaccgcta cgacgtctac aacgacctcg 2160
   gcgagggccg ccccatcctc ggcggcaact ccgaccaccc ttacccgcgc cgcggccgca 2220
   cggagcgcaa gcccaacgcc agcgacccga gcctggagag ccggctgtcg ctgctggagc 2280
   agatctacgt gccgcgggac gagaagttcg gccacctcaa gacgtccgac ttcctgggct 2340
   actccatcaa ggccatcacg cagggcatcc tgccggccgt gcgcacctac gtggacacca 2400
   cccccggcga gttcgactcc ttccaggaca tcatcaacct ctatgagggc ggcatcaagc 2460
   tgcccaaggt ggccgccctg gaggagctcc gtaagcagtt cccgctccag ctcatcaagg 2520
   acctcctccc cgtcggcggc gactccctgc ttaagctccc cgtgccccac atcatccagg 2580
   agaacaagca ggcgtggagg accgacgagg agttcgcacg ggaggtgctc gccnnngtca 2640
   acccggtcat gatcacgcgt ctcacggtga gtcagcgatt atttgttcat tgtgtgtgta 2700
   tggtgtccat ggtgagaaag tgcagatctt gatttgcgtt gggtcgcatg cacgcatgct 2760
   gcatgcatgc aggagttccc gccaaaaagt agtctggacc ctagcaagtt tggtgaccac 2820
   accagcacca tcacggcgga gcacatagag aagaacctcg agggcctcac ggtgcagcag 2880
   gtaattggtc caagccatcg acatcaacta tgatttacct aggagtaatt ggtagctgta 2940
   gataatttgg cttcgttgca attaatttga tgctggccga tcaagtgatc gtattgggtt 3000
   tgaaatttgc aggcgctgga aagcaacagg ctgtacatcc ttgatcacca tgaccggttc    3060
   atgccgttcc tgatcgacgt caacaacctg cccggcaact tcatctacgc cacgaggacc    3120
   ctcttcttcc tgcgcggcga cggcaggctc acgccgctcg ccatcgagct gagcgagccc    3180
   atcatccagg gcggccttac cacggccaag agcaaggttt acacgccggt gcccagcggc    3240
   tccgtcgaag gctgggtgtg ggagctcgcc aaggcctacg tcgccgtcaa tgactccggg    3300
   tggcaccagc tcgtcagcca ctggtacgtt ctccacggtc gatgtgattc agtcagtcga    3360
   tgcacaacaa ctgatcgaaa tatgattgat tgaaacgcgc aggctgaaca ctcacgcggt    3420
   gatggagccg ttcgtgatct cgacgaaccg gcaccttagc gtgacgcacc cggtgcacaa    3480
   gctgctgagc ccgcactacc gcgacaccat gaccatcaac gcgctggcgc ggcagacgct    3540
   catcaacgcc ggcggcatct tcgagatgac ggtgttcccg ggcaagttcg cgttggggat    3600
   gtcggccgtg gtgtacaagg actggaagtt caccgagcag ggactgccgg acgatctcat    3660
   caagaggtac gtacctggta aatgttatga atgtgtaaaa caaattgggc gtctcgctca    3720
   ctgacaggaa cgtggtaaaa aaaatgcagg ggcatggcgg tggaggaccc gtcgagcccg    3780
   tacaaggtgc ggttgctggt gtcggactac ccgtacgcgg cggacgggct ggcgatctgg    3840
   cacgccattg agcagtacgt gagcgagtac ctggccatct actacccgaa cgacggcgtg    3900
   ctgcagggcg atacggaggt gcaggcgtgg tggaaggaga cgcgcgaggt cgggcacggc    3960
   gacctcaagg acgccccatg gtggcccaag atgcaaagtg tgccggagct ggccaaggcg    4020
   tgcaccacca tcatctggat cgggtcggcg ctgcatgcgg cagtcaactt cgggcagtac    4080
   ccctacgcgg ggttcctccc gaaccggccg acggtgagcc ggcgccgcat gccggagccc    4140
   ggcacggagg agtacgcgga gctggagcgc gacccggagc gggccttcat ccacaccatc    4200
   acgagccaga tccagaccat catcggcgtg tcgctgctgg aggtgctgtc gaagcactcc    4260
   tccgacgagc tgtacctcgg gcagcgggac acgccggagt ggacctcgga cccaaaggcc    4320
   ctggaggtgt tcaagcggtt cagcgaccgg ctggtggaga tcgagagcaa ggtggtgggc    4380
   atgaaccatg acccggagct caagaaccgc aacggcccgg ctaagtttcc ctacatgctg    4440
   ctctacccca acacctccga ccacaagggc gccgctgccg ggcttaccgc caagggcatc    4500
   cccaacagca tctccatcta atctaagcca tcggcaacca tggatgaata aagggcgttc    4560
   gccacgtacg aaacttgtcg agagattggt gtagtgtgtg tctgtgacag tactatgtca    4620
   gcagttgctc tttaagccga ataaataaag cagatttgct tcc                      4663
<210> 12
   <211> 862
   <212> PRT
   <213> Hordeum vulgare
<220>
   <221> variation
   <222> (368)..(368)
   <223> "Xaa" is an acidic, basic, or polar amino acid
<400> 12
   Met Leu Leu Gly Gly Leu Ile Asp Thr Leu Thr Gly Ala Asn Lys Ser
   1               5                   10                  15
   Ala Arg Leu Lys Gly Thr Val Val Leu Met Arg Lys Asn Val Leu Asp
               20                  25                  30
   Leu Asn Asp Phe Gly Ala Thr Ile Ile Asp Gly Ile Gly Glu Phe Leu
           35                  40                  45
   Gly Lys Gly Val Thr Cys Gln Leu Ile Ser Ser Thr Ala Val Asp Gln
       50                  55                  60
   Asp Asn Gly Gly Arg Gly Lys Val Gly Ala Glu Ala Glu Leu Glu Gln
   65                  70                  75                  80
   Trp Val Thr Ser Leu Pro Ser Leu Thr Thr Gly Glu Ser Lys Phe Gly
                   85                  90                  95
   Leu Thr Phe Asp Trp Glu Val Glu Lys Leu Gly Val Pro Gly Ala Ile
               100                 105                 110
   Val Val Asn Asn Tyr His Ser Ser Glu Phe Leu Leu Lys Thr Ile Thr
           115                 120                 125
   Leu His Asp Val Pro Gly Arg Ser Gly Asn Leu Thr Phe Val Ala Asn
       130                 135                 140
   Ser Trp Ile Tyr Pro Ala Ala Asn Tyr Arg Tyr Ser Arg Val Phe Phe
   145                 150                 155                 160
   Ala Asn Asp Thr Tyr Leu Pro Ser Gln Met Pro Ala Ala Leu Lys Pro
                   165                 170                 175
   Tyr Arg Asp Asp Glu Leu Arg Asn Leu Arg Gly Asp Asp Gln Gln Gly
               180                 185                 190
   Pro Tyr Gln Glu His Asp Arg Ile Tyr Arg Tyr Asp Val Tyr Asn Asp
           195                 200                 205
   Leu Gly Glu Gly Arg Pro Ile Leu Gly Gly Asn Ser Asp His Pro Tyr
       210                 215                 220
   Pro Arg Arg Gly Arg Thr Glu Arg Lys Pro Asn Ala Ser Asp Pro Ser
   225                 230                 235                 240
   Leu Glu Ser Arg Leu Ser Leu Leu Glu Gln Ile Tyr Val Pro Arg Asp
                   245                 250                 255
   Glu Lys Phe Gly His Leu Lys Thr Ser Asp Phe Leu Gly Tyr Ser Ile
               260                      265                      270
   Lys Ala Ile Thr Gln Gly Ile Leu Pro Ala Val Arg Thr Tyr Val Asp
           275                 280                 285
   Thr Thr Pro Gly Glu Phe Asp Ser Phe Gln Asp Ile Ile Asn Leu Tyr
       290                 295                 300
   Glu Gly Gly Ile Lys Leu Pro Lys Val Ala Ala Leu Glu Glu Leu Arg
   305                 310                 315                 320
   Lys Gln Phe Pro Leu Gln Leu Ile Lys Asp Leu Leu Pro Val Gly Gly
                   325                 330                 335
   Asp Ser Leu Leu Lys Leu Pro Val Pro His Ile Ile Gln Glu Asn Lys
               340                 345                 350
   Gln Ala Trp Arg Thr Asp Glu Glu Phe Ala Arg Glu Val Leu Ala Xaa
           355                 360                 365
   Val Asn Pro Val Met Ile Thr Arg Leu Thr Glu Phe Pro Pro Lys Ser
       370                 375                 380
   Ser Leu Asp Pro Ser Lys Phe Gly Asp His Thr Ser Thr Ile Thr Ala
   385                 390                 395                 400
   Glu His Ile Glu Lys Asn Leu Glu Gly Leu Thr Val Gln Gln Ala Leu
                   405                 410                 415
   Glu Ser Asn Arg Leu Tyr Ile Leu Asp His His Asp Arg Phe Met Pro
               420                 425                 430
   Phe Leu Ile Asp Val Asn Asn Leu Pro Gly Asn Phe Ile Tyr Ala Thr
           435                 440                 445
   Arg Thr Leu Phe Phe Leu Arg Gly Asp Gly Arg Leu Thr Pro Leu Ala
       450                 455                 460
   Ile Glu Leu Ser Glu Pro Ile Ile Gln Gly Gly Leu Thr Thr Ala Lys
   465                 470                 475                 480
   Ser Lys Val Tyr Thr Pro Val Pro Ser Gly Ser Val Glu Gly Trp Val
                   485                 490                 495
   Trp Glu Leu Ala Lys Ala Tyr Val Ala Val Asn Asp Ser Gly Trp His
               500                 505                 510
   Gln Leu Val Ser His Trp Leu Asn Thr His Ala Val Met Glu Pro Phe
           515                 520                 525
   Val Ile Ser Thr Asn Arg His Leu Ser Val Thr His Pro Val His Lys
       530                 535                 540
   Leu Leu Ser Pro His Tyr Arg Asp Thr Met Thr Ile Asn Ala Leu Ala
   545                 550                 555                 560
   Arg Gln Thr Leu Ile Asn Ala Gly Gly Ile Phe Glu Met Thr Val Phe
                   565                 570                 575
   Pro Gly Lys Phe Ala Leu Gly Met Ser Ala Val Val Tyr Lys Asp Trp
               580                 585                 590
   Lys Phe Thr Glu Gln Gly Leu Pro Asp Asp Leu Ile Lys Arg Gly Met
           595                 600                 605
   Ala Val Glu Asp Pro Ser Ser Pro Tyr Lys Val Arg Leu Leu Val Ser
       610                 615                 620
   Asp Tyr Pro Tyr Ala Ala Asp Gly Leu Ala Ile Trp His Ala Ile Glu
   625                 630                 635                 640
   Gln Tyr Val Ser Glu Tyr Leu Ala Ile Tyr Tyr Pro Asn Asp Gly Val
                   645                 650                 655
   Leu Gln Gly Asp Thr Glu Val Gln Ala Trp Trp Lys Glu Thr Arg Glu
               660                 665                 670
   Val Gly His Gly Asp Leu Lys Asp Ala Pro Trp Trp Pro Lys Met Gln
           675                 680                 685
   Ser Val Pro Glu Leu Ala Lys Ala Cys Thr Thr Ile Ile Trp Ile Gly
       690                 695                 700
   Ser Ala Leu His Ala Ala Val Asn Phe Gly Gln Tyr Pro Tyr Ala Gly
   705                 710                 715                 720
   Phe Leu Pro Asn Arg Pro Thr Val Ser Arg Arg Arg Met Pro Glu Pro
                   725                 730                 735
   Gly Thr Glu Glu Tyr Ala Glu Leu Glu Arg Asp Pro Glu Arg Ala Phe
               740                 745                 750
   Ile His Thr Ile Thr Ser Gln Ile Gln Thr Ile Ile Gly Val Ser Leu
           755                 760                 765
   Leu Glu Val Leu Ser Lys His Ser Ser Asp Glu Leu Tyr Leu Gly Gln
       770                 775                 780
   Arg Asp Thr Pro Glu Trp Thr Ser Asp Pro Lys Ala Leu Glu Val Phe
   785                 790                 795                 800
   Lys Arg Phe Ser Asp Arg Leu Val Glu Ile Glu Ser Lys Val Val Gly
                   805                 810                 815
   Met Asn His Asp Pro Glu Leu Lys Asn Arg Asn Gly Pro Ala Lys Phe
               820                 825                 830
   Pro Tyr Met Leu Leu Tyr Pro Asn Thr Ser Asp His Lys Gly Ala Ala
           835                 840                 845
   Ala Gly Leu Thr Ala Lys Gly Ile Pro Asn Ser Ile Ser Ile
       850                 855                 860
<210> 13
   <211> 20
   <212> DNA
   <213> Hordeum vulgare
<400> 13
   cgctacgacg tctacaacga 20
<210> 14
   <211> 20
   <212> DNA
   <213> Hordeum vulgare
<400> 14
   cagactactt tttggcggga 20
<210> 15
   <211> 839
   <212> PRT
   <213> Glycine max
<400> 15
   Met Phe Ser Ala Gly His Lys Ile Lys Gly Thr Val Val Leu Met Pro
   1               5                   10                  15
   Lys Asn Glu Leu Glu Val Asn Pro Asp Gly Ser Ala Val Asp Asn Leu
               20                  25                  30
   Asn Ala Phe Leu Gly Arg Ser Val Ser Leu Gln Leu Ile Ser Ala Thr
           35                  40                  45
   Lys Ala Asp Ala His Gly Lys Gly Lys Val Gly Lys Asp Thr Phe Leu
       50                  55                  60
   Glu Gly Ile Asn Thr Ser Leu Pro Thr Leu Gly Ala Gly Glu Ser Ala
   65                  70                  75                  80
   Phe Asn Ile His Phe Glu Trp Asp Gly Ser Met Gly Ile Pro Gly Ala
                   85                  90                  95
   Phe Tyr Ile Lys Asn Tyr Met Gln Val Glu Phe Phe Leu Lys Ser Leu
               100                 105                 110
   Thr Leu Glu Ala Ile Ser Asn Gln Gly Thr Ile Arg Phe Val Cys Asn
           115                 120                 125
   Ser Trp Val Tyr Asn Thr Lys Leu Tyr Lys Ser Val Arg Ile Phe Phe
       130                 135                 140
   Ala Asn His Thr Tyr Val Pro Ser Glu Thr Pro Ala Pro Leu Val Ser
   145                 150                 155                 160
   Tyr Arg Glu Glu Glu Leu Lys Ser Leu Arg Gly Asn Gly Thr Gly Glu
                   165                 170                 175
   Arg Lys Glu Tyr Asp Arg Ile Tyr Asp Tyr Asp Val Tyr Asn Asp Leu
               180                 185                 190
   Gly Asn Pro Asp Lys Ser Glu Lys Leu Ala Arg Pro Val Leu Gly Gly
           195                 200                 205
   Ser Ser Thr Phe Pro Tyr Pro Arg Arg Gly Arg Thr Gly Arg Gly Pro
       210                 215                 220
   Thr Val Thr Asp Pro Asn Thr Glu Lys Gln Gly Glu Val Phe Tyr Val
   225                 230                 235                 240
   Pro Arg Asp Glu Asn Leu Gly His Leu Lys Ser Lys Asp Ala Leu Glu
                   245                 250                 255
   Ile Gly Thr Lys Ser Leu Ser Gln Ile Val Gln Pro Ala Phe Glu Ser
               260                 265                 270
   Ala Phe Asp Leu Lys Ser Thr Pro Ile Glu Phe His Ser Phe Gln Asp
           275                 280                 285
   Val His Asp Leu Tyr Glu Gly Gly Ile Lys Leu Pro Arg Asp Val Ile
       290                 295                 300
   Ser Thr Ile Ile Pro Leu Pro Val Ile Lys Glu Leu Tyr Arg Thr Asp
   305                 310                 315                 320
   Gly Gln His Ile Leu Lys Phe Pro Gln Pro His Val Val Gln Val Ser
                   325                 330                 335
   Gln Ser Ala Trp Met Thr Asp Glu Glu Phe Ala Arg Glu Met Ile Ala
               340                 345                 350
   Gly Val Asn Pro Cys Val Ile Arg Gly Leu Glu Glu Phe Pro Pro Lys
           355                 360                 365
   Ser Asn Leu Asp Pro Ala Ile Tyr Gly Asp Gln Ser Ser Lys Ile Thr
       370                 375                 380
   Ala Asp Ser Leu Asp Leu Asp Gly Tyr Thr Met Asp Glu Ala Leu Gly
   385                 390                 395                 400
   Ser Arg Arg Leu Phe Met Leu Asp Tyr His Asp Ile Phe Met Pro Tyr
                   405                 410                 415
   Val Arg Gln Ile Asn Gln Leu Asn Ser Ala Lys Thr Tyr Ala Thr Arg
               420                 425                 430
   Thr Ile Leu Phe Leu Arg Glu Asp Gly Thr Leu Lys Pro Val Ala Ile
           435                 440                 445
   Glu Leu Ser Leu Pro His Ser Ala Gly Asp Leu Ser Ala Ala Val Ser
       450                 455                 460
   Gln Val Val Leu Pro Ala Lys Glu Gly Val Glu Ser Thr Ile Trp Leu
   465                 470                 475                 480
   Leu Ala Lys Ala Tyr Val Ile Val Asn Asp Ser Cys Tyr His Gln Leu
                   485                 490                 495
   Met Ser His Trp Leu Asn Thr His Ala Ala Met Glu Pro Phe Val Ile
               500                 505                 510
   Ala Thr His Arg His Leu Ser Val Leu His Pro Ile Tyr Lys Leu Leu
           515                 520                 525
   Thr Pro His Tyr Arg Asn Asn Met Asn Ile Asn Ala Leu Ala Arg Gln
       530                 535                 540
   Ser Leu Ile Asn Ala Asn Gly Ile Ile Glu Thr Thr Phe Leu Pro Ser
   545                 550                 555                 560
   Lys Tyr Ser Val Glu Met Ser Ser Ala Val Tyr Lys Asn Trp Val Phe
                   565                 570                 575
   Thr Asp Gln Ala Leu Pro Ala Asp Leu Ile Lys Arg Gly Val Ala Ile
               580                 585                 590
   Lys Asp Pro Ser Thr Pro His Gly Val Arg Leu Leu Ile Glu Asp Tyr
           595                 600                 605
   Pro Tyr Ala Ala Asp Gly Leu Glu Ile Trp Ala Ala Ile Lys Thr Trp
       610                 615                 620
   Val Gln Glu Tyr Val Pro Leu Tyr Tyr Ala Arg Asp Asp Asp Val Lys
   625                 630                 635                 640
   Asn Asp Ser Glu Leu Gln His Trp Trp Lys Glu Ala Val Glu Lys Gly
                   645                 650                 655
   His Gly Asp Leu Lys Asp Lys Pro Trp Trp Pro Lys Leu Gln Thr Leu
               660                 665                 670
   Glu Asp Leu Val Glu Val Cys Leu Ile Ile Ile Trp Ile Ala Ser Ala
           675                 680                 685
   Leu His Ala Ala Val Asn Phe Gly Gln Tyr Pro Tyr Gly Gly Leu Ile
       690                 695                 700
   Met Asn Arg Pro Thr Ala Ser Arg Arg Leu Leu Pro Glu Lys Gly Thr
   705                 710                 715                 720
   Pro Glu Tyr Glu Glu Met Ile Asn Asn His Glu Lys Ala Tyr Leu Arg
                   725                 730                 735
   Thr Ile Thr Ser Lys Leu Pro Thr Leu Ile Ser Leu Ser Val Ile Glu
               740                 745                 750
   Ile Leu Ser Thr His Ala Ser Asp Glu Val Tyr Leu Gly Gln Arg Asp
           755                 760                 765
   Asn Pro His Trp Thr Ser Asp Ser Lys Ala Leu Gln Ala Phe Gln Lys
       770                 775                 780
   Phe Gly Asn Lys Leu Lys Glu Ile Glu Glu Lys Leu Val Arg Arg Asn
   785                 790                 795                 800
   Asn Asp Pro Ser Leu Gln Gly Asn Arg Leu Gly Pro Val Gln Leu Pro
                   805                 810                 815
   Tyr Thr Leu Leu Tyr Pro Ser Ser Glu Glu Gly Leu Thr Phe Arg Gly
               820                 825                 830
   Ile Pro Asn Ser Ile Ser Ile
           835
<210> 16
   <211> 865
   <212> PRT
   <213> Glycine max
<400> 16
   Met Phe Ser Val Pro Gly Val Ser Gly Ile Leu Asn Arg Gly Gly Gly
   1               5                   10                  15
   His Lys Ile Lys Gly Thr Val Val Leu Met Arg Lys Asn Val Leu Asp
               20                  25                  30
   Phe Asn Ser Val Ala Asp Leu Thr Lys Gly Asn Val Gly Gly Leu Ile
           35                  40                  45
   Gly Thr Gly Leu Asn Val Val Gly Ser Thr Leu Asp Asn Leu Thr Ala
       50                  55                  60
   Phe Leu Gly Arg Ser Val Ala Leu Gln Leu Ile Ser Ala Thr Lys Pro
   65                  70                  75                  80
   Leu Ala Asn Gly Lys Gly Lys Val Gly Lys Asp Thr Phe Leu Glu Gly
                   85                  90                  95
   Ile Ile Val Ser Leu Pro Thr Leu Gly Ala Gly Glu Ser Ala Phe Asn
               100                 105                 110
   Ile Gln Phe Glu Trp Asp Glu Ser Met Gly Ile Pro Gly Ala Phe Tyr
           115                 120                 125
   Ile Lys Asn Tyr Met Gln Val Glu Phe Tyr Leu Lys Ser Leu Thr Leu
       130                 135                 140
   Glu Asp Val Pro Asn Gln Gly Thr Ile Arg Phe Val Cys Asn Ser Trp
   145                 150                 155                 160
   Val Tyr Asn Thr Lys Leu Tyr Lys Ser Val Arg Ile Phe Phe Ala Asn
                   165                 170                 175
   His Thr Tyr Val Pro Ser Glu Thr Pro Ala Ala Leu Val Gly Tyr Arg
               180                 185                 190
   Glu Glu Glu Leu Lys Asn Leu Arg Gly Asp Gly Lys Gly Glu Arg Lys
           195                 200                 205
   Glu His Asp Arg Ile Tyr Asp Tyr Asp Val Tyr Asn Asp Leu Gly Asn
       210                 215                 220
   Pro Asp His Gly Glu Asn Phe Ala Arg Pro Ile Leu Gly Gly Ser Ser
   225                 230                 235                 240
   Thr His Pro Tyr Pro Arg Arg Gly Arg Thr Gly Arg Tyr Pro Thr Arg
                   245                 250                 255
   Lys Asp Gln Asn Ser Glu Lys Pro Gly Glu Val Tyr Val Pro Arg Asp
               260                 265                 270
   Glu Asn Phe Gly His Leu Lys Ser Ser Asp Phe Leu Ala Tyr Gly Ile
           275                 280                 285
   Lys Ser Leu Ser Gln Tyr Val Leu Pro Ala Phe Glu Ser Val Phe Asp
       290                 295                 300
   Leu Asn Phe Thr Pro Asn Glu Phe Asp Ser Phe Gln Asp Val Arg Asp
   305                 310                 315                 320
   Leu His Glu Gly Gly Ile Lys Leu Pro Thr Glu Val Ile Ser Thr Ile
                   325                 330                 335
   Met Pro Leu Pro Val Val Lys Glu Leu Phe Arg Thr Asp Gly Glu Gln
               340                 345                 350
   Val Leu Lys Phe Pro Pro Pro His Val Ile Gln Val Ser Lys Ser Ala
           355                 360                 365
   Trp Met Thr Asp Glu Glu Phe Ala Arg Glu Met Val Ala Gly Val Asn
       370                 375                 380
   Pro Cys Val Ile Arg Gly Leu Gln Glu Phe Pro Pro Lys Ser Asn Leu
   385                 390                 395                 400
   Asp Pro Thr Ile Tyr Gly Glu Gln Thr Ser Lys Ile Thr Ala Asp Ala
                   405                 410                 415
   Leu Asp Leu Asp Gly Tyr Thr Val Asp Glu Ala Leu Ala Ser Arg Arg
               420                 425                 430
   Leu Phe Met Leu Asp Tyr His Asp Val Phe Met Pro Tyr Ile Arg Arg
           435                 440                 445
   Ile Asn Gln Thr Tyr Ala Lys Ala Tyr Ala Thr Arg Thr Ile Leu Phe
       450                 455                 460
   Leu Arg Glu Asn Gly Thr Leu Lys Pro Val Ala Ile Glu Leu Ser Leu
   465                 470                 475                 480
   Pro His Pro Ala Gly Asp Leu Ser Gly Ala Val Ser Gln Val Ile Leu
                   485                 490                 495
   Pro Ala Lys Glu Gly Val Glu Ser Thr Ile Trp Leu Leu Ala Lys Ala
               500                 505                 510
   Tyr Val Val Val Asn Asp Ser Cys Tyr His Gln Leu Met Ser His Trp
           515                 520                 525
   Leu Asn Thr His Ala Val Ile Glu Pro Phe Ile Ile Ala Thr Asn Arg
       530                 535                 540
   His Leu Ser Ala Leu His Pro Ile Tyr Lys Leu Leu Thr Pro His Tyr
   545                 550                 555                 560
   Arg Asp Thr Met Asn Ile Asn Ala Leu Ala Arg Gln Ser Leu Ile Asn
                   565                 570                 575
   Ala Asp Gly Ile Ile Glu Lys Ser Phe Leu Pro Ser Lys His Ser Val
               580                 585                 590
   Glu Met Ser Ser Ala Val Tyr Lys Asn Trp Val Phe Thr Asp Gln Ala
           595                 600                 605
   Leu Pro Ala Asp Leu Ile Lys Arg Gly Val Ala Ile Lys Asp Pro Ser
       610                 615                 620
   Ala Pro His Gly Leu Arg Leu Leu Ile Glu Asp Tyr Pro Tyr Ala Val
   625                 630                 635                 640
   Asp Gly Leu Glu Ile Trp Ala Ala Ile Lys Thr Trp Val Gln Glu Tyr
                   645                 650                 655
   Val Ser Leu Tyr Tyr Ala Arg Asp Asp Asp Val Lys Pro Asp Ser Glu
   660 665 670
   Leu Gln Gln Trp Trp Lys Glu Ala Val Glu Lys Gly His Gly Asp Leu
   675 680 685
   Lys Asp Lys Pro Trp Trp Pro Lys Leu Gln Thr Ile Glu Glu Leu Val
   690 695 700
   Glu Ile Cys Thr Ile Ile Ile Trp Thr Ala Ser Ala Leu His Ala Ala
   705 710 715 720
   Val Asn Phe Gly Gln Tyr Pro Tyr Gly Gly Phe Ile Leu Asn Arg Pro
   725 730 735
   Thr Ser Ser Arg Arg Leu Leu Pro Glu Lys Gly Thr Pro Glu Tyr Glu
   740 745 750
   Glu Met Val Lys Ser His Gln Lys Ala Tyr Leu Arg Thr Ile Thr Ser
   755 760 765
   Lys Phe Gln Thr Leu Val Asp Leu Ser Val Ile Glu Ile Leu Ser Arg
   770 775 780
   His Ala Ser Asp Glu Val Tyr Leu Gly Gln Arg Asp Asn Pro His Trp
   785 790 795 800
   Thr Ser Asp Ser Lys Ala Leu Gln Ala Phe Gln Lys Phe Gly Asn Lys
   805 810 815
   Leu Lys Glu Ile Glu Glu Lys Leu Ala Arg Lys Asn Asn Asp Gln Ser
   820 825 830
   Leu Ser Asn Arg Leu Gly Pro Val Gln Leu Pro Tyr Thr Leu Leu His
   835 840 845
   Pro Asn Ser Glu Gly Leu Thr Cys Arg Gly Ile Pro Asn Ser Ile Ser
   850 855 860
   Ile
   865
<210> 17
   <211> 857
   <212> PRT
   <213> Glycine max
<400> 17
   Met Leu Gly Gly Leu Leu His Arg Gly His Lys Ile Lys Gly Thr Val
   1 5 10 15
   Val Leu Met Arg Lys Asn Val Leu Asp Val Asn Ser Val Thr Ser Val
   20 25 30
   Gly Gly Ile Ile Gly Gln Gly Leu Asp Leu Val Gly Ser Thr Leu Asp
   35 40 45
   Thr Leu Thr Ala Phe Leu Gly Arg Ser Val Ser Leu Gln Leu Ile Ser
   50 55 60
   Ala Thr Lys Ala Asp Ala Asn Gly Lys Gly Lys Leu Gly Lys Ala Thr
   65                  70                  75                  80
   Phe Leu Glu Gly Ile Ile Thr Ser Leu Pro Thr Leu Gly Ala Gly Gln
                   85                  90                  95
   Ser Ala Phe Lys Ile Asn Phe Glu Trp Asp Asp Gly Ser Gly Ile Pro
               100                 105                 110
   Gly Ala Phe Tyr Ile Lys Asn Phe Met Gln Thr Glu Phe Phe Leu Val
           115                 120                 125
   Ser Leu Thr Leu Glu Asp Ile Pro Asn His Gly Ser Ile His Phe Val
       130                 135                 140
   Cys Asn Ser Trp Ile Tyr Asn Ala Lys Leu Phe Lys Ser Asp Arg Ile
   145                 150                 155                 160
   Phe Phe Ala Asn Gln Thr Tyr Leu Pro Ser Glu Thr Pro Ala Pro Leu
                   165                 170                 175
   Val Lys Tyr Arg Glu Glu Glu Leu His Asn Leu Arg Gly Asp Gly Thr
               180                 185                 190
   Gly Glu Arg Lys Glu Trp Glu Arg Ile Tyr Asp Tyr Asp Val Tyr Asn
           195                 200                 205
   Asp Leu Gly Asp Pro Asp Lys Gly Glu Asn His Ala Arg Pro Val Leu
       210                 215                 220
   Gly Gly Asn Asp Thr Phe Pro Tyr Pro Arg Arg Gly Arg Thr Gly Arg
   225                 230                 235                 240
   Lys Pro Thr Arg Lys Asp Pro Asn Ser Glu Ser Arg Ser Asn Asp Val
                   245                 250                 255
   Tyr Leu Pro Arg Asp Glu Ala Phe Gly His Leu Lys Ser Ser Asp Phe
               260                 265                 270
   Leu Thr Tyr Gly Leu Lys Ser Val Ser Gln Asn Val Leu Pro Leu Leu
           275                 280                 285
   Gln Ser Ala Phe Asp Leu Asn Phe Thr Pro Arg Glu Phe Asp Ser Phe
       290                 295                 300
   Asp Glu Val His Gly Leu Tyr Ser Gly Gly Ile Lys Leu Pro Thr Asp
   305                 310                 315                 320
   Ile Ile Ser Lys Ile Ser Pro Leu Pro Val Leu Lys Glu Ile Phe Arg
                   325                 330                 335
   Thr Asp Gly Glu Gln Ala Leu Lys Phe Pro Pro Pro Lys Val Ile Gln
               340                 345                 350
   Val Ser Lys Ser Ala Trp Met Thr Asp Glu Glu Phe Ala Arg Glu Met
           355                 360                 365
   Leu Ala Gly Val Asn Pro Asn Leu Ile Arg Cys Leu Lys Asp Phe Pro
       370                 375                 380
   Pro Arg Ser Lys Leu Asp Ser Gln Val Tyr Gly Asp His Thr Ser Gln
   385                 390                 395                 400
   Ile Thr Lys Glu His Leu Glu Pro Asn Leu Glu Gly Leu Thr Val Asp
   405 410 415
   Glu Ala Ile Gln Asn Lys Arg Leu Phe Leu Leu Asp His His Asp Pro
   420 425 430
   Ile Met Pro Tyr Leu Arg Arg Ile Asn Ala Thr Ser Thr Lys Ala Tyr
   435 440 445
   Ala Thr Arg Thr Ile Leu Phe Leu Lys Asn Asp Gly Thr Leu Arg Pro
   450 455 460
   Leu Ala Ile Glu Leu Ser Leu Pro His Pro Gln Gly Asp Gln Ser Gly
   465 470 475 480
   Ala Phe Ser Gln Val Phe Leu Pro Ala Asp Glu Gly Val Glu Ser Ser
   485 490 495
   Ile Trp Leu Leu Ala Lys Ala Tyr Val Val Val Asn Asp Ser Cys Tyr
   500 505 510
   His Gln Leu Val Ser His Trp Leu Asn Thr His Ala Val Val Glu Pro
   515 520 525
   Phe Ile Ile Ala Thr Asn Arg His Leu Ser Val Val His Pro Ile Tyr
   530 535 540
   Lys Leu Leu His Pro His Tyr Arg Asp Thr Met Asn Ile Asn Gly Leu
   545 550 555 560
   Ala Arg Leu Ser Leu Val Asn Asp Gly Gly Val Ile Glu Gln Thr Phe
   565 570 575
   Leu Trp Gly Arg Tyr Ser Val Glu Met Ser Ala Val Val Tyr Lys Asp
   580 585 590
   Trp Val Phe Thr Asp Gln Ala Leu Pro Ala Asp Leu Ile Lys Arg Gly
   595 600 605
   Met Ala Ile Glu Asp Pro Ser Cys Pro His Gly Ile Arg Leu Val Ile
   610 615 620
   Glu Asp Tyr Pro Tyr Thr Val Asp Gly Leu Glu Ile Trp Asp Ala Ile
   625 630 635 640
   Lys Thr Trp Val His Glu Tyr Val Phe Leu Tyr Tyr Lys Ser Asp Asp
   645 650 655
   Thr Leu Arg Glu Asp Pro Glu Leu Gln Ala Cys Trp Lys Glu Leu Val
   660 665 670
   Glu Val Gly His Gly Asp Lys Lys Asn Glu Pro Trp Trp Pro Lys Met
   675 680 685
   Gln Thr Arg Glu Glu Leu Val Glu Ala Cys Ala Ile Ile Ile Trp Thr
   690 695 700
   Ala Ser Ala Leu His Ala Ala Val Asn Phe Gly Gln Tyr Pro Tyr Gly
   705 710 715 720
   Gly Leu Ile Leu Asn Arg Pro Thr Leu Ser Arg Arg Phe Met Pro Glu
   725 730 735
   Lys Gly Ser Ala Glu Tyr Glu Glu Leu Arg Lys Asn Pro Gln Lys Ala
   740 745 750
   Tyr Leu Lys Thr Ile Thr Pro Lys Phe Gln Thr Leu Ile Asp Leu Ser
   755 760 765
   Val Ile Glu Ile Leu Ser Arg His Ala Ser Asp Glu Val Tyr Leu Gly
   770 775 780
   Glu Arg Asp Asn Pro Asn Trp Thr Ser Asp Thr Arg Ala Leu Glu Ala
   785 790 795 800
   Phe Lys Arg Phe Gly Asn Lys Leu Ala Gln Ile Glu Asn Lys Leu Ser
   805 810 815
   Glu Arg Asn Asn Asp Glu Lys Leu Arg Asn Arg Cys Gly Pro Val Gln
   820 825 830
   Met Pro Tyr Thr Leu Leu Leu Pro Ser Ser Lys Glu Gly Leu Thr Phe
   835 840 845
   Arg Gly Ile Pro Asn Ser Ile Ser Ile
   850 855
<210> 18
   <211> 864
   <212> PRT
   <213> Hordeum vulgare
<400> 18
   Met Leu Gly Val Gly Gly Ile Val Ser Asp Leu Thr Gly Gly Ile Arg
   1 5 10 15
   Gly Ala His Leu Lys Gly Ser Val Val Leu Met Arg Lys Asn Ala Leu
   20 25 30
   Asp Phe Asn Asp Phe Gly Ala His Val Met Asp Gly Val Thr Glu Leu
   35 40 45
   Leu Gly Arg Gly Val Thr Cys Gln Leu Ile Ser Ser Thr Asn Val Asp
   50 55 60
   His Asn Asn Gly Gly Arg Gly Lys Val Gly Ala Glu Ala Asn Leu Glu
   65 70 75 80
   Gln Trp Leu Leu Pro Thr Asn Leu Pro Phe Ile Thr Thr Gly Glu Asn
   85 90 95
   Lys Phe Ala Val Thr Phe Asp Trp Ser Val Asp Lys Leu Gly Val Pro
   100 105 110
   Gly Ala Ile Ile Val Lys Asn Asn His Ala Ser Glu Phe Phe Leu Lys
   115 120 125
   Thr Ile Thr Leu Asp Asn Val Pro Gly Arg Gly Thr Ile Val Phe Val
   130 135 140
   Ala Asn Ser Trp Val Tyr Pro Gln Ala Lys Tyr Arg Tyr Asn Arg Val
   145 150 155 160
   Phe Phe Ala Asn Asp Thr Tyr Leu Pro His Gln Met Pro Ala Ala Leu
                   165                 170                 175
   Lys Pro Tyr Arg Asp Asp Glu Leu Arg Asn Leu Arg Gly Asp Asp Gln
               180                 185                 190
   Gln Gly Pro Tyr Leu Asp His Asp Arg Val Tyr Arg Tyr Asp Val Tyr
           195                 200                 205
   Asn Asp Leu Gly Asp Ser Arg Asp Val Leu Gly Gly Ser Lys Asp Leu
       210                 215                 220
   Pro Tyr Pro Arg Arg Cys Arg Thr Gly Arg Lys Pro Ser Asp Ser Lys
   225                 230                 235                 240
   Pro Asp His Glu Ser Arg Leu Leu Leu Leu Val Gln Asn Val Tyr Val
                   245                 250                 255
   Leu Arg Asp Glu Leu Phe Gly His Leu Lys Gln Ser Asp Leu Leu Gly
               260                 265                 270
   Tyr Thr Leu Lys Gly Trp Leu Asp Gly Ile Ile Leu Ala Ile Arg Thr
           275                 280                 285
   Tyr Val Asp Leu Ser Pro Gly Glu Phe Asp Ser Phe Ala Asp Ile Leu
       290                 295                 300
   Lys Leu Tyr Glu Gly Gly Ile Lys Leu Pro Asn Ile Pro Ala Leu Glu
   305                 310                 315                 320
   Glu Val Arg Lys Arg Phe Pro Leu Gln Leu Val Lys Asp Leu Ile Pro
                   325                 330                 335
   Lys Gly Gly Asp Phe Leu Leu Lys Leu Pro Lys Pro Glu Ile Ile Lys
               340                 345                 350
   Val Asp Gln Lys Ala Trp Met Thr Asp Glu Glu Phe Ala Arg Glu Met
           355                 360                 365
   Leu Ala Gly Val Asn Pro Met Met Ile Lys Arg Leu Thr Glu Phe Pro
       370                 375                 380
   Pro Lys Ser Thr Leu Asp Pro Ser Lys Tyr Gly Asp His Thr Ser Thr
   385                 390                 395                 400
   Met Thr Glu Glu His Val Ala Lys Ser Leu Glu Gly Leu Thr Val Gln
                   405                 410                 415
   Gln Ala Leu Ala Gly Asn Arg Leu Tyr Ile Val Asp Gln His Asp Asn
               420                 425                 430
   Leu Met Pro Phe Leu Ile Asp Ile Asn Asn Leu Asp Ala Ser Phe Val
           435                 440                 445
   Tyr Ala Thr Arg Thr Leu Leu Phe Leu Arg Gly Asp Gly Thr Leu Ala
       450                 455                 460
   Pro Val Ala Ile Glu Leu Ser Ser Pro Leu Ile Gln Gly Glu Leu Thr
   465                 470                 475                 480
   Thr Ala Lys Ser Ala Val Tyr Thr Pro Gln His Ala Gly Val Glu Gly
                   485                 490                 495
   Trp Ile Trp Gln Leu Ala Lys Ala Tyr Ala Ser Val Asn Asp Tyr Gly
   500 505 510
   Trp His Gln Leu Ile Ser His Trp Leu Asn Thr His Ala Val Met Glu
   515 520 525
   Pro Phe Val Ile Ala Thr Asn Arg Gln Leu Ser Val Thr His Pro Val
   530 535 540
   Tyr Lys Leu Leu His Pro His Tyr Arg Asp Thr Met Asn Ile Asn Ala
   545 550 555 560
   Arg Ala Arg Gly Leu Leu Ile Asn Ala Gly Gly Val Ile Glu Met Thr
   565 570 575
   Val Phe Pro His Lys His Ala Met Pro Met Ser Ser Met Val Tyr Lys
   580 585 590
   His Trp Asn Phe Thr Glu Gln Ala Leu Pro Ala Asp Leu Ile Lys Arg
   595 600 605
   Gly Met Ala Val Glu Asp Ala Ser Ser Pro His Lys Val Arg Leu Leu
   610 615 620
   Ile Lys Asp Tyr Pro Tyr Ala Thr Asp Gly Leu Ala Val Trp Asp Ala
   625 630 635 640
   Ile Glu Gln Trp Val Ser Asp Tyr Leu Thr Ile Tyr Tyr Pro Asn Asp
   645 650 655
   Gly Val Leu Gln Gly Asp Val Glu Leu Gln Ala Trp Trp Lys Glu Val
   660 665 670
   Arg Glu Val Gly His Gly Asp Leu Lys Asp Ala Ala Trp Trp Pro Lys
   675 680 685
   Met Gln Thr Val Ala Glu Leu Ile Lys Ala Cys Ala Thr Ile Ile Trp
   690 695 700
   Thr Gly Ser Ala Leu His Ala Ala Val Asn Phe Gly Gln Tyr Pro Tyr
   705 710 715 720
   Ser Gly Tyr His Pro Asn Lys Pro Ser Ala Ser Arg Arg Pro Met Pro
   725 730 735
   Val Gln Gly Ser Glu Glu Tyr Ala Glu Leu Glu Arg Asp Pro Glu Lys
   740 745 750
   Ala Phe Ile Arg Thr Ile Thr Ser Gln Phe His Ala Leu Val Gly Ile
   755 760 765
   Ser Leu Met Glu Ile Leu Ser Lys His Ser Ser Asp Glu Val Tyr Leu
   770 775 780
   Gly Gln His Asp Thr Pro Ala Trp Thr Ser Asp Ala Lys Ala Leu Glu
   785 790 795 800
   Ala Phe Lys Arg Phe Gly Ala Lys Leu Glu Gly Ile Glu Lys Gln Val
   805 810 815
   Val Ala Met Asn Ser Asp Pro Gln Leu Lys Asn Arg Thr Gly Pro Ala
   820 825 830
   Lys Phe Pro Tyr Met Leu Leu Tyr Pro Asn Thr Ser Asp His Thr Gly
   835 840 845
   Gln Ala Glu Gly Leu Thr Ala Arg Gly Ile Pro Asn Ser Ile Ser Ile
   850 855 860